# EUROPEAN PATENT APPLICATION

(11) **EP 3 827 847 A1**
(43) Date of publication of application: **02.06.2021**
(21) Application number: 19841509.3
(22) Date of filing: 25.07.2019
(51) Int. Cl.: A61K 48/00, A61P 7/04, C12N 15/10, C12N 15/113, C12N 15/86, C12N 15/90

(54) **GENE EDITING OF ANTICOAGULANTS**

(30) Priority: 26.07.2018 US 201862703578 P
(71) Applicant: Toolgen Incorporated, Seoul 08501 (KR)
(72) Inventor: SONG, Dong Woo, Seoul 08532 (KR); LEE, Jung Min, Pohang-si Gyeongsangbuk-do 37669 (KR); LEE, Kyu Jun, Seoul 08804 (KR); KIM, Un Gi, Seoul 08767 (KR)
(74) Representative: dompatent von Kreisler Selting Werner - Partnerschaft von Patent- und Rechtsanwälten mbB
(86) International application number: PCT/KR2019/009238
(87) International publication number: WO 2020/022803

(57) **Abstract**

The present invention relates to a composition for gene manipulation for artificially manipulating a blood coagulation inhibitory gene present in the genome of a cell to regulate a blood coagulation system. More particularly, the present invention relates to a composition for gene manipulation, which includes a guide nucleic acid capable of targeting a blood coagulation inhibitory gene, and an editor protein. Also, the present invention relates to a method of treating or improving coagulopathy using the composition for gene manipulation for artificially manipulating a blood coagulation inhibitory gene.

## Description

### [Technical Field]

The present invention relates to artificial manipulation or modification of a blood coagulation inhibitory gene for a normal blood coagulation system. More particularly, the present invention relates to a system capable of artificially regulating blood coagulation, which includes a composition capable of artificially manipulating a blood coagulation inhibitory gene to activate an abnormal blood coagulation system, that is, an inactivated blood coagulation system.

### [Background Art]

Hemophilia is a hemorrhagic disease caused by the deficiency of blood coagulation factors. Coagulation factors in blood consists of factors I to XIII, and hemophilia is caused by the genetic defects of the corresponding factors. Hemophilia A is caused by the deficiency of factor VIII and is known to affect about one in every 5000 male babies. Hemophilia B is caused by the deficiency of factor IX and is known to affect about one in every 20,000 male babies. Hemophilia patients are estimated to exceed approximately 700,000 all over the world.

Therapies known so far are mainly to continuously administer the deficient factors, and there are no basic therapeutic methods.

Therefore, there is a need for a therapeutic agent capable of achieving a long-term effect in a single therapy, which can essentially knock out the expression of proteins that inactivate a blood coagulation system using the gene editing.

### [Disclosure]

### [Technical Problem]

One embodiment of the present invention is to provide a method of treating coagulopathy. Another embodiment of the present invention is to provide a composition for gene manipulation.

Still another embodiment of the present invention is to provide a guide nucleic acid capable of targeting a blood coagulation inhibitory gene.

### [Technical Solution]

To solve the above problems, the present invention provides a composition for gene manipulation for artificially manipulating a blood coagulation inhibitory gene present in the genome of a cell to regulate a blood coagulation system. More particularly, the present invention provides a composition for gene manipulation, which includes a guide nucleic acid capable of targeting a blood coagulation inhibitory gene, and an editor protein. Also, the present invention provides a method of treating or improving coagulopathy using the composition for gene manipulation for artificially manipulating a blood coagulation inhibitory gene.

To treat hemophilia, the present invention provides a method of treating a hemophilia including administration(introduction) of a composition for gene manipulation into a subject to be treated.

In one embodiment, the method of treating a hemophilia may include an introduction (administration) of a composition for gene manipulation into a subject to be treated.

The composition for gene manipulation may include the following:
a guide nucleic acid including a guide sequence forming a complementary bond with a target sequence located in a blood coagulation inhibitory gene, or a nucleic acid sequence encoding the same; and
an editor protein, or a nucleic acid sequence encoding the same,

The blood coagulation inhibitory gene may be an AT(antithrombin) gene or TFPI(tissue factor pathway inhibitor) gene.

The guide sequence may be one or more guide sequences selected from a SEC ID NO:425 to 830.

The complementary bond may include mismatching bonds of 0 to 5.

In one embodiment, the guide sequence may be one or more sequences selected from a SEQ ID NO : 427, 428, 436, 437, 443, 444, 447, 448, 449, 454, 458, 460, 461, 463, 464, 466, 467, 469, 473, 474, 622, 623, 624, 625, 626, 627, 628, 630, 632, 634, 635, 638, 639, 641, 642, 659, 660, 661, 662, 663, 664, 665, 666, 667, 668, 669, 670, 671, 672, 673, 674, 675, 676, 677, 678, 679, 680, 681, 682, 683, 684, 686, 687 and 688.

In one embodiment, the guide sequence may be one or more sequences selected from a SEQ ID NO : 692, 694, 705, 709, 710, 721, 726, 731, 733, 735, 740, 741, 742, 748, 781, 783, 786, 788, 791, 792, 794, 795, 796, 797, 798, 800, 802, 803, 804, 809, 810, 811, 813, 814, 815, 816, 817, 818, 819, 820, 821, 822, 823, 824, 825, 826, 827, 828, 829 and 830.

The guide sequence may form a complementary bond with a target sequence located in exon 1, exon 2, exon 3, exon 4, exon 5, exon 6 or exon 7 of AT gene.

The guide sequence may form a complementary bond with a target sequence located in exon 2, exon 3, exon 5, exon 6 or exon 7 of TFPI gene.

The editor protein may be a Streptococcus pyogenes-derived Cas9 protein, a Staphylococcus aureus-derived Cas9 protein, or a Campylobacter jejuni-derived Cas9 protein.

The composition for gene manipulation may be a form of guide nucleic acid-editor protein complex combined guide nucleic acid and editor protein.

Here, the guide nucleic acid may be a guide RNA(gRNA).

The guide nucleic acid and the editor protein may be present in one or more vectors in a form of a nucleic acid sequence, respectively.

Here, the vector may be a plasmid or viral vector.

Here, the viral vector may be one or more viral vectors selected from the group consisting of a retrovirus, a lentivirus, an adenovirus, an adeno-associated virus (AAV), a vaccinia virus, a poxvirus and a herpes simplex virus.

The composition for gene manipulation may optionally further include a donor including a nucleic acid sequence to be inserted, or a nucleic acid sequence encoding the same.

Here, the nucleic acid sequence to be inserted may be a partial nucleic acid sequence of blood coagulation inhibitory gene.

Here, the nucleic acid sequence to be inserted may be a complete or partial sequence of a gene encoding a blood coagulation associated protein.

For example, the blood coagulation associated protein may be one or more proteins selected from the group consisting of a factor XII, factor XIIa, factor XI, factor XIa, factor IX, factor IXa, factor X, factor Xa, factor VIII, factor VIIIa, factor VII, factor VIIa, factor V, factor Va, prothrombin, thrombin, factor XIII, factor XIIIa, fibrinogen, fibrin and tissue factor.

The guide nucleic acid, the editor protein and donor may be present in one or more vectors in a form of a nucleic acid sequence, respectively.

Here, the vector may be a plasmid or viral vector.

Here, the viral vector may be one or more viral vectors selected from the group consisting of a retrovirus, a lentivirus, an adenovirus, an adeno-associated virus (AAV), a vaccinia virus, a poxvirus and a herpes simplex virus.

The administration(introduction) may be performed by injection, transfusion, implantation or transplantation.

The administration(introduction) may be performed via an administration route selected from intrahepatic, subcutaneous, intradermal, intraocular, intravitreal, intratumoral, intranodal, intramedullary, intramuscular, intravenous, intralymphatic and intraperitoneal route.

The hemophilia may be a hemophilia A, hemophilia B or hemophilia C.

The subject to be treated is a mammal including a human, a monkey, a mouse and a rat.

Alternatively, the present invention provides a composition for gene manipulation for a specific purpose.

In one embodiment, the composition for gene manipulation may include the following:
a guide nucleic acid including a guide sequence forming a complementary bond with a target sequence located in blood coagulation inhibitory gene, or a nucleic acid sequence encoding the same; and
an editor protein, or a nucleic acid sequence encoding the same,

The blood coagulation inhibitory gene may be an AT(antithrombin) gene or TFPI(tissue factor pathway inhibition) gene.

The guide sequence may be one or more guide sequences selected from a SEQ ID to 830.

The complementary bond may include mismatching bonds of 0 to 5.

The target sequence may be one or more sequences selected from a SEQ ID NO: 19 to 424.

In one embodiment, the target sequence may be one or more sequences selected from a SEQ ID NO: 21, 22, 30, 31, 37, 38, 41, 42, 43, 48, 52, 54, 55, 57, 58, 60, 61, 63, 67, 68, 216, 217, 218, 219, 220, 221, 222, 224, 226, 228, 229, 232, 233, 235, 236, 253, 254, 255, 256, 257, 258, 259, 260, 261, 262, 263, 264, 265, 266, 267, 268, 269, 270, 271, 272, 273, 274, 275, 276, 277, 278, 280, 281 and 282.

In one embodiment, the target sequence may be one or more sequences selected from a SEQ ID NO: 286, 288, 299, 303, 304, 315, 320, 325, 327, 329, 334, 335, 336, 342, 375, 377, 380, 382, 385, 386, 388, 389, 390, 391, 392, 394, 396, 397, 398, 403, 404, 405, 407, 408, 409, 410, 411, 412, 413, 414, 415, 416, 417, 418, 419, 420, 421, 422, 423 and 424.

The guide sequence may be one or more sequences selected from a SEQ ID NO: 427, 428, 436, 437, 443, 444, 447, 448, 449, 454, 458, 460, 461, 463, 464, 466, 467, 469, 473, 474, 622, 623, 624, 625, 626, 627, 628, 630, 632, 634, 635, 638, 639, 641, 642, 659, 660, 661, 662, 663, 664, 665, 666, 667, 668, 669, 670, 671, 672, 673, 674, 675, 676, 677, 678, 679, 680, 681, 682, 683, 684, 686, 687 and 688.

The guide sequence may be one or more sequences selected from a SEQ ID NO: 692, 694, 705, 709, 710, 721, 726, 731, 733, 735, 740, 741, 742, 748, 781, 783, 786, 788, 791, 792, 794, 795, 796, 797, 798, 800, 802, 803, 804, 809, 810, 811, 813, 814, 815, 816, 817, 818, 819, 820, 821, 822, 823, 824, 825, 826, 827, 828, 829 and 830.

The guide sequence may form a complementary bond with a target sequence located in exon 1, exon 2, exon 3, exon 4, exon 5, exon 6 or exon 7 of AT gene.

The guide sequence may form a complementary bond with a target sequence located in exon 2, exon 3, exon 5, exon 6 or exon 7 of TFPI gene.

The guide nucleic acid may include a guide domain.

Here, the guide sequence may be included in the guide domain.

The guide nucleic acid may include one or more domains selected from a first complementary domain, a second complementary domain, a linker domain, a proximal domain and a tail domain.

The editor protein may be a Streptococcus pyogenes-derived Cas9 protein, a Staphylococcus aureus-derived Cas9 protein, or a Campylobacter jejuni-derived Cas9 protein.

The composition for gene manipulation may be a form of guide nucleic acid-editor protein complex combined guide nucleic acid and editor protein.

Here, the guide nucleic acid may be a guide RNA(gRNA).

The guide nucleic acid and the editor protein may be present in one or more vectors in a form of a nucleic acid sequence, respectively.

Here, the vector may be a plasmid or viral vector.

Here, the viral vector may be one or more viral vectors selected from the group consisting of a retrovirus, a lentivirus, an adenovirus, an adeno-associated virus (AAV), a vaccinia virus, a poxvirus and a herpes simplex virus.

The composition for gene manipulation may optionally further include a donor including a nucleic acid sequence to be inserted, or a nucleic acid sequence encoding the same.

Here, the nucleic acid sequence to be inserted may be a partial nucleic acid sequence of blood coagulation inhibitory gene.

Here, the nucleic acid sequence to be inserted may be a complete or partial sequence of a gene encoding a blood coagulation associated protein.

For example, the blood coagulation associated protein may be one or more proteins selected from the group consisting of a factor XII, factor XIIa, factor XI, factor XIa, factor IX, factor IXa, factor X, factor Xa, factor VIII, factor VIIIa, factor VII, factor VIIa, factor V, factor Va, prothrombin, thrombin, factor XIII, factor XIIIa, fibrinogen, fibrin and tissue factor.

The guide nucleic acid, the editor protein and donor may be present in one or more vectors in a form of a nucleic acid sequence, respectively.

Here, the vector may be a plasmid or viral vector.

Here, the viral vector may be one or more viral vectors selected from the group consisting of a retrovirus, a lentivirus, an adenovirus, an adeno-associated virus (AAV), a vaccinia virus, a poxvirus and a herpes simplex virus.

The present invention provides a guide nucleic acid capable of targeting an immune participating gene for a specific purpose.

In one embodiment, the guide nucleic acid may include a guide sequence forming a complementary bond with a target sequence located in blood coagulation inhibitory gene.

The blood coagulation inhibitory gene may be an AT(antithrombin) gene or TFPI(tissue factor pathway inhibition) gene.

The guide sequence may be one or more guide sequences selected from a SEQ ID to 830

The complementary bond may include mismatching bonds of 0 to 5.

The guide nucleic acid may form a complex with an editor protein.

In one embodiment, the guide sequence may be one or more sequences selected from a SEQ ID NO: 427, 428, 436, 437, 443, 444, 447, 448, 449, 454, 458, 460, 461, 463, 464, 466, 467, 469, 473, 474, 622, 623, 624, 625, 626, 627, 628, 630, 632, 634, 635, 638, 639, 641, 642, 659, 660, 661, 662, 663, 664, 665, 666, 667, 668, 669, 670, 671, 672, 673, 674, 675, 676, 677, 678, 679, 680, 681, 682, 683, 684, 686, 687 and 688.

In one embodiment, the guide sequence may be one or more sequences selected from a SEQ ID NO: 692, 694, 705, 709, 710, 721, 726, 731, 733, 735, 740, 741, 742, 748, 781, 783, 786, 788, 791, 792, 794, 795, 796, 797, 798, 800, 802, 803, 804, 809, 810, 811, 813, 814, 815, 816, 817, 818, 819, 820, 821, 822, 823, 824, 825, 826, 827, 828, 829 and 830.

The guide sequence may form a complementary bond with a target sequence located in exon 1, exon 2, exon 3, exon 4, exon 5, exon 6 or exon 7 of AT gene

The guide sequence may form a complementary bond with a target sequence located in exon 2, exon 3, exon 5, exon 6 or exon 7 of TFPI gene.

The guide nucleic acid may include a guide domain

Here, the guide sequence may be included in the guide domain.

The guide nucleic acid may include one or more domains selected from a first complementary domain, a second complementary domain, a linker domain, a proximal domain and a tail domain.

### [Advantageous Effects]

According to the present invention, a blood coagulation system can be regulated using a composition for gene manipulation. More particularly, the blood coagulation system can be regulated using the composition for gene manipulation, which includes a guide nucleic acid targeting a blood coagulation inhibitory gene, and an editor protein, by artificially manipulating and/or modifying the blood coagulation inhibitory gene to regulate the function and/or expression of the blood coagulation inhibitory gene. Also, coagulopathy can be treated or improved using the composition for gene manipulation for artificially manipulating a blood coagulation inhibitory gene.

### [Modes of the Invention]

Unless defined otherwise, all technical and scientific terms used herein have the same meaning as commonly understood by those of ordinary skill in the art to which the present invention belongs. Methods and materials similar or identical to those described herein can be used in practice or testing of the present invention. All publications, patent applications, patents and other references mentioned herein are incorporated by reference in their entirety. In addition, materials, methods and examples are merely illustrative, and not intended to be limitive.

### One aspect of the disclosure of the present specification relates to a guide nucleic acid.

The "guide nucleic acid" refers to a nucleotide sequence that recognizes a target nucleic acid, gene or chromosome, and interacts with an editor protein. Here, the guide nucleic acid may complementarily bind to a partial nucleotide sequence in the target nucleic acid, gene or chromosome. In addition, a partial nucleotide sequence of the guide nucleic acid may interact with some amino acids of the editor protein, thereby forming a guide nucleic acid-editor protein complex.

The guide nucleic acid may perform a function to induce a guide nucleic acid-editor protein complex to be located in a target region of a target nucleic acid, gene or chromosome.

The guide nucleic acid may be present in the form of DNA, RNA or a DNA/RNA hybrid, and may have a nucleic acid sequence of 5 to 150 nt.

The guide nucleic acid may have one continuous nucleic acid sequence.

For example, the one continuous nucleic acid sequence may be (N)ₘ, where N represents A, T, C or G, or A, U, C or G, and m is an integer of 1 to 150.

The guide nucleic acid may have two or more continuous nucleic acid sequences.

For example, the two or more continuous nucleic acid sequences may be (N)ₘ and (N)ₒ, where N represents A, T, C or G, or A, U, C or G, m and o are an integer of 1 to 150, and m and o may be the same as or different from each other.

The guide nucleic acid may include one or more domains.

The domains may be a functional domain which is a guide domain, a first complementary domain, a linker domain, a second complementary domain, a proximal domain, or a tail domain, but are not limited to.

Here, one guide nucleic acid may have two or more functional domains. Here, the two or more functional domains may be different from each other. Alternatively, the two or more functional domains included in one guide nucleic acid may be the same as each other. For one example, one guide nucleic acid may have two or more proximal domains. For another example, one guide nucleic acid may have two or more tail domains. However, the description that the functional domains included in one guide nucleic acid are the same domains does not mean that the sequences of the two functional domains are the same. Even if the sequences are different, the two functional domain can be the same domain when perform functionally the same function.

The functional domain will be described in detail below.

### i) Guide domain

The term "guide domain" is a domain capable of complementary binding with partial sequence of either strand of a double strand of a target gene or a nucleic acid, and acts for specific interaction with a target gene or a nucleic acid. For example, the guide domain may perform a function to induce a guide nucleic acid-editor protein complex to be located to a specific nucleotide sequence of a target gene or a nucleic acid.

The guide domain may be a sequence of 10 to 35 nucleotides.

In an example, the guide domain may be a sequence of 10 to 35, 15 to 35, 20 to 35, 25 to 35 or 30 to 35 nucleotides.

In another example, the guide domain may be a sequence of 10 to 15, 15 to 20, 20 to 25, 25 to 30 or 30 to 35 nucleotides.

The guide domain may include a guide sequence.

The "guide sequence" is a nucleotide sequence complementary to partial sequence of either strand of a double strand of a target gene or a nucleic acid. Here, the guide sequence may be a nucleotide sequence having at least 50%, 55%, 60%, 65%, 70%, 75%, 80%, 85%, 90%, 95% or more complementarity or complete complementarity.

The guide sequence may be a sequence of 10 to 25 nucleotides.

In an example, the guide sequence may be a sequence of 10 to 25, 15 to 25 or 20 to 25 nucleotides.

In another example, the guide sequence may be a sequence of 10 to 15, 15 to 20 or 20 to 25 nucleotides.

In addition, the guide domain may further include an additional nucleotide sequence.

The additional nucleotide sequence may be utilized to improve or degrade the function of the guide domain.

The additional nucleotide sequence may be utilized to improve or degrade the function of the guide sequence.

The additional nucleotide sequence may be a sequence of 1 to 10 nucleotides.

In one example, the additional nucleotide sequence may be a sequence of 2 to 10, 4 to 10, 6 to 10 or 8 to 10 nucleotides.

In another example, the additional nucleotide sequence may be a sequence of 1 to 3, 3 to 6 or 7 to 10 nucleotides.

In one embodiment, the additional nucleotide sequence may be a sequence of 1, 2, 3, 4, 5, 6, 7, 8, 9 or 10 nucleotides.

For example, the additional nucleotide sequence may be one nucleotide sequence G (guanine), or two nucleotide sequence GG.

The additional nucleotide sequence may be located at the 5' end of the guide sequence.

The additional nucleotide sequence may be located at the 3' end of the guide sequence.

### ii) First complementary domain

The term "first complementary domain" is a domain including a nucleotide sequence complementary to a second complementary domain to be described in below, and has enough complementarity so as to form a double strand with the second complementary domain. For example, the first complementary domain may be a nucleotide sequence having at least 50%, 55%, 60%, 65%, 70%, 75%, 80%, 85%, 90%, 95% or more complementarity or complete complementarity to a second complementary domain.

The first complementary domain may form a double strand with a second complementary domain by a complementary binding. Here, the formed double strand may act to form a guide nucleic acid-editor protein complex by interacting with some amino acids of the editor protein.

The first complementary domain may be a sequence of 5 to 35 nucleotides.

In an example, the first complementary domain may be a sequence of 5 to 35, 10 to 35, 15 to 35, 20 to 35, 25 to 35, or 30 to 35 nucleotides.

In another example, the first complementary domain may be a sequence of 1 to 5, 5 to 10, 10 to 15, 15 to 20, 20 to 25, 25 to 30 or 30 to 35 nucleotides.

### iii) Linker domain

The term "linker domain" is a nucleotide sequence connecting two or more domains, which are two or more identical or different domains. The linker domain may be connected with two or more domains by covalent bonding or non-covalent bonding, or may connect two or more domains covalently or non-covalently.

The linker domain may be a sequence of 1 to 30 nucleotides.

In one example, the linker domain may be a sequence of 1 to 5, 5 to 10, 10 to 15, 15 to 20, 20 to 25, or 25 to 30 nucleotides.

In another example, the linker domain may be a sequence of 1 to 30, 5 to 30, 10 to 30, 15 to 30, 20 to 30, or 25 to 30 nucleotides.

### iv) Second complementary domain

The term "second complementary domain" is a domain including a nucleotide sequence complementary to the first complementary domain described above, and has enough complementarity so as to form a double strand with the first complementary domain. For example, the second complementary domain may be a nucleotide sequence having at least 50%, 55%, 60%, 65%, 70%, 75%, 80%, 85%, 90%, 95% or more complementarity or complete complementarity to a first complementary domain.

The second complementary domain may form a double strand with a first complementary domain by a complementary binding. Here, the formed double strand may act to form a guide nucleic acid-editor protein complex by interacting with some amino acids of the editor protein. The second complementary domain may have a nucleotide sequence complementary to a first complementary domain, and a nucleotide sequence having no complementarity to the first complementary domain, for example, a nucleotide sequence not forming a double strand with the first complementary domain, and may have a longer sequence than the first complementary domain.

The second complementary domain may be a sequence of 5 to 35 nucleotides.

In an example, the second complementary domain may be a sequence of 1 to 35, 5 to 35, 10 to 35, 15 to 35, 20 to 35, 25 to 35, or 30 to 35 nucleotides.

In another example, the second complementary domain may be a sequence of 1 to 5, 5 to 10, 10 to 15, 15 to 20, 20 to 25, 25 to 30 or 30 to 35 nucleotides.

### v) Proximal domain

The term "proximal domain" is a nucleotide sequence located adjacent to a second complementary domain.

The proximal domain may include a complementary nucleotide sequence therein, and may form a double strand due to a complementary nucleotide sequence.

The proximal domain may be a sequence of 1 to 20 nucleotides.

In one example, the proximal domain may be a sequence of 1 to 20, 5 to 20, 10 to 20 or 15 to 20 nucleotide.

In another example, the proximal domain may be a sequence of 1 to 5, 5 to 10, 10 to 15 or 15 to 20 nucleotides.

### vi) Tail domain

The term "tail domain" is a nucleotide sequence located at one or more ends of the both ends of the guide nucleic acid.

The tail domain may include a complementary nucleotide sequence therein, and may form a double strand due to a complementary nucleotide sequence.

The tail domain may be a sequence of 1 to 50 nucleotides.

In an example, the tail domain may be a sequence of 5 to 50, 10 to 50, 15 to 50, 20 to 50, 25 to 50, 30 to 50, 35 to 50, 40 to 50, or 45 to 50 nucleotides.

In another example, the tail domain may be a sequence of 1 to 5, 5 to 10, 10 to 15, 15 to 20, 20 to 25, 25 to 30, 30 to 35, 35 to 40, 40 to 45, or 45 to 50 nucleotides.

Meanwhile, a part or all of the nucleic acid sequences included in the domains, that is, the guide domain, the first complementary domain, the linker domain, the second complementary domain, the proximal domain and the tail domain may selectively or additionally include a chemical modification.

The chemical modification may be, but is not limited to, methylation, acetylation, phosphorylation, phosphorothioate linkage, a locked nucleic acid (LNA), 2'-O-methyl 3'phosphorothioate (MS) or 2'-O-methyl 3'thioPACE (MSP).

The guide nucleic acid includes one or more domains.

The guide nucleic acid may include a guide domain.

The guide nucleic acid may include a first complementary domain.

The guide nucleic acid may include a linker domain.

The guide nucleic acid may include a second complementary domain.

The guide nucleic acid may include a proximal domain.

The guide nucleic acid may include a tail domain.

Here, the guide nucleic acid may include 1, 2, 3, 4, 5, 6 or more domains.

The guide nucleic acid may include 1, 2, 3, 4, 5, 6 or more guide domains.

The guide nucleic acid may include 1, 2, 3, 4, 5, 6 or more first complementary domains.

The guide nucleic acid may include 1, 2, 3, 4, 5, 6 or more linker domains.

The guide nucleic acid may include 1, 2, 3, 4, 5, 6 or more second complementary domains. The guide nucleic acid may include 1, 2, 3, 4, 5, 6 or more proximal domains.

The guide nucleic acid may include 1, 2, 3, 4, 5, 6 or more tail domains.

Here, the guide nucleic acid may include one type of domain doubly.

The guide nucleic acid may include several domains singly or doubly.

The guide nucleic acid may include the same type of domain. Here, the same type of domain may have the same nucleic acid sequence or different nucleic acid sequences.

The guide nucleic acid may include two types of domains. Here, the two different types of domains may have different nucleic acid sequences or the same nucleic acid sequence.

The guide nucleic acid may include three types of domains. Here, the three different types of domains may have different nucleic acid sequences or the same nucleic acid sequence.

The guide nucleic acid may include four types of domains. Here, the four different types of domains may have different nucleic acid sequences, or the same nucleic acid sequence.

The guide nucleic acid may include five types of domains. Here, the five different types of domains may have different nucleic acid sequences, or the same nucleic acid sequence.

The guide nucleic acid may include six types of domains. Here, the six different types of domains may have different nucleic acid sequences, or the same nucleic acid sequence.

For example, the guide nucleic acid may consist of [guide domain]-[first complementary domain]-[linker domain]-[second complementary domain]-[linker domain]-[guide domain]-[first complementary domain]-[linker domain]-[second complementary domain]. Here, the two guide domains may include guide sequences for different or the same targets, the two first complementary domains and the two second complementary domains may have the same or different nucleic acid sequences. When the guide domains include guide sequences for different targets, the guide nucleic acids may specifically bind to two different targets, and here, the specific bindings may be performed simultaneously or sequentially. In addition, the linker domains may be cleaved by specific enzymes, and the guide nucleic acids may be divided into two or three parts in the presence of specific enzymes.

### In one embodiment of the disclosure of the present specification, the guide nucleic acid may be a gRNA. gRNA

The "gRNA" refers to an RNA capable of specifically targeting a gRNA-CRISPR enzyme complex, that is, a CRISPR complex, with respect to a target gene or a nucleic acid. In addition, the gRNA refers to an RNA specific to a target gene or a nucleic acid, which may bind to a CRISPR enzyme and guide the CRISPR enzyme to the target gene or the nucleic acid.

The gRNA may include multiple domains. Due to each domain, interactions may occur in a three-dimensional structure or active form of a gRNA strand, or between these strands.

The gRNA may be called single-stranded gRNA (single RNA molecule, single gRNA or sgRNA); or double-stranded gRNA (including more than one, generally, two discrete RNA molecules).

In one exemplary embodiment, the single-stranded gRNA may include a guide domain, that is, a domain including a guide sequence capable of forming a complementary bond with a target gene or a nucleic acid; a first complementary domain; a linker domain; a second complementary domain, which is a domain having a sequence complementary to the first complementary domain sequence, thereby forming a double-stranded nucleic acid with the first complementary domain; a proximal domain; and optionally a tail domain in the 5' to 3' direction.

In another embodiment, the double-stranded gRNA may include a first strand which includes a guide domain, that is, a domain including a guide sequence capable of forming a complementary bond with a target gene or a nucleic acid and a first complementary domain; and a second strand which includes a second complementary domain, which is a domain having a sequence complementary to the first complementary domain sequence, thereby forming a double-stranded nucleic acid with the first complementary domain, a proximal domain; and optionally a tail domain in the 5' to 3' direction.

Here, the first strand may be referred to as crRNA, and the second strand may be referred to as tracrRNA. The crRNA may include a guide domain and a first complementary domain, and the tracrRNA may include a second complementary domain, a proximal domain and optionally a tail domain.

In still another embodiment, the single-stranded gRNA may include a guide domain, that is, a domain including a guide sequence capable of forming a complementary bond with a target gene or a nucleic acid; a first complementary domain; a second complementary domain, and a domain having a sequence complementary to the first complementary domain sequence, thereby forming a double-stranded nucleic acid with the first complementary domain in the 3' to 5' direction.

Here, the first complementary domain may have homology with a natural first complementary domain or may be derived from a natural first complementary domain. In addition, the first complementary domain may have a difference in the nucleotide sequence of a first complementary domain depending on the species existing in nature, may be derived from a first complementary domain contained in the species existing in nature, or may have partial or complete homology with the first complementary domain contained in the species existing in nature.

In one exemplary embodiment, the first complementary domain may have partial, that is, at least 50% or more, or complete homology with a first complementary domain of *Streptococcus pyogenes, Campylobacter jejuni, Streptococcus thermophilus, Staphylococcus aureus* or *Neisseria meningitides,* or a first complementary domain derived therefrom.

For example, when the first complementary domain is the first complementary domain of *Streptococcus pyogenes* or a first complementary domain derived therefrom, the first complementary domain may be 5'-GUUUUAGAGCUA-3' (SEQ ID NO: 1) or a nucleotide sequence having partial, that is, at least 50% or more, or complete homology with 5'-GUUUUAGAGCUA-3' (SEQ ID NO: 1). Here, the first complementary domain may further include (X)ₙ, resulting in 5'-GUUUUAGAGCUA(X)ₙ-3' (SEQ ID NO: 1). The X may be selected from the group consisting of nucleotides A, T, U and G, and the n may represent the number of nucleotide, which is an integer of 5 to 15. Here, the (X)ₙ may be an integer n repeats of the same nucleotide, or an integer n number of nucleotide sequences in which nucleotides of A, T, U and G are mixed.

In another example, when the first complementary domain is the first complementary domain of *Campylobacter jejuni* or a first complementary domain derived therefrom, the first complementary domain may be 5'-GUUUUAGUCCCUUUUUAAAUUUCUU-3' (SEQ ID NO: 2) or 5'-GUUUUAGUCCCUU-3'(SEQ ID NO: 3), or a nucleotide sequence having partial, that is, at least 50% or more, or complete homology with 5'-GUUUUAGUCCCUUUUUAAAUUUCUU-3' (SEQ ID NO: 2) or 5'-GUUUUAGUCCCUU-3'(SEQ ID NO: 3). Here, the first complementary domain may further include (X)ₙ, resulting in 5'-GUUUUAGUCCCUUUUUAAAUUUCUU(X)ₙ-3'(SEQ ID NO: 2) or 5'-GUUUUAGUCCCUU(X)n-3' (SEQ ID NO: 3). The X may be selected from the group consisting of nucleotides A, T, U and G, and the n may represent the number of nucleotide, which is an integer of 5 to 15. Here, the (X)ₙ may be an integer n repeats of the same nucleotide, or an integer n number of nucleotide sequences in which nucleotides of A, T, U and G are mixed.

In another embodiment, the first complementary domain may have partial, that is, at least 50% or more, or complete homology with a first complementary domain of *Parcubacteria bacterium* (GWC2011_GWC2_44_17), *Lachnospiraceae bacterium* (MC2017), *Butyrivibrio proteoclasiicus, Peregrinibacteria bacterium* (GW2011_GWA_33_10), *Acidaminococcus sp.* (BV3L6), *Porphyromonas macacae, Lachnospiraceae bacterium* (ND2006), *Porphyromonas crevioricanis, Prevotella disiens, Moraxella bovoculi* (237), *Smiihella sp.* (SC_K08D17), *Leptospira inadai, Lachnospiraceae bacterium* (MA2020), *Francisella novicida* (U112), *Candidatus Methanoplasma termitum* or *Eubacterium eligens,* or a first complementary domain derived therefrom.

For example, when the first complementary domain is the first complementary domain of *Parcubacteria bacterium* or a first complementary domain derived therefrom, the first complementary domain may be 5'-UUUGUAGAU-3' (SEQ ID NO: 4), or a nucleotide sequence having partial, that is, at least 50% or more homology with 5'-UUUGUAGAU-3' (SEQ ID NO: 4). Here, the first complementary domain may further include (X)ₙ, resulting in 5'-(X)ₙUUUGUAGAU-3' (SEQ ID NO: 4). The X may be selected from the group consisting of nucleotides A, T, U and G, and the n may represent the number of nucleotide, which is an integer of 1 to 5. Here, the (X)ₙ may be an integer n repeats of the same nucleotide, or an integer n number of nucleotide sequences in which nucleotides of A, T, U and G are mixed.

Here, the linker domain may be a nucleotide sequence connecting a first complementary domain with a second complementary domain.

The linker domain may form a covalent or non-covalent bonding with a first complementary domain and a second complementary domain, respectively.

The linker domain may connect the first complementary domain with the second complementary domain covalently or non-covalently.

The linker domain is suitable to be used in a single-stranded gRNA molecule, and may be used to produce single-stranded gRNA by being connected with a first strand and a second strand of double-stranded gRNA or connecting the first strand with the second strand by covalent or non-covalent bonding.

The linker domain may be used to produce single-stranded gRNA by being connected with crRNA and tracrRNA of double-stranded gRNA or connecting the crRNA with the tracrRNA by covalent or non-covalent bonding.

Here, the second complementary domain may have homology with a natural second complementary domain, or may be derived from the natural second complementary domain. In addition, the second complementary domain may have a difference in nucleotide sequence of a second complementary domain according to a species existing in nature, and may be derived from a second complementary domain contained in the species existing in nature, or may have partial or complete homology with the second complementary domain contained in the species existing in nature.

In an exemplary embodiment, the second complementary domain may have partial, that is, at least 50% or more, or complete homology with a second complementary domain of *Streptococcus pyogenes, Campylobacter jejuni, Streptococcus thermophilus, Staphylococcus aureus* or *Neisseria meningitides,* or a second complementary domain derived therefrom.

For example, when the second complementary domain is a second complementary domain of *Streptococcus pyogenes* or a second complementary domain derived therefrom, the second complementary domain may be 5'-UAGCAAGUUAAAAU-3' (SEQ ID NO: 5), or a nucleotide sequence having partial, that is, at least 50% or more homology with 5'-UAGCAAGUUAAAAU-3' (SEQ ID NO: 5) (a nucleotide sequence forming a double strand with the first complementary domain is underlined). Here, the second complementary domain may further include (X)ₙ and/or (X)ₘ, resulting in 5'-(X)ₙUAGCAAGUUAAAAU(X)ₘ₋3' (SEQ ID NO: 5). The X may be selected from the group consisting of nucleotides A, T, U and G, and each of the n and m may represent the number of nucleotide, in which the n may be an integer of 1 to 15, and the m may be an integer of 1 to 6. Here, the (X)ₙ may be an integer n repeats of the same nucleotide, or an integer n number of nucleotide sequences in which nucleotides of A, T, U and G are mixed. In addition, the (X)ₘ may be an integer m repeats of the same nucleotide, or an integer m number of nucleotide sequences in which nucleotides of A, T, U and G are mixed.

In another example, when the second complementary domain is the second complementary domain of *Campylobacter jejuni* or a second complementary domain derived therefrom, the second complementary domain may be 5'-AAGAAAUUUAAAAAGGGACUAAAAU-3' (SEQ ID NO: 6) or 5'-AAGGGACUAAAAU-3'(SEQ ID NO: 7), or a nucleotide sequence having partial, that is, at least 50% or more homology with 5'-AAGAAAUUUAAAAAGGGACUAAAAU -3'(SEQ ID NO: 6) or 5'-AAGGGACUAAAAU-3'(SEQ ID NO: 7) (a nucleotide sequence forming a double strand with the first complementary domain is underlined). Here, the second complementary domain may further include (X)ₙ and/or (X)ₘ, resulting in 5'-(X)ₙAAGAAAUUUAAAAAGGGACUAAAAU(X)ₘ-3' (SEQ ID NO: 6) or 5'-(X)ₙAAGGGACUAAAAU(X)ₘ₋3'(SEQ ID NO: 7). The X may be selected from the group consisting of nucleotides A, T, U and G, in which the n may be an integer of 1 to 15, and the m may be an integer of 1 to 6. Here, the (X)ₙ may be an integer n repeats of the same nucleotide, or an integer n number of nucleotide sequences in which nucleotides of A, T, U and G are mixed. In addition, the (X)ₘ may be an integer m repeats of the same nucleotide, or an integer m number of nucleotide sequences in which nucleotides of A, T, U and G are mixed.

In another embodiment, the second complementary domain may have partial, that is, at least 50% or more, or complete homology with a second complementary domain of *Parcubacteria bacterium* (GWC2011_GWC2_44_17), *Lachnospiraceae bacterium* (MC2017), *Butyrivibrio proteoclasiicus, Peregrinibacteria bacterium* (GW2011_GWA_33_10), *Acidaminococcus sp.* (BV3L6), *Porphyromonas macacae, Lachnospiraceae bacterium* (ND2006), *Porphyromonas crevioricanis, Prevotella disiens, Moraxella bovoculi* (237), *Smiihella sp.* (SC_KP8D17), *Leptospira inadai, Lachnospiraceae bacterium* (MA2020), *Francisella novicida* (U112), *Candidatus Methanoplasma termitum* or *Eubacterium eligens,* or a second complementary domain derived therefrom.

For example, when the second complementary domain is a second complementary domain of *Parcubacteria bacterium* or a second complementary domain derived therefrom, the second complementary domain may be 5'-AAAUUUCUACU-3' (SEQ ID NO: 8), or a nucleotide sequence having partial, that is, at least 50% or more homology with 5'-AAAUUUCUACU-3' (SEQ ID NO: 8) (a nucleotide sequence forming a double strand with the first complementary domain is underlined). Here, the second complementary domain may further include (X)ₙ and/or (X)ₘ, resulting in 5'-(X)ₙAAAUUUCUACU(X)ₘ-3' (SEQ ID NO: 8). The X may be selected from the group consisting of nucleotides A, T, U and G, and each of the n and m may represent the number of nucleotides sequences, in which the n may be an integer of 1 to 10, and the m may be an integer of 1 to 6. Here, the (X)ₙ may be an integer n repeats of the same nucleotide, or an integer n number of nucleotide sequences in which nucleotides of A, T, U and G are mixed. In addition, the (X)ₘ may be an integer m repeats of the same nucleotide, or an integer m number of nucleotide sequences in which nucleotides of A, T, U and G are mixed.

Here, the first complementary domain and the second complementary domain may complementarily bind to each other.

The first complementary domain and the second complementary domain may form a double strand by the complementary binding.

The formed double strand may interact with a CRISPR enzyme.

Optionally, the first complementary domain may include an additional nucleotide sequence that does not complementarily bind to a second complementary domain of a second strand.

Here, the additional nucleotide sequence may be a sequence of 1 to 15 nucleotides. For example, the additional nucleotide sequence may be a sequence of 1 to 5, 5 to 10 or 10 to 15 nucleotides.

Here, the proximal domain may be a domain located at the 3'end direction of the second complementary domain.

The proximal domain may have homology with a natural proximal domain, or may be derived from the natural proximal domain. In addition, the proximal domain may have a difference in nucleotide sequence according to a species existing in nature, may be derived from a proximal domain contained in the species existing in nature, or may have partial or complete homology with the proximal domain contained in the species existing in nature.

In an exemplary embodiment, the proximal domain may have partial, that is, at least 50% or more, or complete homology with a proximal domain of *Streptococcus pyogenes, Campylobacter jejuni, Streptococcus thermophilus, Staphylococcus aureus* or *Neisseria meningitides,* or a proximal domain derived therefrom.

For example, when the proximal domain is a proximal domain of *Streptococcus pyogenes* or a proximal domain derived therefrom, the proximal domain may be 5'-AAGGCUAGUCCG-3' (SEQ ID NO: 9), or a nucleotide sequence having partial, that is, at least 50% or more homology with 5'-AAGGCUAGUCCG-3' (SEQ ID NO: 9). Here, the proximal domain may further include (X)ₙ, resulting in 5'-AAGGCUAGUCCG(X)ₙ-3' (SEQ ID NO: 9). The X may be selected from the group consisting of nucleotides A, T, U and G, and the n may represent the number of nucleotide, which is an integer of 1 to 15. Here, the (X)ₙ may be an integer n repeats of the same nucleotide, or an integer n number of nucleotide sequences in which nucleotides of A, T, U and G are mixed.

In yet another example, when the proximal domain is a proximal domain of *Campylobacter jejuni* or a proximal domain derived therefrom, the proximal domain may be 5'-AAAGAGUUUGC-3' (SEQ ID NO: 10), or a nucleotide sequence having at least 50% or more homology with 5'-AAAGAGUUUGC-3' (SEQ ID NO: 10). Here, the proximal domain may further include (X)ₙ, resulting in 5'-AAAGAGUUUGC(X)ₙ-3' (SEQ ID NO: 10). The X may be selected from the group consisting of nucleotides A, T, U and G, and the n may represent the number of nucleotide, which is an integer of 1 to 40. Here, the (X)ₙ may be an integer n repeats of the same nucleotide, or an integer n number of nucleotide sequences in which nucleotides of A, T, U and G are mixed.

Here, the tail domain is a domain which is able to be selectively added to the 3' end of single-stranded gRNA or the first or second strand of double-stranded gRNA.

The tail domain may have homology with a natural tail domain, or may be derived from the natural tail domain. In addition, the tail domain may have a difference in nucleotide sequence according to a species existing in nature, may be derived from a tail domain contained in a species existing in nature, or may have partial or complete homology with a tail domain contained in a species existing in nature.

In one exemplary embodiment, the tail domain may have partial, that is, at least 50% or more, or complete homology with a tail domain of *Streptococcus pyogenes, Campylobacter jejuni, Streptococcus thermophilus, Staphylococcus aureus* or *Neisseria meningitides* or a tail domain derived therefrom.

For example, when the tail domain is a tail domain of *Streptococcus pyogenes* or a tail domain derived therefrom, the tail domain may be 5'-UUAUCAACUUGAAAAAGUGGCACCGAGUCGGUGC-3' (SEQ ID NO: 11), or a nucleotide sequence having partial, that is, at least 50% or more homology with 5'-UUAUCAACUUGAAAAAGUGGCACCGAGUCGGUGC-3' (SEQ ID NO: 11). Here, the tail domain may further include (X)ₙ, resulting in 5'-UUAUCAACUUGAAAAAGUGGCACCGAGUCGGUGC(X)ₙ₋3' (SEQ ID NO: 11). The X may be selected from the group consisting of nucleotides A, T, U and G, and the n may represent the number of nucleotide, which is an integer of 1 to 15. Here, the (X)ₙ may be an integer n repeats of the same nucleotide, or an integer n number of nucleotide sequences in which nucleotides of A, T, U and G are mixed.

In another example, when the tail domain is a tail domain of *Campylobacter jejuni* or a tail domain derived therefrom, the tail domain may be 5'-GGGACUCUGCGGGGUUACAAUCCCCUAAAACCGCUUUU-3' (SEQ ID NO: 12), or a nucleotide sequence having partial, that is, at least 50% or more homology with 5'-GGGACUCUGCGGGGUUACAAUCCCCUAAAACCGCUUUU-3' (SEQ ID NO: 12). Here, the tail domain may further include (X)ₙ, resulting in 5'-GGGACUCUGCGGGGUUACAAUCCCCUAAAACCGCUUUU(X)ₙ₋3' (SEQ ID NO: 12). The X may be selected from the group consisting of nucleotides A, T, U and G, and the n may represent the number of nucleotide, which is an integer of 1 to 15. Here, the (X)ₙ may be an integer n repeats of the same nucleotide, or an integer n number of nucleotide sequences in which nucleotides of A, T, U and G are mixed.

In another embodiment, the tail domain may include a 1 to 10-nt sequence at the 3' end involved in an in vitro or in vivo transcription method.

For example, when a T7 promoter is used in *in vitro* transcription of gRNA, the tail domain may be an arbitrary nucleotide sequence present at the 3' end of a DNA template. In addition, when a U6 promoter is used in *in vivo* transcription, the tail domain may be UUUUUU, when an HI promoter is used in transcription, the tail domain may be UUUU, and when a pol-III promoter is used, the tail domain may be several uracil nucleotides or may include alternative nucleotides.

The gRNA may include a plurality of domains as described above, and therefore, the length of the nucleic acid sequence may be regulated according to a domain contained in the gRNA, and interactions may occur in strands in a three-dimensional structure or active form of gRNA or between theses strands due to each domain.

The gRNA may be referred to as single-stranded gRNA (single RNA molecule); or double-stranded gRNA (including more than one, generally two discrete RNA molecules).

### Double-stranded gRNA

The double-stranded gRNA consists of a first strand and a second strand.

Here, the first strand may consist of
5'-[guide domain]-[first complementary domain]-3', and
the second strand may consist of
5'-[second complementary domain]-[proximal domain]-3' or
5'-[second complementary domain]-[proximal domain]-[tail domain]-3'.

Here, the first strand may be referred to as crRNA, and the second strand may be referred to as tracrRNA.

Here, the first strand and the second strand may optionally include an additional nucleotide sequence.

In one embodiment, the first strand may be
5'-(N_{target})-(Q)ₘ-3'; or
5'-(X)ₐ-(N_{target})-(X)_{b}-(Q)ₘ-(X)_{c}-3'.

Here, the N_{target} is a nucleotide sequence complementary to partial sequence of either strand of a double strand of a target gene or a nucleic acid, and a nucleotide sequence region which may be changed according to a target sequence on a target gene or a nucleic acid.

Here, the (Q)ₘ is a nucleotide sequence including a first complementary domain. It includes a nucleotide sequence which is able to form a complementary bond with the second complementary domain of the second strand. The (Q)ₘ may be a sequence having partial or complete homology with the first complementary domain of a species existing in nature, and the nucleotide sequence of the first complementary domain may be changed according to the species of origin. The Q may be each independently selected from the group consisting of A, U, C and G, and the m may be the number of nucleotide sequences, which is an integer of 5 to 35.

For example, when the first complementary domain has partial or complete homology with a first complementary domain of *Streptococcus pyogenes* or a *Streptococcus pyogenes-*derived first complementary domain, the (Q)ₘ may be 5'-GUUUUAGAGCUA-3' (SEQ ID NO: 1), or a nucleotide sequence having at least 50% or more homology with 5'-GUUUUAGAGCUA-3' (SEQ ID NO: 1).

In another example, when the first complementary domain has partial or complete homology with a first complementary domain of *Campylobacter jejuni* or a *Campylobacter jejuni-*derived first complementary domain, the (Q)ₘ may be 5'-GUUUUAGUCCCUUUUUAAAUUUCUU-3' (SEQ ID NO: 2) or 5'-GUUUUAGUCCCUU-3'(SEQ ID NO: 3), or a nucleotide sequence having at least 50% or more homology with 5'-GUUUUAGUCCCUUUUUAAAUUUCUU-3' (SEQ ID NO: 2) or 5'-GUUUUAGUCCCUU-3'(SEQ ID NO: 3).

In still another example, when the first complementary domain has partial or complete homology with a first complementary domain of *Streptococcus thermophilus* or a *Streptococcus thermophilus-*derived first complementary domain, the (Q)ₘ may be 5'-GUUUUAGAGCUGUGUUGUUUCG-3' (SEQ ID NO: 13), or a nucleotide sequence having at least 50% or more homology with 5'-GUUUUAGAGCUGUGUUGUUUCG-3' (SEQ ID NO: 13).

In addition, each of the (X)ₐ, (X)_{b} and (X)_{c} is selectively an additional nucleotide sequence, where the X may be each independently selected from the group consisting of A, U, C and G, and each of the a, b and c may be the number of nucleotide sequences, which is 0 or an integer of 1 to 20.

In one exemplary embodiment, the second strand may be 5'-(Z)ₕ-(P)ₖ-3'; or 5'-(X)_{d}-(Z)ₕ-(X)ₑ-(P)ₖ-(X)_{f}-3'.

In another embodiment, the second strand may be 5'-(Z)ₕ-(P)ₖ-(F)ᵢ-3'; or 5'-(X)_{d}-(Z)ₕ-(X)ₑ-(P)ₖ-(X)_{f}-(F)ᵢ-3'.

Here, the (Z)ₕ is a nucleotide sequence including a second complementary domain. It includes a nucleotide sequence which is able to form a complementary bond with the first complementary domain of the first strand. The (Z)ₕ may be a sequence having partial or complete homology with the second complementary domain of a species existing in nature, and the nucleotide sequence of the second complementary domain may be modified according to the species of origin. The Z may be each independently selected from the group consisting of A, U, C and G, and the h may be the number of nucleotide sequences, which is an integer of 5 to 50.

For example, when the second complementary domain has partial or complete homology with a second complementary domain of *Streptococcus pyogenes* or a second complementary domain derived therefrom, the (Z)ₕ may be 5'-UAGCAAGUUAAAAU-3' (SEQ ID NO: 5), or a nucleotide sequence having at least 50% or more homology with 5'-UAGCAAGUUAAAAU-3' (SEQ ID NO: 5).

In another example, when the second complementary domain has partial or complete homology with a second complementary domain of *Campylobacter jejuni* or a second complementary domain derived therefrom, the (Z)ₕ may be 5'-AAGAAAUUUAAAAAGGGACUAAAAU-3' (SEQ ID NO: 6) or 5'-AAGGGACUAAAAU-3'(SEQ ID NO: 7), or a nucleotide sequence having at least 50% or more homology with 5'-AAGAAAUUUAAAAAGGGACUAAAAU-3' or 5'-AAGGGACUAAAAU-3'(SEQ ID NO: 7). In still another example, when the second complementary domain has partial or complete homology with a second complementary domain of *Streptococcus thermophilus* or a second complementary domain derived therefrom, the (Z)ₕ may be 5'-CGAAACAACACAGCGAGUUAAAAU-3' (SEQ ID NO: 14), or a nucleotide sequence having at least 50% or more homology with 5'-CGAAACAACACAGCGAGUUAAAAU-3' (SEQ ID NO: 14).

The (P)ₖ is a nucleotide sequence including a proximal domain. It may be a sequence having partial or complete homology with a proximal domain of a species existing in nature, and the nucleotide sequence of the proximal domain may be modified according to the species of origin. The P may be each independently selected from the group consisting of A, U, C and G, and the k may be the number of nucleotide sequences, which is an integer of 1 to 20.

For example, when the proximal domain has partial or complete homology with a proximal domain of *Streptococcus pyogenes* or a proximal domain derived therefrom, the (P)ₖ may be 5'-AAGGCUAGUCCG-3' (SEQ ID NO: 9), or a nucleotide sequence having at least 50% or more homology with 5'-AAGGCUAGUCCG-3' (SEQ ID NO: 9).

In another example, when the proximal domain has partial or complete homology with a proximal domain of *Campylobacter jejuni* or a proximal domain derived therefrom, the (P)ₖ may be 5'-AAAGAGUUUGC-3' (SEQ ID NO: 10), or a nucleotide sequence having at least 50% or more homology with 5'-AAAGAGUUUGC-3' (SEQ ID NO: 10).

In still another example, when the proximal domain has partial or complete homology with a proximal domain of *Streptococcus thermophilus* or a proximal domain derived therefrom, the (P)ₖ may be 5'-AAGGCUUAGUCCG-3' (SEQ ID NO: 15), or a nucleotide sequence having at least 50% or more homology with 5'-AAGGCUUAGUCCG-3' (SEQ ID NO: 15).

The (F)ᵢ may be a nucleotide sequence including a tail domain. It may be a sequence having partial or complete homology with a tail domain of a species existing in nature, and the nucleotide sequence of the tail domain may be modified according to the species of origin. The F may be each independently selected from the group consisting of A, U, C and G, and the i may be the number of nucleotide sequences, which is an integer of 1 to 50.

For example, when the tail domain has partial or complete homology with a tail domain of *Streptococcus pyogenes* or a tail domain derived therefrom, the (F)ᵢ may be 5'-UUAUCAACUUGAAAAAGUGGCACCGAGUCGGUGC-3' (SEQ ID NO: 11), or a nucleotide sequence having at least 50% or more homology with 5'-UUAUCAACUUGAAAAAGUGGCACCGAGUCGGUGC-3' (SEQ ID NO: 11).

In another example, when the tail domain has partial or complete homology with a tail domain of *Campylobacter jejuni* or a tail domain derived therefrom, the (F)ᵢ may be 5'-GGGACUCUGCGGGGUUACAAUCCCCUAAAACCGCUUUU-3' (SEQ ID NO: 12), or a nucleotide sequence having at least 50% or more homology with 5'-GGGACUCUGCGGGGUUACAAUCCCCUAAAACCGCUUUU-3' (SEQ ID NO: 12).

In still another example, when the tail domain has partial or complete homology with a tail domain of *Streptococcus thermophilus* or a tail domain derived therefrom, the (F)ᵢ may be 5'-UACUCAACUUGAAAAGGUGGCACCGAUUCGGUGUUUUU-3' (SEQ ID NO: 16), or a nucleotide sequence having at least 50% or more homology with 5'-UACUCAACUUGAAAAGGUGGCACCGAUUCGGUGUUUUU-3' (SEQ ID NO: 16).

In addition, the (F)ᵢ may include a sequence of 1 to 10 nucleotides at the 3' end involved in an *in vitro* or *in vivo* transcription method.

For example, when a T7 promoter is used in *in vitro* transcription of gRNA, the tail domain may be an arbitrary nucleotide sequence present at the 3' end of a DNA template. In addition, when a U6 promoter is used in *in vivo* transcription, the tail domain may be UUUUUU, when an HI promoter is used in transcription, the tail domain may be UUUU, and when a pol-III promoter is used, the tail domain may be several uracil nucleotides or may include alternative nucleotides.

In addition, the (X)_{d}, (X)ₑ and (X)_{f} may be nucleotide sequences selectively added, where the X may be each independently selected from the group consisting of A, U, C and G, and each of the d, e and f may be the number of nucleotides, which is 0 or an integer of 1 to 20.

### Single-stranded gRNA

Single-stranded gRNA may be classified into a first single-stranded gRNA and a second single-stranded gRNA.

### First single-stranded gRNA

First single-stranded gRNA is single-stranded gRNA in which a first strand and a second strand of the double-stranded gRNA is linked by a linker domain.

Specifically, the single-stranded gRNA may consist of
5'-[guide domain]-[first complementary domain]-[linker domain]-[second complementary domain]-3',
5'-[guide domain]-[first complementary domain]-[linker domain]-[second complementary domain]-[proximal domain]-3' or
5'-[guide domain]-[first complementary domain]-[linker domain]-[second complementary domain]-[proximal domain]-[tail domain]-3'.

The first single-stranded gRNA may selectively include an additional nucleotide sequence.

In one exemplary embodiment, the first single-stranded gRNA may be
5'-(N_{target})-(Q)ₘ-(L)ⱼ-(Z)ₕ-3';
5'-(N_{target})-(Q)ₘ-(_{L})ⱼ-(Z)ₕ-(P)ₖ-3'; or
5'-(N_{target})-(Q)ₘ-(L)ⱼ-(Z)ₕ-(P)ₖ-(F)ᵢ-3'.

In another embodiment, the single-stranded gRNA may be
5'-(X)ₐ-(N_{target})-(X)_{b}-(Q)ₘ-(X)_{c}-(L)ⱼ-(X)_{d}-(Z)ₕ-(X)ₑ-3';
5'-(X)ₐ-(N_{target})-(X)_{b}-(Q)ₘ-(X)_{c}-(L)ⱼ-(X)_{d}-(Z)ₕ-(X)ₑ-(P)ₖ-(X)_{f}-3'; ₒr
5'-(X)ₐ-(N_{target})-(X)_{b}-(Q)ₘ-(X)_{c}-(L)ⱼ-(X)_{d}-(Z)ₕ-(X)ₑ-(P)ₖ-(X)_{f}-(F)ᵢ-3'.

Here, the N_{target} is a nucleotide sequence complementary to partial sequence of either strand of a double strand of a target gene or a nucleic acid, and a nucleotide sequence region capable of being changed according to a target sequence on a target gene or a nucleic acid.

The (Q)ₘ includes a nucleotide sequence including the first complementary domain. It includes a nucleotide sequence which is able to form a complementary bond with a second complementary domain. The (Q)ₘ may be a sequence having partial or complete homology with a first complementary domain of a species existing in nature, and the nucleotide sequence of the first complementary domain may be changed according to the species of origin. The Q may be each independently selected from the group consisting of A, U, C and G, and the m may be the number of nucleotide sequences, which is an integer of 5 to 35.

For example, when the first complementary domain has partial or complete homology with a first complementary domain of *Streptococcus pyogenes* or a first complementary domain derived therefrom, the (Q)ₘ may be 5'-GUUUUAGAGCUA-3' (SEQ ID NO: 1), or a nucleotide sequence having at least 50% or more homology with 5'-GUUUUAGAGCUA-3' (SEQ ID NO: 1).

In another example, when the first complementary domain has partial or complete homology with a first complementary domain of *Campylobacter jejuni* or a first complementary domain derived therefrom, the (Q)ₘ may be 5'-GUUUUAGUCCCUUUUUAAAUUUCUU-3' (SEQ ID NO: 2) or 5'-GUUUUAGUCCCUU-3'(SEQ ID NO: 3), or a nucleotide sequence having at least 50% or more homology with 5'-GUUUUAGUCCCUUUUUAAAUUUCUU-3' (SEQ ID NO: 2) or 5'-GUUUUAGUCCCUU-3'(SEQ ID NO: 3).

In still another example, when the first complementary domain has partial or complete homology with a first complementary domain of *Streptococcus thermophilus* or a first complementary domain derived therefrom, the (Q)ₘ may be 5'-GUUUUAGAGCUGUGUUGUUUCG-3' (SEQ ID NO: 13), or a nucleotide sequence having at least 50% or more homology with 5'-GUUUUAGAGCUGUGUUGUUUCG-3' (SEQ ID NO: 13).

In addition, the (L)ⱼ is a nucleotide sequence including the linker domain, and connecting the first complementary domain with the second complementary domain, thereby producing single-stranded gRNA. Here, the L may be each independently selected from the group consisting of A, U, C and G, and the j may be the number of nucleotide sequences, which is an integer of 1 to 30.

The (Z)ₕ is a nucleotide sequence including the second complementary domain, and includes a nucleotide sequence capable of complementary binding with the first complementary domain. The (Z)ₕ may be a sequence having partial or complete homology with the second complementary domain of a species existing in nature, and the nucleotide sequence of the second complementary domain may be changed according to the species of origin. The Z may be each independently selected from the group consisting of A, U, C and G, and the h is the number of nucleotide sequences, which may be an integer of 5 to 50.

For example, when the second complementary domain has partial or complete homology with a second complementary domain of *Streptococcus pyogenes* or a second complementary domain derived therefrom, the (Z)ₕ may be 5'-UAGCAAGUUAAAAU-3' (SEQ ID NO: 5), or a nucleotide sequence having at least 50% or more homology with 5'-UAGCAAGUUAAAAU-3' (SEQ ID NO: 5).

In another example, when the second complementary domain has partial or complete homology with a second complementary domain of *Campylobacter jejuni* or a second complementary domain derived therefrom, the (Z)ₕ may be 5'-AAGAAAUUUAAAAAGGGACUAAAAU-3' (SEQ ID NO: 6) or 5'-AAGGGACUAAAAU-3'(SEQ ID NO: 7), or a nucleotide sequence having at least 50% or more homology with 5'-AAGAAAUUUAAAAAGGGACUAAAAU-3' (SEQ ID NO: 6) or 5'-AAGGGACUAAAAU-3'(SEQ ID NO: 7).

In still another example, when the second complementary domain has partial or complete homology with a second complementary domain of *Streptococcus thermophilus* or a second complementary domain derived therefrom, the (Z)ₕ may be 5'-CGAAACAACACAGCGAGUUAAAAU-3' (SEQ ID NO: 14), or a nucleotide sequence having at least 50% or more homology with 5'-CGAAACAACACAGCGAGUUAAAAU-3' (SEQ ID NO: 14).

The (P)ₖ is a nucleotide sequence including a proximal domain. It may be a sequence having partial or complete homology with a proximal domain of a species existing in nature, and the nucleotide sequence of the proximal domain may be modified according to the species of origin. The P may be each independently selected from the group consisting of A, U, C and G, and the k may be the number of nucleotide sequences, which is an integer of 1 to 20.

For example, when the proximal domain has partial or complete homology with a proximal domain of *Streptococcus pyogenes* or a proximal domain derived therefrom, the (P)ₖ may be 5'-AAGGCUAGUCCG-3' (SEQ ID NO: 9), or a nucleotide sequence having at least 50% or more homology with 5'-AAGGCUAGUCCG-3' (SEQ ID NO: 9).

In another example, when the proximal domain has partial or complete homology with a proximal domain of *Campylobacter jejuni* or a proximal domain derived therefrom, the (P)ₖ may be 5'-AAAGAGUUUGC-3' (SEQ ID NO: 10), or a nucleotide sequence having at least 50% or more homology with 5'-AAAGAGUUUGC-3' (SEQ ID NO: 10).

In still another example, when the proximal domain has partial or complete homology with a proximal domain of *Streptococcus thermophilus* or a proximal domain derived therefrom, the (P)ₖ may be 5'-AAGGCUUAGUCCG-3' (SEQ ID NO: 15), or a nucleotide sequence having at least 50% or more homology with 5'-AAGGCUUAGUCCG-3' (SEQ ID NO: 15).

The (F)ᵢ may be a nucleotide sequence including a tail domain, and having partial or complete homology with a tail domain of a species existing in nature, and the nucleotide sequence of the tail domain may be modified according to the species of origin. The F may be each independently selected from the group consisting of A, U, C and G, and the i may be the number of nucleotide sequences, which is an integer of 1 to 50.

For example, when the tail domain has partial or complete homology with a tail domain of *Streptococcus pyogenes* or a tail domain derived therefrom, the (F)ᵢ may be 5'-UUAUCAACUUGAAAAAGUGGCACCGAGUCGGUGC-3' (SEQ ID NO: 11), or a nucleotide sequence having at least 50% or more homology with 5'-UUAUCAACUUGAAAAAGUGGCACCGAGUCGGUGC-3' (SEQ ID NO: 11).

In another example, when the tail domain has partial or complete homology with a tail domain of *Campylobacter jejuni* or a tail domain derived therefrom, the (F)ᵢ may be 5'-GGGACUCUGCGGGGUUACAAUCCCCUAAAACCGCUUUU-3' (SEQ ID NO: 12), or a nucleotide sequence having at least 50% or more homology with 5'-GGGACUCUGCGGGGUUACAAUCCCCUAAAACCGCUUUU-3' (SEQ ID NO: 12).

In still another example, when the tail domain has partial or complete homology with a tail domain of *Streptococcus thermophilus* or a tail domain derived therefrom, the (F)ᵢ may be 5'-UACUCAACUUGAAAAGGUGGCACCGAUUCGGUGUUUUU-3' (SEQ ID NO: 16), or a nucleotide sequence having at least 50% or more homology with 5'-UACUCAACUUGAAAAGGUGGCACCGAUUCGGUGUUUUU-3' (SEQ ID NO: 16).

In addition, the (F)ᵢ may include a sequence of 1 to 10 nucleotides at the 3' end involved in an *in vitro* or *in vivo* transcription method.

For example, when a T7 promoter is used in *in vitro* transcription of gRNA, the tail domain may be an arbitrary nucleotide sequence present at the 3' end of a DNA template. In addition, when a U6 promoter is used in *in vivo* transcription, the tail domain may be UUUUUU, when an HI promoter is used in transcription, the tail domain may be UUUU, and when a pol-III promoter is used, the tail domain may be several uracil nucleotides or may include alternative nucleotides.

In addition, the (X)ₐ, (X)_{b}, (X)_{c}, (X)_{d}, (X)ₑ and (X)_{f} may be nucleotide sequences selectively added, where the X may be each independently selected from the group consisting of A, U, C and G, and each of the a, b, c, d, e and f may be the number of nucleotide sequences, which is 0 or an integer of 1 to 20.

### Second single-stranded gRNA

Second single-stranded gRNA may be single-stranded gRNA consisting of a guide domain, a first complementary domain and a second complementary domain.

Here, the second single-stranded gRNA may consist of:
5'-[second complementary domain]-[first complementary domain]-[guide domain]-3'; or
5'-[second complementary domain]-[linker domain]-[first complementary domain]-[guide domain]-3'.

The second single-stranded gRNA may selectively include an additional nucleotide sequence.

In one exemplary embodiment, the second single-stranded gRNA may be
5'-(Z)ₕ-(Q)ₘ-(N_{target})-3'; or
5'-(X)ₐ-(Z)ₕ-(X)_{b}-(Q)ₘ-(X)_{c}-(N_{target})-3'.

In another embodiment, the single-stranded gRNA may be
5'-(Z)ₕ-(L)ⱼ-(Q)ₘ-(N_{target})-3'; or
5'-(X)ₐ-(Z)ₕ-(L)ⱼ-(Q)ₘ-(X)_{c}-(N_{target})-3'.

Here, the N_{target} is a nucleotide sequence complementary to partial sequence of either strand of a double strand of a target gene or a nucleic acid, and a nucleotide sequence region capable of being changed according to a target sequence on a target gene or a nucleic acid.

The (Q)ₘ is a nucleotide sequence including the first complementary domain, and includes a nucleotide sequence capable of complementary binding with a second complementary domain. The (Q)ₘ may be a sequence having partial or complete homology with the first complementary domain of a species existing in nature, and the nucleotide sequence of the first complementary domain may be changed according to the species of origin. The Q may be each independently selected from the group consisting of A, U, C and G, and the m may be the number of nucleotide sequences, which is an integer of 5 to 35.

For example, when the first complementary domain has partial or complete homology with a first complementary domain of *Parcubacteria bacterium* or a first complementary domain derived therefrom, the (Q)ₘ may be 5'-UUUGUAGAU-3' (SEQ ID NO: 4), or a nucleotide sequence having at least 50% or more homology with 5'-UUUGUAGAU-3' (SEQ ID NO: 4).

The (Z)ₕ is a nucleotide sequence including a second complementary domain, and includes a nucleotide sequence capable of complementary binding with a second complementary domain. The (Z)ₕ may be a sequence having partial or complete homology with the second complementary domain of a species existing in nature, and the nucleotide sequence of the second complementary domain may be modified according to the species of origin. The Z may be each independently selected from the group consisting of A, U, C and G, and the h may be the number of nucleotide sequences, which is an integer of 5 to 50.

For example, when the second complementary domain has partial or complete homology with a second complementary domain of *Parcubacteria bacterium* or a *Parcubacteria bacterium-*derived second complementary domain, the (Z)ₕ may be 5'-AAAUUUCUACU-3' (SEQ ID NO: 8), or a nucleotide sequence having at least 50% or more homology with 5'-AAAUUUCUACU-3' (SEQ ID NO: 8).

In addition, the (L)ⱼ is a nucleotide sequence including the linker domain. It is a nucleotide sequence connecting the first complementary domain with the second complementary domain. Here, the L may be each independently selected from the group consisting of A, U, C and G, and the j may be the number of nucleotide sequences, which is an integer of 1 to 30.

In addition, each of the (X)ₐ, (X)_{b} and (X)_{c} may be nucleotide selectively added, where the X may be each independently selected from the group consisting of A, U, C and G, and the a, b and c may be the number of nucleotide, which is 0 or an integer of 1 to 20.

### In one aspect of the disclosure in the present specification, the guide nucleic acid may be gRNA capable of complementarily binding to a target sequence of a blood coagulation inhibitory gene.

The term "blood coagulation inhibitory gene" refers to all types of genes that directly participate in or have an indirect effect of inhibiting blood coagulation or the formation of blood clots or inactivating a blood coagulation system. In this case, the blood coagulation inhibitory gene may function to inhibit or suppress the activities of various genes or various proteins which are involved in the blood coagulation system due to the blood coagulation inhibitory gene itself or a protein expressed by the blood coagulation inhibitory gene and function to directly inhibit blood coagulation. The term "blood coagulation system" refers to the overall process of coagulating blood, which occurs *in vivo.* In this case, the blood coagulation system includes all types of normal blood coagulation systems and abnormal blood coagulation systems in which blood coagulation is delayed. A protein expressed by the blood coagulation inhibitory gene may be used interchangeably with the term "blood coagulation inhibitory factor" or "anticoagulant factor."

The blood coagulation inhibitory gene may inhibit or suppress the blood coagulation.

The blood coagulation inhibitory gene may inhibit or suppress the expression of blood coagulation-associated proteins.

The blood coagulation inhibitory gene may inhibit or suppress the activities of the blood coagulation-associated proteins.

In this case, the blood coagulation-associated proteins may include factor XII, factor XIIa, factor XI, factor XIa, factor IX, factor IXa, factor X, factor Xa, factor VIII, factor VIIIa, factor VII, factor VIIa, factor V, factor Va, prothrombin, thrombin, factor XIII, factor XIIIa, fibrinogen, fibrin, or a tissue factor.

The blood coagulation inhibitory gene may be an antithrombin (AT) gene.

The AT gene refers to a gene that encodes a protein that consisting of 432 amino acids, which can inactivate various enzymes in the blood coagulation system, and is also referred to as a SERPINC 1 gene. As one example, the AT gene may include one or more selected from the group consisting of the following, but the present invention is not limited thereto: genes encoding human ATs (e.g., NCBI Accession No. NP_000479, and the like), for example, AT genes represented by NCBI Accession No. NM_000488, and the like.

The blood coagulation inhibitory gene may be a tissue factor pathway inhibitor (TFPI).

The TFPI gene refers to a gene (full-length DNA, cDNA, or mRNA) that encodes a protein capable of reversibly suppressing factor Xa. In the case of a human being, the TFPI gene is located on chromosomes 2q31-q32.1, and has 9 exons. As one example, the TFPI gene may include one or more selected from the group consisting of the following, but the present invention is not limited thereto: genes encoding human TFPIs (e.g., NCBI Accession Nos. NP_001027452, NP_001305870, NP_001316168, NP_001316169, NP_001316170, and the like), for example TFPI genes represented by NCBI Accession Nos. NM_001032281, NM_006287, NM_001318941, NM_001329239, NM_001329240, and the like.

The blood coagulation inhibitory gene may be derived from mammals, which include primates such as a human, a monkey, and the like, rodents such as a rat, a mouse, and the like.

The genetic information may be obtained from the known databases such as GenBank of NCBI (National Center for Biotechnology Information).

### In one embodimentof the disclosure in the present specification, the guide nucleic acid may be gRNA targeting a target sequence of a AT gene and/or a TFP1 gene.

The "target sequence" is a nucleotide sequence present in a target gene or nucleic acid, and specifically, a partial nucleotide sequence of a target region in a target gene or a nucleic acid, and here, the "target region" is a region that can be modified by a guide nucleic acid-editor protein in a target gene or a nucleic acid.

Hereinafter, the target sequence may be used to refer to both of two types of nucleotide sequence information. For example, in the case of a target gene, the target sequence may refer to the nucleotide sequence information of a transcribed strand of target gene DNA, or the nucleotide sequence information of a non-transcribed strand.

For example, the target sequence may refer to a partial nucleotide sequence (transcribed strand), that is, 5'-ATCATTGGCAGACTAGTTCG-3' (SEQ ID NO: 17), in the target region of target gene A, or a nucleotide sequence complementary thereto (non-transcribed strand), that is, 5'-CGAACTAGTCTGCCAATGAT-3' (SEQ ID NO: 18).

The target sequence may be a 5 to 50-nt sequence.

In one exemplary embodiment, the target sequence may be a 16-nt sequence, a 17-nt sequence, a 18-nt sequence, a 19-nt sequence, a 20-nt sequence, a 21-nt sequence, a 22-nt sequence, a 23-nt sequence, a 24-nt sequence or a 25-nt sequence.

The target sequence includes a guide nucleic acid-binding sequence or a guide nucleic acid-non binding sequence.

The "guide nucleic acid-binding sequence" is a nucleotide sequence having partial or complete complementarity with a guide sequence included in the guide domain of the guide nucleic acid, and may be complementarily bonded with the guide sequence included in the guide domain of the guide nucleic acid. The target sequence and guide nucleic acid-binding sequence are nucleotide sequences that may vary according to a target gene or a nucleic acid, that is, the subject to be genetically manipulated or edited, and may be designed variously according to a target gene or a nucleic acid.

The "guide nucleic acid-non binding sequence" is a nucleotide sequence having partial or complete homology with a guide sequence included in the guide domain of the guide nucleic acid, and may not be complementarily bonded with the guide sequence included in the guide domain of the guide nucleic acid. In addition, the guide nucleic acid-non binding sequence may be a nucleotide sequence having complementarity with the guide nucleic acid-binding sequence, and may be complementarily bonded with the guide nucleic acid-binding sequence.

The guide nucleic acid-binding sequence may be a partial nucleotide sequence of a target sequence, and one nucleotide sequence of two nucleotide sequences having different sequence order to each other the target sequence, that is, one of the two nucleotide sequences capable of complementary binding to each other. Here, the guide nucleic acid-non binding sequence may be a nucleotide sequence other than the guide nucleic acid-binding sequence of the target sequence.

For example, in a target region of the target gene A, when target sequences are designed as a partial nucleotide sequence, that is, 5'-ATCATTGGCAGACTAGTTCG-3' (SEQ ID NO: 17), and a complementary nucleotide sequence thereof, that is, 5'-CGAACTAGTCTGCCAATGAT-3' (SEQ ID NO: 18), the guide nucleic acid-binding sequence may be one of the two target sequences, that is, 5'-ATCATTGGCAGACTAGTTCG-3'(SEQ ID NO: 17) or 5'-CGAACTAGTCTGCCAATGAT-3'(SEQ ID NO: 18). Here, when the guide nucleic acid-binding sequence is 5'-ATCATTGGCAGACTAGTTCG-3'(SEQ ID NO: 17), the guide nucleic acid-non binding sequence may be 5'-CGAACTAGTCTGCCAATGAT-3' (SEQ ID NO: 18), or when the guide nucleic acid-binding sequence is 5'-CGAACTAGTCTGCCAATGAT-3' (SEQ ID NO: 18), the guide nucleic acid-non binding sequence may be 5'-ATCATTGGCAGACTAGTTCG-3' (SEQ ID NO: 17).

The guide nucleic acid-binding sequence may be one of the target sequences, that is, a nucleotide sequence which is the same as a transcribed strand and a nucleotide sequence which is the same as a non-transcribed strand. Here, the guide nucleic acid-non binding sequence may be a nucleotide sequence other than the guide nucleic acid-binding sequence of the target sequences. It may be the nucleotide sequence other than a nucleotide sequence which is the same as a transcribed strand or a non-transcribed strand.

The guide nucleic acid-binding sequence may have the same length as the target sequence.

The guide nucleic acid-non binding sequence may have the same length as the target sequence or the guide nucleic acid-binding sequence.

The guide nucleic acid-binding sequence may be a 5 to 50-nt sequence.

In one exemplary embodiment, the guide nucleic acid-binding sequence may be a 16-nt sequence, a 17-nt sequence, a 18-nt sequence, a 19-nt sequence, a 20-nt sequence, a 21-nt sequence, a 22-nt sequence, a 23-nt sequence, a 24-nt sequence or a 25-nt sequence.

The guide nucleic acid-non binding sequence may be a 5 to 50-nt sequence.

In one exemplary embodiment, the guide nucleic acid-non binding sequence may be a 16-nt sequence, a 17-nt sequence, a 18-nt sequence, a 19-nt sequence, a 20-nt sequence, a 21-nt sequence, a 22-nt sequence, a 23-nt sequence, a 24-nt sequence or a 25-nt sequence.

The guide nucleic acid-binding sequence may partially or completely complementarily bind to the guide sequence included in the guide domain of the guide nucleic acid, and the length of the guide nucleic acid-binding sequence may be the same as that of the guide sequence.

The guide nucleic acid-binding sequence may be a nucleotide sequence complementary to the guide sequence included in the guide domain of the guide nucleic acid, and for example, a nucleotide sequence which has at least 70%, 75%, 80%, 85%, 90%, 95% or more complementarity or complete complementarity.

As an example, the guide nucleic acid-binding sequence may have or include a 1 to 8-nt sequence which is not complementary to the guide sequence included in the guide domain of the guide nucleic acid.

The guide nucleic acid-non binding sequence may have partial or complete homology with the guide sequence included in the guide domain of the guide nucleic acid, and the length of the guide nucleic acid-non binding sequence may be the same as that of the guide sequence.

The guide nucleic acid-non binding sequence may be a nucleotide sequence having homology with the guide sequence included in the guide domain of the guide nucleic acid, and for example, a nucleotide sequence which has at least 70%, 75%, 80%, 85%, 90%, 95% or more homology or complete homology.

In one example, the guide nucleic acid-non binding sequence may have or include a 1 to 8-nt sequence which is not homologous to the guide sequence included in the guide domain of the guide nucleic acid.

The guide nucleic acid-non binding sequence may complementarily bind with the guide nucleic acid-binding sequence, and the guide nucleic acid-non binding sequence may have the same length as the guide nucleic acid-binding sequence.

The guide nucleic acid-non binding sequence may be a nucleotide sequence complementary to the guide nucleic acid-binding sequence, and for example, a nucleotide sequence having at least 90%, 95% or more complementarity or complete complementarity.

In one example, the guide nucleic acid-non binding sequence may have or include a 1 to 2-nt sequence which is not complementary to the guide nucleic acid-binding sequence.

In addition, the guide nucleic acid-binding sequence may be a nucleotide sequence located near a nucleotide sequence recognized by an editor protein.

In one example, the guide nucleic acid-binding sequence may be a consecutive 5 to 50-nt sequence located adjacent to the 5' end and/or 3' end of a nucleotide sequence recognized by an editor protein.

In addition, the guide nucleic acid-non binding sequence may be a nucleotide sequence located near a nucleotide sequence recognized by an editor protein.

In one example, the guide nucleic acid-non binding sequence may be a 5 to 50-nt contiguous sequence located adjacent to the 5' end and/or 3' end of a nucleotide sequence recognized by an editor protein.

The "targeting" refers to complementary binding with the guide nucleic acid-binding sequence of the target sequence present in a target gene or a nucleic acid. Here, the complementary binding may be 100% completely complementary binding, or 70% or more and less than 100%, incomplete complementary binding. Therefore, the "targeting gRNA" refers to gRNA complementarily binding to the guide nucleic acid-binding sequence of the target sequence present in a target gene or a nucleic acid.

The target gene disclosed in the specification may be a blood coagulation inhibitory gene.

The target gene disclosed in the specification may be a AT gene and/or TFPI gene.

In an embodiment,
the target sequence disclosed in the present specification may be a 10 to 35-nt contiguous sequence located in the promoter region of the blood coagulation inhibitory gene.

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence located in the promoter region of the AT gene.

In another example, the target sequence may be a 10 to 25 contiguous nucleotide sequence located in the promoter region of the TFPI gene.

The target sequence disclosed in the present specification may be a 10 to 35-nt contiguous sequence located in an intron region of the blood coagulation inhibitory gene.

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence located in an intron region of the AT gene.

In another example, the target sequence may be a 10 to 25-nt contiguous sequence located in an intron region of the TFPI gene.

The target sequence disclosed in the present specification may be a 10 to 35-nt contiguous sequence located in an exon region of the blood coagulation inhibitory gene.

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence located in an exon region of the AT gene.

In another example, the target sequence may be a 10 to 25-nt contiguous sequence located in an exon region of the TFPI gene.

The target sequence disclosed in the present specification may be a 10 to 35-nt contiguous sequence located in an enhancer region of the blood coagulation inhibitory gene.

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence located in an enhancer region of the AT gene.

In another example, the target sequence may be a 10 to 25-nt contiguous sequence located in an enhancer region of the TFPI gene.

The target sequence disclosed in the present specification may be a 10 to 35-nt contiguous sequence located in a coding region, a non-coding region or a mixed region of the blood coagulation inhibitory gene.

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence located in a coding region, a non-coding region or a mixed region of the AT gene.

In another example, the target sequence may be a 10 to 25-nt contiguous sequence located in a coding region, a non-coding region or a mixed region of the TFPI gene.

The target sequence disclosed in the present specification may be a 10 to 35-nt contiguous sequence located in a promoter, an enhancer, a 3' UTR, a polyA region or a mixed region of the blood coagulation inhibitory gene.

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence located in a promoter, an enhancer, a 3' UTR, a polyA region or a mixed region of the AT gene.

In another example the target sequence may be a 10 to 25-nt contiguous sequence located in a promoter, an enhancer, a 3' UTR, a polyA region or a mixed region of the TFPI gene.

The target sequence disclosed in the present specification may be a 10 to 35-nt contiguous sequence located in an exon, an intron or a mixed region of the blood coagulation inhibitory gene.

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence located in an exon, an intron or a mixed region of the AT gene.

In another example, the target sequence may be a 10 to 25-nt contiguous sequence located in an exon, an intron or a mixed region of the TFPI gene.

The target sequence disclosed in the present specification may be a 10 to 35-nt contiguous sequence which includes or is adjacent to a mutant part (e.g., a part different from a wild-type gene) of the blood coagulation inhibitory gene.

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence which includes or is adjacent to a mutant part (e. g, a part different from a wild-type gene) of the AT gene.

In another example, the target sequence may be a 10 to 25-nt contiguous sequence which includes or is adjacent to a mutant part (e. g, a part different from a wild-type gene) of the TFPI gene.

The target sequence disclosed in the present specification may be a 10 to 35-nt contiguous sequence which is adjacent to the 5' end and/or 3' end of a proto-spacer-adjacent motif (PAM) sequence in the nucleic acid sequence of the blood coagulation inhibitory gene.

The "proto-spacer-adjacent motif (PAM) sequence" is a nucleotide sequence that can be recognized by an editor protein. Here, the PAM sequence may have different nucleotide sequences according to the type of the editor protein and an editor protein-derived species.

Here, the PAM sequence may be, for example, one or more sequences of the following sequences (described in a 5' to 3' direction).

NGG (N is A, T, C or G);
NNNNRYAC (N is each independently A, T, C or G, R is A or G, and Y is C or T);
NNAGAAW (N is each independently A, T, C or G, and W is A or T);
NNNNGATT (N is each independently A, T, C or G);
NNGRR(T) (N is each independently A, T, C or G, and R is A or G); and
TTN (N is A, T, C or G).

Here, the target sequence may be a 10 to 35-nt sequence, a 15 to 35-nt sequence, a 20 to 35-nt sequence, a 25 to 35-nt sequence or a 30 to 35-nt sequence.

Alternatively, the target sequence may be a 10 to 15-nt sequence, a 15 to 20-nt sequence, a 20 to 25-nt sequence, a 25 to 30-nt sequence or a 30 to 35-nt sequence.

In one example, the target sequence may be a 10 to 25-nt contiguous sequence adjacent to the 5' end and/or 3' end of a PAM sequence in the nucleic acid sequence of the AT gene.

In one embodiment, when the PAM sequence recognized by an editor protein is 5'-NGG-3', 5'-NAG-3' and/or 5'-NGA-3' (N= A, T, G or C; or A, U, G or C), the target sequence may be a 10 to 25-nt contiguous sequence adjacent to the 5' end and/or 3' end of the 5'-NGG-3', 5'-NAG-3' and/or 5'-NGA-3' (N= A, T, G or C; or A, U, G or C) sequence in the nucleic acid sequence of the AT gene.

In another embodiment, when the PAM sequence recognized by an editor protein is 5'-NGGNG-3' and/or 5'-NNAGAAW-3' (W = A or T, N= A, T, G or C; or A, U, G or C), the target sequence may be a 10 to 25-nt contiguous sequence adjacent to the 5' end and/or 3' end of the 5'-NGGNG-3' and/or 5'-NNAGAAW-3' (W = A or T, N= A, T, G or C; or A, U, G or C) sequence in the nucleic acid sequence of the AT gene.

In still another embodiment, when the PAM sequence recognized by an editor protein is 5'-NNNNGATT-3' and/or 5'-NNNGCTT-3' (N= A, T, G or C; or A, U, G or C), the target sequence may be a 10 to 25-nt contiguous sequence adjacent to the 5' end and/or 3' end of the 5'-NNNNGATT-3' and/or 5'-NNNGCTT-3' (N= A, T, G or C; or A, U, G or C) sequence in the nucleic acid sequence of the AT gene.

In one embodiment, when the PAM sequence recognized by an editor protein is 5'-NNNVRYAC-3' (V = G, C or A; R = A or G, Y = C or T, N= A, T, G or C; or A, U, G or C), the target sequence may be a 10 to 25-nt contiguous sequence adjacent to the 5' end and/or 3' end of the 5'-NNNVRYAC-3' (V = G, C or A; R = A or G, Y = C or T, N= A, T, G or C; or A, U, G or C) sequence in the nucleic acid sequence of the AT gene.

In another embodiment, when the PAM sequence recognized by an editor protein is 5'-NAAR-3'(R = A or G, N= A, T, G or C; or A, U, G or C), the target sequence may be a 10 to 25-nt contiguous sequence adjacent to the 5' end and/or 3' end of the 5'-NAAR-3'(R = A or G, N= A, T, G or C; or A, U, G or C) sequence in the nucleic acid sequence of the AT gene.

In still another embodiment, when the PAM sequence recognized by an editor protein is 5'-NNGRR-3', 5'-NNGRRT-3' and/or 5'-NNGRRV-3' (R = A or G, V = G, C or A, N= A, T, G or C; or A, U, G or C), the target sequence may be a 10 to 25-nt contiguous sequence adjacent to the 5' end and/or 3' end of the 5'-NNGRR-3', 5'-NNGRRT-3' and/or 5'-NNGRRV-3' (R = A or G, V = G, C or A, N= A, T, G or C; or A, U, G or C) sequence in the nucleic acid sequence of the AT gene.

In one embodiment, when the PAM sequence recognized by an editor protein is 5'-TTN-3' (N= A, T, G or C; or A, U, G or C), the target sequence may be a 10 to 25-nt contiguous sequence adjacent to the 5' end and/or 3' end of the 5'-TTN-3' (N= A, T, G or C; or A, U, G or C) sequence in the nucleic acid sequence of the AT gene.

In another example, the target sequence may be a 10 to 25-nt contiguous sequence adjacent to the 5' end and/or 3' end of a PAM sequence in the nucleic acid sequence of the TFPI gene.

In one embodiment, when the PAM sequence recognized by an editor protein is 5'-NGG-3', 5'-NAG-3' and/or 5'-NGA-3' (N= A, T, G or C; or A, U, G or C), the target sequence may be a 10 to 25-nt contiguous sequence adjacent to the 5' end and/or 3' end of the 5'-NGG-3', 5'-NAG-3' and/or 5'-NGA-3' (N= A, T, G or C; or A, U, G or C) sequence in the nucleic acid sequence of the TFPI gene.

In another embodiment, when the PAM sequence recognized by an editor protein is 5'-NGGNG-3' and/or 5'-NNAGAAW-3' (W = A or T, N= A, T, G or C; or A, U, G or C), the target sequence may be a 10 to 25-nt contiguous sequence adjacent to the 5' end and/or 3' end of the 5'-NGGNG-3' and/or 5'-NNAGAAW-3' (W = A or T, N= A, T, G or C; or A, U, G or C) sequence in the nucleic acid sequence of the TFPI gene.

In still another embodiment, when the PAM sequence recognized by an editor protein is 5'-NNNNGATT-3' and/or 5'-NNNGCTT-3' (N= A, T, G or C; or A, U, G or C), the target sequence may be a 10 to 25-nt contiguous sequence adjacent to the 5' end and/or 3' end of the 5'-NNNNGATT-3' and/or 5'-NNNGCTT-3' (N= A, T, G or C; or A, U, G or C) sequence in the nucleic acid sequence of the TFPI gene.

In one embodiment, when the PAM sequence recognized by an editor protein is 5'-NNNVRYAC-3' (V = G, C or A; R = A or G, Y = C or T, N= A, T, G or C; or A, U, G or C), the target sequence may be a 10 to 25-nt contiguous sequence adjacent to the 5' end and/or 3' end of the 5'-NNNVRYAC-3' (V = G, C or A; R = A or G, Y = C or T, N= A, T, G or C; or A, U, G or C) sequence in the nucleic acid sequence of the TFPI gene.

In another embodiment, when the PAM sequence recognized by an editor protein is 5'-NAAR-3'(R = A or G, N= A, T, G or C; or A, U, G or C), the target sequence may be a 10 to 25-nt contiguous sequence adjacent to the 5' end and/or 3' end of the 5'-NAAR-3'(R = A or G, N= A, T, G or C; or A, U, G or C) sequence in the nucleic acid sequence of the TFPI gene.

In still another embodiment, when the PAM sequence recognized by an editor protein is 5'-NNGRR-3', 5'-NNGRRT-3' and/or 5'-NNGRRV-3' (R = A or G, V = G, C or A, N= A, T, G or C; or A, U, G or C), the target sequence may be a 10 to 25-nt contiguous sequence adjacent to the 5' end and/or 3' end of the 5'-NNGRR-3', 5'-NNGRRT-3' and/or 5'-NNGRRV-3' (R = A or G, V = G, C or A, N= A, T, G or C; or A, U, G or C) sequence in the nucleic acid sequence of the TFPI gene.

In one embodiment, when the PAM sequence recognized by an editor protein is 5'-TTN-3' (N= A, T, G or C; or A, U, G or C), the target sequence may be a 10 to 25-nt contiguous sequence adjacent to the 5' end and/or 3' end of the 5'-TTN-3' (N= A, T, G or C; or A, U, G or C) sequence in the nucleic acid sequence of the TFPI gene.

Hereinafter, examples of target sequences that can be used in an exemplary embodiment disclosed in the specification are listed in Tables 1 and 2. The target sequences disclosed in Tables 1 and 2 are guide nucleic acid-non binding sequences, and complementary sequences thereof, that is, guide nucleic acid-binding sequences may be predicted from the sequences listed in the tables. In addition, target names shown in Tables 1 and 2 were named Sp for SpCas9, Sa for SaCas9 and Cj for CjCas9 according to an editor protein.

**[Table 1]**

| Target sequences of SERPINC 1 gene(AT gene) | | | | |
|---|---|---|---|---|
| Target name | Loci. | Target(w/o PAM) | GC Contents (%) | SEQ ID NO |
| hSerpinc 1-Sp-1 | Exon 01(E01) | TCCTATCACATTGGA ATACA | 35 | SEQ ID NO: 19 |
| hSerpinc 1-Sp-2 | E01 | GGTTACAGTTCCTAT CACAT | 40 | SEQ ID NO: 20 |
| hSerpinc 1-Sp-3 | E01 | GCCATGTATTCCAAT GTGAT | 40 | SEQ ID NO: 21 |
| hSerpinc1-Sp-4 | E01 | GTGATAGGAACTGTA ACCTC | 45 | SEQ ID NO: 22 |
| hSerpinc 1-Sp-5 | E01 | CCCCTCTTACCTTTTT CCAG | 50 | SEQ ID NO: 23 |
| hSerpinc 1-Sp-6 | E01 | GAACTGTAACCTCTG | 40 | SEQ ID NO: 24 |
| | | GAAAA | | |
| hSerpinc1-Sp-7 | E02 | CCAGAAGCCAATGA GCAGCA | 55 | SEQ ID NO: 25 |
| hSerpinc1-Sp-8 | E02 | CTTTTGTCCTTGCTGC TCAT | 45 | SEQ ID NO: 26 |
| hSerpinc1-Sp-9 | E02 | CCTTGCTGCTCATTG GCTTC | 55 | SEQ ID NO: 27 |
| hSerpinc1-Sp-1 0 | E02 | CTTGCTGCTCATTGG CTTCT | 50 | SEQ ID NO: 28 |
| hSerpinc1-Sp-11 | E02 | TGGGACTGCGTGACC TGTCA | 60 | SEQ ID NO: 29 |
| hSerpinc1-Sp-12 | E02 | GGGACTGCGTGACCT GTCAC | 65 | SEQ ID NO: 30 |
| hSerpinc1-Sp-13 | E02 | GTCCACAGGGCTCCC GTGAC | 70 | SEQ ID NO: 31 |
| hSerpinc1-Sp-14 | E02 | GACCTGTCACGGGAG CCCTG | 70 | SEQ ID NO: 32 |
| hSerpinc 1-Sp-15 | E02 | TGGCTGTGCAGATGT CCACA | 55 | SEQ ID NO: 33 |
| hSerpinc1-Sp-16 | E02 | TTGGCTGTGCAGATG TCCAC | 55 | SEQ ID NO: 34 |
| hSerpinc1-Sp-17 | E02 | ACATCTGCACAGCCA AGCCG | 60 | SEQ ID NO: 35 |
| hSerpinc1-Sp-18 | E02 | CATCTGCACAGCCAA GCCGC | 65 | SEQ ID NO: 36 |
| hSerpinc1-Sp-19 | E02 | CATGGGAATGTCCCG CGGCT | 65 | SEQ ID NO: 37 |
| hSerpinc1-Sp-20 | E02 | GGATTCATGGGAATG TCCCG | 55 | SEQ ID NO: 38 |
| hSerpinc1-Sp-21 | E02 | TAAATGCACATGGGA TTCAT | 35 | SEQ ID NO: 39 |
| hSerpinc1-Sp-22 | E02 | GTAAATGCACATGGG ATTCA | 40 | SEQ ID NO: 40 |
| hSerpinc 1-Sp-23 | E02 | GGGGAGCGGTAAAT GCACAT | 55 | SEQ ID NO: 41 |
| hSerpinc 1-Sp-24 | E02 | CGGGGAGCGGTAAA TGCACA | 60 | SEQ ID NO: 42 |
| hSerpinc 1-Sp-25 | E02 | CATGTGCATTTACCG CTCCC | 55 | SEQ ID NO: 43 |
| hSerpinc1-Sp-26 | E02 | TTGCCTTCTTCTCCG GGGAG | 60 | SEQ ID NO: 44 |
| hSerpinc1-Sp-27 | E02 | CTCAGTTGCCTTCTT CTCCG | 55 | SEQ ID NO: 45 |
| hSerpinc1-Sp-28 | E02 | CCTCAGTTGCCTTCT TCTCC | 55 | SEQ ID NO: 46 |
| hSerpinc1-Sp-29 | E02 | TCCTCAGTTGCCTTC TTCTC | 50 | SEQ ID NO: 47 |
| hSerpinc1-Sp-30 | E02 | TTACCGCTCCCCGGA GAAGA | 60 | SEQ ID NO: 48 |
| hSerpinc 1-Sp-31 | E02 | CCCGGAGAAGAAGG CAACTG | 60 | SEQ ID NO: 49 |
| hSerpinc1-Sp-32 | E02 | GAAGAAGGCAACTG AGGATG | 50 | SEQ ID NO: 50 |
| hSerpinc1-Sp-33 | E02 | AAGAAGGCAACTGA GGATGA | 45 | SEQ ID NO: 51 |
| hSerpinc 1-Sp-34 | E02 | GGGCTCAGAACAGA AGATCC | 55 | SEQ ID NO: 52 |
| hSerpinc1-Sp-35 | E02 | CTCAGAACAGAAGA TCCCGG | 55 | SEQ ID NO: 53 |
| hSerpinc1-Sp-36 | E02 | CACGCCGGTTGGTGG CCTCC | 75 | SEQ ID NO: 54 |
| hSerpinc1-Sp-37 | E02 | ACACGCCGGTTGGTG | 70 | SEQ ID NO: 55 |
| | | GCCTC | | |
| hSerpinc1-Sp-38 | E02 | AGATCCCGGAGGCC ACCAAC | 65 | SEQ ID NO: 56 |
| hSerpinc1-Sp-39 | E02 | TTCCCAGACACGCCG GTTGG | 65 | SEQ ID NO: 57 |
| hSerpinc 1-Sp-40 | E02 | CAGTTCCCAGACACG CCGGT | 65 | SEQ ID NO: 58 |
| hSerpinc1-Sp-41 | E02 | TGGACAGTTCCCAGA CACGC | 60 | SEQ ID NO: 59 |
| hSerpinc 1-Sp-42 | E02 | AGGCCACCAACCGG CGTGTC | 70 | SEQ ID NO: 60 |
| hSerpinc 1-Sp-43 | E02 | GGCCACCAACCGGC GTGTCT | 70 | SEQ ID NO: 61 |
| hSerpinc1-Sp-44 | E02 | GCGTGTCTGGGAACT GTCCA | 60 | SEQ ID NO: 62 |
| hSerpinc1-Sp-45 | E02 | AGCAAAGCGGGAAT TGGCCT | 55 | SEQ ID NO: 63 |
| hSerpinc1-Sp-46 | E02 | AGTGGTAGCAAAGC GGGAAT | 50 | SEQ ID NO: 64 |
| hSerpinc1-Sp-47 | E02 | ATAGAAAGTGGTAG CAAAGC | 40 | SEQ ID NO: 65 |
| hSerpinc1-Sp-48 | E02 | GATAGAAAGTGGTA GCAAAG | 40 | SEQ ID NO: 66 |
| hSerpinc1-Sp-49 | E02 | TGCCAGGTGCTGATA GAAAG | 50 | SEQ ID NO: 67 |
| hSerpinc1-Sp-50 | E02 | TACCACTTTCTATCA GCACC | 45 | SEQ ID NO: 68 |
| hSerpinc1-Sp-51 | E02 | TGTCATTCTTGGAAT CTGCC | 45 | SEQ ID NO: 69 |
| hSerpinc1-Sp-52 | E02 | AATGTTATCATTGTC ATTCT | 25 | SEQ ID NO: 70 |
| hSerpinc 1-Sp-53 | E02 | TGGAGATACTCAGGG GTGAC | 55 | SEQ ID NO: 71 |
| hSerpinc1-Sp-54 | E02 | AAAGCCGTGGAGAT ACTCAG | 50 | SEQ ID NO: 72 |
| hSerpinc1-Sp-55 | E02 | AAAAGCCGTGGAGA TACTCA | 45 | SEQ ID NO: 73 |
| hSerpinc1-Sp-56 | E02 | CAAAAGCCGTGGAG ATACTC | 50 | SEQ ID NO: 74 |
| hSerpinc1-Sp-57 | E02 | GTCACCCCTGAGTAT CTCCA | 55 | SEQ ID NO: 75 |
| hSerpinc1-Sp-58 | E02 | CTTGGTCATAGCAAA AGCCG | 50 | SEQ ID NO: 76 |
| hSerpinc1-Sp-59 | E02 | GGCTTTTGCTATGAC CAAGC | 50 | SEQ ID NO: 77 |
| hSerpinc1-Sp-60 | E02 | GCTTTTGCTATGACC AAGCT | 45 | SEQ ID NO: 78 |
| hSerpinc1-Sp-61 | E02 | GTCATTACAGGCACC CAGCT | 55 | SEQ ID NO: 79 |
| hSerpinc1-Sp-62 | E02 | TTGCTGGAGGGTGTC ATTAC | 50 | SEQ ID NO: 80 |
| hSerpinc1-Sp-63 | E02 | TACCTCCATCAGTTG CTGGA | 50 | SEQ ID NO: 81 |
| hSerpinc1-Sp-64 | E02 | GTACCTCCATCAGTT GCTGG | 55 | SEQ ID NO: 82 |
| hSerpinc1-Sp-65 | E02 | GTCGTACCTCCATCA GTTGC | 55 | SEQ ID NO: 83 |
| hSerpinc1-Sp-66 | E02 | TGACACCCTCCAGCA ACTGA | 55 | SEQ ID NO: 84 |
| hSerpinc1-Sp-67 | E02 | CACCCTCCAGCAACT GATGG | 60 | SEQ ID NO: 85 |
| hSerpinc1-Sp-68 | E03 | ATCAGATGTTTTCTC | 30 | SEQ ID NO: 86 |
| | | AGATA | | |
| hSerpinc1-Sp-69 | E03 | TCAGTTTGGCAAAGA AGAAG | 40 | SEQ ID NO: 87 |
| hSerpinc1-Sp-70 | E03 | ATAGAGTCGGCAGTT CAGTT | 45 | SEQ ID NO: 88 |
| hSerpinc1-Sp-71 | E03 | TGTTGGCTTTTCGAT AGAGT | 40 | SEQ ID NO: 89 |
| hSerpinc1-Sp-72 | E03 | TACTAACTTGGAGGA TTTGT | 35 | SEQ ID NO: 90 |
| hSerpinc 1-Sp-73 | E03 | ATTGGCTGATACTAA CTTGG | 40 | SEQ ID NO: 91 |
| hSerpinc1-Sp-74 | E03 | GCGATTGGCTGATAC TAACT | 45 | SEQ ID NO: 92 |
| hSerpinc1-Sp-75 | E03 | TTTGTCTCCAAAAAG GCGAT | 40 | SEQ ID NO: 93 |
| hSerpinc1-Sp-76 | E03 | GTATCAGCCAATCGC CTTTT | 45 | SEQ ID NO: 94 |
| hSerpinc1-Sp-77 | E03 | TAAGGGATTTGTCTC CAAAA | 35 | SEQ ID NO: 95 |
| hSerpinc1-Sp-78 | E03 | GTAGGTCTCATTGAA GGTAA | 40 | SEQ ID NO: 96 |
| hSerpinc1-Sp-79 | E03 | GGTAGGTCTCATTGA AGGTA | 45 | SEQ ID NO: 97 |
| hSerpinc1-Sp-80 | E03 | GTCCTGGTAGGTCTC ATTGA | 50 | SEQ ID NO: 98 |
| hSerpinc1-Sp-81 | E03 | TACCTTCAATGAGAC CTACC | 45 | SEQ ID NO: 99 |
| hSerpinc1-Sp-82 | E03 | CAACTCACTGATGTC CTGGT | 50 | SEQ ID NO: 100 |
| hSerpinc1-Sp-83 | E03 | ATACCAACTCACTGA TGTCC | 45 | SEQ ID NO: 101 |
| hSerpinc1-Sp-84 | E03 | CTACCAGGACATCAG TGAGT | 50 | SEQ ID NO: 102 |
| hSerpinc1-Sp-85 | E03 | GACATCAGTGAGTTG GTATA | 40 | SEQ ID NO: 103 |
| hSerpinc1-Sp-86 | E03 | GAAGTCCAGGGGCT GGAGCT | 65 | SEQ ID NO: 104 |
| hSerpinc1-Sp-87 | E03 | TGGAGCCAAGCTCCA GCCCC | 70 | SEQ ID NO: 105 |
| hSerpinc1-Sp-88 | E03 | TCACCTTGAAGTCCA GGGGC | 60 | SEQ ID NO: 106 |
| hSerpinc1-Sp-89 | E03 | CAACTCACCTTGAAG TCCAG | 50 | SEQ ID NO: 107 |
| hSerpinc1-Sp-90 | E03 | GCAACTCACCTTGAA GTCCA | 50 | SEQ ID NO: 108 |
| hSerpinc1-Sp-91 | E03 | TGCAACTCACCTTGA AGTCC | 50 | SEQ ID NO: 109 |
| hSerpinc1-Sp-92 | E03 | GCTCCAGCCCCTGGA CTTCA | 65 | SEQ ID NO: 110 |
| hSerpinc1-Sp-93 | E04 | GATTGCTCTGCATTT TCCTG | 45 | SEQ ID NO: 111 |
| hSerpinc1-Sp-94 | E04 | AAATGCAGAGCAAT CCAGAG | 45 | SEQ ID NO: 112 |
| hSerpinc 1-Sp-95 | E04 | CCATTTGTTGATGGC CGCTC | 55 | SEQ ID NO: 113 |
| hSerpinc1-Sp-96 | E04 | ATTGGACACCCATTT GTTGA | 40 | SEQ ID NO: 114 |
| hSerpinc1-Sp-97 | E04 | CCAGAGCGGCCATCA ACAAA | 55 | SEQ ID NO: 115 |
| hSerpinc1-Sp-98 | E04 | CAGAGCGGCCATCA ACAAAT | 50 | SEQ ID NO: 116 |
| hSerpinc1-Sp-99 | E04 | GATTCGGCCTTCGGT | 50 | SEQ ID NO: 117 |
| | | CTTAT | | |
| hSerpinc1-Sp-100 | E04 | TGGGTGTCCAATAAG ACCGA | 50 | SEQ ID NO: 118 |
| hSerpinc1-Sp-101 | E04 | GACATCGGTGATTCG GCCTT | 55 | SEQ ID NO: 119 |
| hSerpinc1-Sp-102 | E04 | AGGGAATGACATCG GTGATT | 45 | SEQ ID NO: 120 |
| hSerpinc1-Sp-103 | E04 | GGCTTCCGAGGGAAT GACAT | 55 | SEQ ID NO: 121 |
| hSerpinc1-Sp-104 | E04 | AATCACCGATGTCAT TCCCT | 45 | SEQ ID NO: 122 |
| hSerpinc1-Sp-105 | E04 | AGCTCATTGATGGCT TCCGA | 50 | SEQ ID NO: 123 |
| hSerpinc1-Sp-106 | E04 | GAGCTCATTGATGGC TTCCG | 55 | SEQ ID NO: 124 |
| hSerpinc1-Sp-107 | E04 | CAGAACAGTGAGCTC ATTGA | 45 | SEQ ID NO: 125 |
| hSerpinc1-Sp-108 | E04 | CATCAATGAGCTCAC TGTTC | 45 | SEQ ID NO: 126 |
| hSerpinc1-Sp-109 | E04 | TGAGCTCACTGTTCT GGTGC | 55 | SEQ ID NO: 127 |
| hSerpinc1-Sp-110 | E04 | TCTGAGTACCTTGAA GTAAA | 35 | SEQ ID NO: 128 |
| hSerpinc1-Sp-111 | E04 | GGTTAACACCATTTA CTTCA | 35 | SEQ ID NO: 129 |
| hSerpinc1-Sp-112 | E05 | ACTTTGACTTCCACA GGCCC | 55 | SEQ ID NO: 130 |
| hSerpinc1-Sp-113 | E05 | TGATTCTCTTCCAGG GCCTG | 55 | SEQ ID NO: 131 |
| hSerpinc1-Sp-114 | E05 | GGCTGAACTTTGACT TCCAC | 50 | SEQ ID NO: 132 |
| hSerpinc1-Sp-115 | E05 | GTTCCTTCCTTGTGTT CTCA | 45 | SEQ ID NO: 133 |
| hSerpinc1-Sp-116 | E05 | AGTTCCTTCCTTGTG TTCTC | 45 | SEQ ID NO: 134 |
| hSerpinc1-Sp-117 | E05 | AGTTCAGCCCTGAGA ACACA | 50 | SEQ ID NO: 135 |
| hSerpinc1-Sp-118 | E05 | CAGCCCTGAGAACAC AAGGA | 55 | SEQ ID NO: 136 |
| hSerpinc1-Sp-119 | E05 | AAGGAAGGAACTGT TCTACA | 40 | SEQ ID NO: 137 |
| hSerpincl-Sp-120 | E05 | GAACTGTTCTACAAG GCTGA | 45 | SEQ ID NO: 138 |
| hSerpinc1-Sp-121 | E05 | TTCAGCATCTATGAT GTACC | 40 | SEQ ID NO: 139 |
| hSerpinc1-Sp-122 | E05 | GCATCTATGATGTAC CAGGA | 45 | SEQ ID NO: 140 |
| hSerpinc1-Sp-123 | E05 | GATAACGGAACTTGC CTTCC | 50 | SEQ ID NO: 141 |
| hSerpinc1-Sp-124 | E05 | AGGAAGGCAAGTTC CGTTAT | 45 | SEQ ID NO: 142 |
| hSerpinc1-Sp-125 | E05 | CTTCAGCCACGCGCC GATAA | 60 | SEQ ID NO: 143 |
| hSerpinc1-Sp-126 | E05 | CAAGTTCCGTTATCG GCGCG | 60 | SEQ ID NO: 144 |
| hSerpinc1-Sp-127 | E05 | CGTTATCGGCGCGTG GCTGA | 65 | SEQ ID NO: 145 |
| hSerpinc1-Sp-128 | E05 | GCGCGTGGCTGAAG GCACCC | 75 | SEQ ID NO: 146 |
| hSerpinc1-Sp-129 | E05 | GAAGGGCAACTCAA GCACCT | 55 | SEQ ID NO: 147 |
| hSerpinc1-Sp-130 | E05 | TGAAGGGCAACTCA | 55 | SEQ ID NO: 148 |
| | | AGCACC | | |
| hSerpinc1-Sp-131 | E05 | GTGCTTGAGTTGCCC TTCAA | 50 | SEQ ID NO: 149 |
| hSerpinc1-Sp-132 | E05 | GTGATGTCATCACCT TTGAA | 40 | SEQ ID NO: 150 |
| hSerpinc1-Sp-133 | E05 | GGTGATGTCATCACC TTTGA | 45 | SEQ ID NO: 151 |
| hSerpinc1-Sp-134 | E05 | CAAAGGTGATGACAT CACCA | 45 | SEQ ID NO: 152 |
| hSerpinc1-Sp-135 | E05 | CTTGGGCAAGATGAG GACCA | 55 | SEQ ID NO: 153 |
| hSerpinc1-Sp-136 | E05 | TCTCAGGCTTGGGCA AGATG | 55 | SEQ ID NO: 154 |
| hSerpinc1-Sp-137 | E05 | GCCAGGCTCTTCTCA GGCTT | 60 | SEQ ID NO: 155 |
| hSerpinc1-Sp-138 | E05 | GGCCAGGCTCTTCTC AGGCT | 65 | SEQ ID NO: 156 |
| hSerpinc1-Sp-139 | E05 | ACCTTGGCCAGGCTC TTCTC | 60 | SEQ ID NO: 157 |
| hSerpinc1-Sp-140 | E05 | GCCCAAGCCTGAGA AGAGCC | 65 | SEQ ID NO: 158 |
| hSerpinc1-Sp-141 | E05 | GCCTGAGAAGAGCCT GGCCA | 65 | SEQ ID NO: 159 |
| hSerpinc1-Sp-142 | E05 | GTTCCTTCTCTACCTT GGCC | 55 | SEQ ID NO: 160 |
| hSerpinc1-Sp-143 | E05 | GGTGAGTTCCTTCTC TACCT | 50 | SEQ ID NO: 161 |
| hSerpinc1-Sp-144 | E05 | GAGCCTGGCCAAGGT AGAGA | 60 | SEQ ID NO: 162 |
| hSerpinc1-Sp-145 | E05 | AGAGAAGGAACTCA CCCCAG | 55 | SEQ ID NO: 163 |
| hSerpinc1-Sp-146 | E05 | CCACTCTTGCAGCAC CTCTG | 60 | SEQ ID NO: 164 |
| hSerpinc1-Sp-147 | E05 | GCCACTCTTGCAGCA CCTCT | 60 | SEQ ID NO: 165 |
| hSerpinc1-Sp-148 | E05 | AGCCACTCTTGCAGC ACCTC | 60 | SEQ ID NO: 166 |
| hSerpincl-Sp-149 | E05 | CCCCAGAGGTGCTGC AAGAG | 65 | SEQ ID NO: 167 |
| hSerpinc1-Sp-150 | E05 | AGAGGTGCTGCAAG AGTGGC | 60 | SEQ ID NO: 168 |
| hSerpinc1-Sp-151 | E05 | GCAAGAGTGGCTGG ATGAAT | 50 | SEQ ID NO: 169 |
| hSerpinc1-Sp-152 | E05 | AGAGTGGCTGGATG AATTGG | 50 | SEQ ID NO: 170 |
| hSerpinc1-Sp-153 | E05 | TGAATTGGAGGAGAT GATGC | 45 | SEQ ID NO: 171 |
| hSerpinc1-Sp-154 | E05 | ATTGGAGGAGATGAT GCTGG | 50 | SEQ ID NO: 172 |
| hSerpinc1-Sp-155 | E05 | CAATGCGGAAGCGG GGCATG | 65 | SEQ ID NO: 173 |
| hSerpinc1-Sp-156 | E05 | CCGTCCTCAATGCGG AAGCG | 65 | SEQ ID NO: 174 |
| hSerpinc1-Sp-157 | E05 | GCCGTCCTCAATGCG GAAGC | 65 | SEQ ID NO: 175 |
| hSerpinc1-Sp-158 | E05 | AGCCGTCCTCAATGC GGAAG | 60 | SEQ ID NO: 176 |
| hSerpinc1-Sp-159 | E05 | CATGCCCCGCTTCCG CATTG | 65 | SEQ ID NO: 177 |
| hSerpinc1-Sp-160 | E05 | AACTGAAGCCGTCCT CAATG | 50 | SEQ ID NO: 178 |
| hSerpinc1-Sp-161 | E05 | CCCCGCTTCCGCATT | 65 | SEQ ID NO: 179 |
| | | GAGGA | | |
| hSerpinc1-Sp-162 | E05 | TGAGGACGGCTTCAG TTTGA | 50 | SEQ ID NO: 180 |
| hSerpinc1-Sp-163 | E05 | GAAGGAGCAGCTGC AAGACA | 55 | SEQ ID NO: 181 |
| hSerpinc1-Sp-164 | E05 | AAGGAGCAGCTGCA AGACAT | 50 | SEQ ID NO: 182 |
| hSerpinc1-Sp-165 | E05 | CAGGGCTGAACAGA TCGACA | 55 | SEQ ID NO: 183 |
| hSerpinc1-Sp-166 | E05 | CTGGGAGTTTGGACT TTTCA | 45 | SEQ ID NO: 184 |
| hSerpinc1-Sp-167 | E05 | CCTGGGAGTTTGGAC TTTTC | 50 | SEQ ID NO: 185 |
| hSerpinc1-Sp-168 | E05 | CCTAGACAAACCTGG GAGTT | 50 | SEQ ID NO: 186 |
| hSerpinc1-Sp-169 | E05 | CCTGAAAAGTCCAAA CTCCC | 50 | SEQ ID NO: 187 |
| hSerpinc1-Sp-170 | E06 | CGGCCTTCTGCAACA ATACC | 55 | SEQ ID NO: 188 |
| hSerpinc1-Sp-171 | E06 | CTTCCAGGTATTGTT GCAGA | 45 | SEQ ID NO: 189 |
| hSerpinc1-Sp-172 | E06 | CTGAGACATAGAGGT CATCT | 45 | SEQ ID NO: 190 |
| hSerpinc1-Sp-173 | E06 | GGAATGCATCTGAGA CATAG | 45 | SEQ ID NO: 191 |
| hSerpinc1-Sp-174 | E06 | TGTCTCAGATGCATT CCATA | 40 | SEQ ID NO: 192 |
| hSerpinc1-Sp-175 | E06 | TCACCTCAAGAAATG CCTTA | 40 | SEQ ID NO: 193 |
| hSerpinc1-Sp-176 | E06 | ATTCCATAAGGCATT TCTTG | 35 | SEQ ID NO: 194 |
| hSerpinc1-Sp-177 | E07 | GACCTGCAGGTAAAT GAAGA | 45 | SEQ ID NO: 195 |
| hSerpinc1-Sp-178 | E07 | ACGGCCAGCAATCAC AACAG | 55 | SEQ ID NO: 196 |
| hSerpinc1-Sp-179 | E07 | AGTACCGCTGTTGTG ATTGC | 50 | SEQ ID NO: 197 |
| hSerpinc1-Sp-180 | E07 | CCCTGTTGGGGTTTA GCGAA | 55 | SEQ ID NO: 198 |
| hSerpinc1-Sp-181 | E07 | GCCGTTCGCTAAACC CCAAC | 60 | SEQ ID NO: 199 |
| hSerpinc1-Sp-182 | E07 | CCGTTCGCTAAACCC CAACA | 55 | SEQ ID NO: 200 |
| hSerpinc1-Sp-183 | E07 | CCTTGAAAGTCACCC TGTTG | 50 | SEQ ID NO: 201 |
| hSerpinc1-Sp-184 | E07 | GCCTTGAAAGTCACC CTGTT | 50 | SEQ ID NO: 202 |
| hSerpinc1-Sp-185 | E07 | GGCCTTGAAAGTCAC CCTGT | 55 | SEQ ID NO: 203 |
| hSerpinc1-Sp-186 | E07 | CCCCAACAGGGTGAC TTTCA | 55 | SEQ ID NO: 204 |
| hSerpinc1-Sp-187 | E07 | GGGTGACTTTCAAGG CCAAC | 55 | SEQ ID NO: 205 |
| hSerpinc1-Sp-188 | E07 | AAAAACCAGGAAAG GCCTGT | 45 | SEQ ID NO: 206 |
| hSerpinc1-Sp-189 | E07 | CAAGGCCAACAGGC CTTTCC | 60 | SEQ ID NO: 207 |
| hSerpinc1-Sp-190 | E07 | TCTCTTATAAAAACC AGGAA | 30 | SEQ ID NO: 208 |
| hSerpinc1-Sp-191 | E07 | GAACTTCTCTTATAA AAACC | 30 | SEQ ID NO: 209 |
| hSerpinc1-Sp-192 | E07 | ATGAAGATAATAGTG | 30 | SEQ ID NO: 210 |
| | | TTCAG | | |
| hSerpinc1-Sp-193 | E07 | TCTGAACACTATTAT CTTCA | 30 | SEQ ID NO: 211 |
| hSerpinc1-Sp-194 | E07 | CTGAACACTATTATC TTCAT | 30 | SEQ ID NO: 212 |
| hSerpinc1-Sp-195 | E07 | ATTTTACTTAACACA AGGGT | 30 | SEQ ID NO: 213 |
| hSerpinc1-Sp-196 | E07 | GAACATTTTACTTAA CACAA | 25 | SEQ ID NO: 214 |
| hSerpinc1-Sp-197 | E07 | AGAACATTTTACTTA ACACA | 25 | SEQ ID NO: 215 |
| hSerpinc1-Sa-1 | E01 | GGTTACAGTTCCTAT CACAT | 40 | SEQ ID NO: 216 |
| hSerpinc1-Sa-2 | E02 | CCAAGCCGCGGAC ATTCCC | 70 | SEQ ID NO: 217 |
| hSerpinc 1-Sa-3 | E02 | TAAATGCACATGGGA TTCAT | 35 | SEQ ID NO: 218 |
| hSerpinc1-Sa-4 | E02 | CGGGGAGCGGTAAA TGCACA | 60 | SEQ ID NO: 219 |
| hSerpinc1-Sa-5 | E02 | CCCCGGAGAAGAAG GCAACT | 60 | SEQ ID NO: 220 |
| hSerpinc1-Sa-6 | E02 | ACACGCCGGTTGGTG GCCTC | 70 | SEQ ID NO: 221 |
| hSerpinc1-Sa-7 | E02 | ATAGAAAGTGGTAG CAAAGC | 40 | SEQ ID NO: 222 |
| hSerpinc1-Sa-8 | E02 | ATCAGCACCTGGCAG ATTCC | 55 | SEQ ID NO: 223 |
| hSerpinc1-Sa-9 | E02 | AATGTTATCATTGTC ATTCT | 25 | SEQ ID NO: 224 |
| hSerpinc1-Sa-10 | E02 | ATAACATTTTCCTGT CACCC | 40 | SEQ ID NO: 225 |
| hSerpinc1-Sa-11 | E02 | CAAAAGCCGTGGAG ATACTC | 50 | SEQ ID NO: 226 |
| hSerpinc1-Sa-12 | E02 | CGGCTTTTGCTATGA CCAAG | 50 | SEQ ID NO: 227 |
| hSerpinc1-Sa-13 | E02 | CGTACCTCCATCAGT TGCTG | 55 | SEQ ID NO: 228 |
| hSerpinc1-Sa-14 | E03 | TTCAGTTTGGCAAAG AAGAA | 35 | SEQ ID NO: 229 |
| hSerpinc1-Sa-15 | E03 | AGGATTTGTTGGCTT TTCGA | 40 | SEQ ID NO: 230 |
| hSerpinc1-Sa-16 | E03 | GATTGGCTGATACTA ACTTG | 40 | SEQ ID NO: 231 |
| hSerpinc1-Sa-17 | E03 | GGTAGGTCTCATTGA AGGTA | 45 | SEQ ID NO: 232 |
| hSerpinc1-Sa-18 | E03 | TGAGACCTACCAGGA CATCA | 50 | SEQ ID NO: 233 |
| hSerpinc1-Sa-19 | E03 | TCCAGCCCCTGGACT TCAAG | 60 | SEQ ID NO: 234 |
| hSerpinc1-Sa-20 | E04 | CCCATTTGTTGATGG CCGCT | 55 | SEQ ID NO: 235 |
| hSerpinc1-Sa-21 | E04 | TCCAGAGCGGCCATC AACAA | 55 | SEQ ID NO: 236 |
| hSerpinc1-Sa-22 | E04 | GTGTCCAATAAGACC GAAGG | 50 | SEQ ID NO: 237 |
| hSerpinc 1-Sa-23 | E04 | AGCTCATTGATGGCT TCCGA | 50 | SEQ ID NO: 238 |
| hSerpinc1-Sa-24 | E05 | ACTGTTCTACAAGGC TGATG | 45 | SEQ ID NO: 239 |
| hSerpinc1-Sa-25 | E05 | GGCTGAAGGCACCC AGGTGC | 70 | SEQ ID NO: 240 |
| hSerpinc1-Sa-26 | E05 | TTGAAGGGCAACTCA | 50 | SEQ ID NO: 241 |
| | | AGCAC | | |
| hSerpinc 1-Sa-27 | E05 | ACTCTTGCAGCACCT CTGGG | 60 | SEQ ID NO: 242 |
| hSerpinc1-Sa-28 | E05 | AGCCACTCTTGCAGC ACCTC | 60 | SEQ ID NO: 243 |
| hSerpinc1-Sa-29 | E05 | ACTCACCCCAGAGGT GCTGC | 65 | SEQ ID NO: 244 |
| hSerpinc1-Sa-30 | E05 | CAGAGGTGCTGCAA GAGTGG | 60 | SEQ ID NO: 245 |
| hSerpinc 1-Sa-31 | E05 | GGTGCTGCAAGAGTG GCTGG | 65 | SEQ ID NO: 246 |
| hSerpinc1-Sa-32 | E06 | TCACCTCAAGAAATG CCTTA | 40 | SEQ ID NO: 247 |
| hSerpinc1-Sa-33 | E06 | TCCATAAGGCATTTC TTGAG | 40 | SEQ ID NO: 248 |
| hSerpinc1-Sa-34 | E07 | GGCCGTTCGCTAAAC CCCAA | 60 | SEQ ID NO: 249 |
| hSerpinc 1-Sa-35 | E07 | GGCCTTGAAAGTCAC CCTGT | 55 | SEQ ID NO: 250 |
| hSerpinc1-Sa-36 | E07 | AACACTATTATCTTC ATGGG | 35 | SEQ ID NO: 251 |
| hSerpinc1-Sa-37 | E07 | AAGAACATTTTACTT AACAC | 25 | SEQ ID NO: 252 |
| hSerpinc1-Cj-1 | E01 | GAGGTTACAGTTCCT ATCACAT | 41 | SEQ ID NO: 253 |
| hSerpinc 1-Cj-2 | E02 | CTGTCACGGGAGCCC TGTGGAC | 68 | SEQ ID NO: 254 |
| hSerpinc 1-Cj-3 | E02 | GCCTTCTTCTCCGGG GAGCGGT | 68 | SEQ ID NO: 255 |
| hSerpinc 1-Cj-4 | E02 | GGGAATTGGCCTTGG ACAGTTC | 55 | SEQ ID NO: 256 |
| hSerpinc1-Cj-5 | E02 | TCCCGCTTTGCTACC ACTTTCT | 50 | SEQ ID NO: 257 |
| hSerpinc 1-Cj-6 | E02 | GCTTGGTCATAGCAA AAGCCGT | 50 | SEQ ID NO: 258 |
| hSerpinc1-Cj -7 | E02 | TATGACCAAGCTGGG TGCCTGT | 55 | SEQ ID NO: 259 |
| hSerpinc1-Cj-8 | E02 | TCAGTTGCTGGAGGG TGTCATT | 50 | SEQ ID NO: 260 |
| hSerpinc 1-Cj-9 | E02 | ATGACACCCTCCAGC AACTGAT | 50 | SEQ ID NO: 261 |
| hSerpinc1-Cj-10 | E03 | TTGACTTCTATAGGT ATTTAAG | 27 | SEQ ID NO: 262 |
| hSerpinc1-Cj-11 | E03 | TTTCTCAGATATGGT GTCAAAC | 36 | SEQ ID NO: 263 |
| hSerpinc1-Cj-12 | E03 | TTTGTCTCCAAAAAG GCGATTG | 41 | SEQ ID NO: 264 |
| hSerpinc1-Cj-13 | E03 | GTCCAGGGGCTGGA GCTTGGCT | 68 | SEQ ID NO: 265 |
| hSerpinc1-Cj-14 | E04 | GGTGATTCGGCCTTC GGTCTTA | 55 | SEQ ID NO: 266 |
| hSerpinc1-Cj-15 | E04 | TGAGCTCACTGTTCT GGTGCTG | 55 | SEQ ID NO: 267 |
| hSerpinc1-Cj-16 | E04 | TACCTTGAAGTAAAT GGTGTTA | 32 | SEQ ID NO: 268 |
| hSerpinc1-Cj-17 | E04 | TGCTGGTTAACACCA TTTACTT | 36 | SEQ ID NO: 269 |
| hSerpinc1-Cj-18 | E05 | GTGGAAGTCAAAGTT CAGCCCT | 50 | SEQ ID NO: 270 |
| hSerpinc1-Cj-19 | E05 | GGAGAGTCGTGTTCA GCATCTA | 50 | SEQ ID NO: 271 |
| hSerpinc1-Cj-20 | E05 | CCTTCCTGGTACATC | 45 | SEQ ID NO: 272 |
| | | ATAGATG | | |
| hSerpinc 1-Cj-21 | E05 | CGCCGATAACGGAA CTTGCCTT | 55 | SEQ ID NO: 273 |
| hSerpinc 1-Cj-22 | E05 | GTTCCGTTATCGGCG CGTGGCT | 64 | SEQ ID NO: 274 |
| hSerpinc 1-Cj-23 | E05 | GTCATCACCTTTGAA GGGCAAC | 50 | SEQ ID NO: 275 |
| hSerpinc 1-Cj-24 | E05 | CTCCAATTCATCCAG CCACTCT | 50 | SEQ ID NO: 276 |
| hSerpinc1-Cj-25 | E06 | AGAGGTCATCTCGGC CTTCTGC | 59 | SEQ ID NO: 277 |
| hSerpinc1-Cj-26 | E06 | ATTCCATAAGGCATT TCTTGAG | 36 | SEQ ID NO: 278 |
| hSerpinc1-Cj-27 | E07 | TGAAGAAGGCAGTG AAGCAGCT | 50 | SEQ ID NO: 279 |
| hSerpinc1-Cj-28 | E07 | GCGAACGGCCAGCA ATCACAAC | 59 | SEQ ID NO: 280 |
| hSerpinc1-Cj-29 | E07 | GGTTTTTATAAGAGA AGTTCCT | 32 | SEQ ID NO: 281 |
| hSerpinc1-Cj-30 | E07 | TGCAAAGAATAAGA ACATTTTA | 23 | SEQ ID NO: 282 |

**[Table 2]**

| Target sequences of TFPI gene | | | | |
|---|---|---|---|---|
| Target name | Loci. | Target(w/o PAM) | GC Contents (%) | SEQ ID NO |
| hTfpi-Sp-1 | E02 | TGAAGAAAGTACATGC ACTT | 35 | SEQ ID NO: 283 |
| hTfpi-Sp-2 | E02 | GAAGAAAGTACATGCA CTTT | 35 | SEQ ID NO: 284 |
| hTfpi-Sp-3 | E02 | CAGGGGCAAGATTAAG | 55 | SEQ ID NO: 285 |
| | | CAGC | | |
| hTfpi-Sp-4 | E02 | ATCAGCATTAAGAGGG GCAG | 50 | SEQ ID NO: 286 |
| hTfpi-Sp-5 | E02 | AATCAGCATTAAGAGG GGCA | 45 | SEQ ID NO: 287 |
| hTfpi-Sp-6 | E02 | GAATCAGCATTAAGAG GGGC | 50 | SEQ ID NO: 288 |
| hTfpi-Sp-7 | E02 | CTCAGAATCAGCATTAA GAG | 40 | SEQ ID NO: 289 |
| hTfpi-Sp-8 | E02 | CCTCAGAATCAGCATTA AGA | 40 | SEQ ID NO: 290 |
| hTfpi-Sp-9 | E02 | TCCTCAGAATCAGCATT AAG | 40 | SEQ ID NO: 291 |
| hTfpi-Sp-10 | E02 | CCCTCTTAATGCTGATT CTG | 45 | SEQ ID NO: 292 |
| hTfpi-Sp-11 | E02 | GAAGAACACACAATTA TCAC | 35 | SEQ ID NO: 293 |
| hTfpi-Sp-12 | E03 | TTATTTTTACTTTATAG ATA | 10 | SEQ ID NO: 294 |
| hTfpi-Sp-13 | E03 | GAATGCATAAGTTTCAG TGG | 40 | SEQ ID NO: 295 |
| hTfpi-Sp-14 | E03 | AATGAATGCATAAGTTT CAG | 30 | SEQ ID NO: 296 |
| hTfpi-Sp-15 | E03 | GCATTCATTTGTGCAT TCA | 35 | SEQ ID NO: 297 |
| hTfpi-Sp-16 | E03 | TTCATTTTGTGCATTCA AGG | 35 | SEQ ID NO: 298 |
| hTfpi-Sp-17 | E03 | TGTGCATTCAAGGCGGA TGA | 50 | SEQ ID NO: 299 |
| hTfpi-Sp-18 | E03 | TTTTCATGATTGCTTTA CAT | 25 | SEQ ID NO: 300 |
| hTfpi-Sp-19 | E03 | CTTTTCATGATTGCTTT ACA | 30 | SEQ ID NO: 301 |
| hTfpi-Sp-20 | E03 | CAGTGCGAAGAATTTAT ATA | 30 | SEQ ID NO: 302 |
| hTfpi-Sp-21 | E03 | AGTGCGAAGAATTTATA TAT | 25 | SEQ ID NO: 303 |
| hTfpi-Sp-22 | E03 | GTGCGAAGAATTTATAT ATG | 30 | SEQ ID NO: 304 |
| hTfpi-Sp-23 | E03 | TGCGAAGAATTTATATA TGG | 30 | SEQ ID NO: 305 |
| hTfpi-Sp-24 | E03 | TTTATATATGGGGGATG TGA | 35 | SEQ ID NO: 306 |
| hTfpi-Sp-25 | E03 | TCAGAATCGATTTGAAA GTC | 35 | SEQ ID NO: 307 |
| hTfpi-Sp-26 | E03 | TGCAAAAAAATGTGTA CAAG | 30 | SEQ ID NO: 308 |
| hTfpi-Sp-27 | E03 | AAAAAATGTGTACAAG AGGT | 30 | SEQ ID NO: 309 |
| hTfpi-Sp-28 | E04 | TGATTACAGATAATGCA AAC | 30 | SEQ ID NO: 310 |
| hTfpi-Sp-29 | E04 | ATAAAGACAACATTGC AACA | 30 | SEQ ID NO: 311 |
| hTfpi-Sp-30 | E05 | TCTTCCAAAAAGCAGA AATC | 35 | SEQ ID NO: 312 |
| hTfpi-Sp-31 | E05 | AAAGCCAGATTTCTGCT TTT | 35 | SEQ ID NO: 313 |
| hTfpi-Sp-32 | E05 | TGCTTTTGGAAGAAGA TCC | 40 | SEQ ID NO: 314 |
| hTfpi-Sp-33 | E05 | ATATAACCTCGACATAT TCC | 35 | SEQ ID NO: 315 |
| hTfpi-Sp-34 | E05 | GAAGATCCTGGAATAT | 45 | SEQ ID NO: 316 |
| | | GTCG | | |
| hTfpi-Sp-35 | E05 | TATGTCGAGGTTATATT ACC | 35 | SEQ ID NO: 317 |
| hTfpi-Sp-36 | E05 | CTGATTGTTATAAAAAT ACC | 25 | SEQ ID NO: 318 |
| hTfpi-Sp-37 | E05 | CAGTGTGAACGTTTCAA GTA | 40 | SEQ ID NO: 319 |
| hTfpi-Sp-38 | E05 | TGTGAACGTTTCAAGTA TGG | 40 | SEQ ID NO: 320 |
| hTfpi-Sp-39 | E05 | TTTCAAGTATGGTGGAT GCC | 45 | SEQ ID NO: 321 |
| hTfpi-Sp-40 | E05 | TTCAAGTATGGTGGATG CCT | 45 | SEQ ID NO: 322 |
| hTfpi-Sp-41 | E05 | CAAAATTGTTCATATTG CCC | 35 | SEQ ID NO: 323 |
| hTfpi-Sp-42 | E05 | TATGAACAATTTTGAGA CAC | 30 | SEQ ID NO: 324 |
| hTfpi-Sp-43 | E05 | TGCAAGAACATTTGTGA AGA | 35 | SEQ ID NO: 325 |
| hTfpi-Sp-44 | E06 | CTGTATTTTTTCCAGC GAA | 35 | SEQ ID NO: 326 |
| hTfpi-Sp-45 | E06 | TCCACCTGGAAACCATT CGC | 55 | SEQ ID NO: 327 |
| hTfpi-Sp-46 | E06 | TTTTCCAGCGAATGGTT TCC | 45 | SEQ ID NO: 328 |
| hTfpi-Sp-47 | E06 | TCCAGCGAATGGTTTCC AGG | 55 | SEQ ID NO: 329 |
| hTfpi-Sp-48 | E06 | GGGTTCCATAATTATCC ACC | 45 | SEQ ID NO: 330 |
| hTfpi-Sp-49 | E06 | GGTTTCCAGGTGGATAA TTA | 40 | SEQ ID NO: 331 |
| hTfpi-Sp-50 | E06 | GTTATTCACAGCATTGA GCT | 40 | SEQ ID NO: 332 |
| hTfpi-Sp-51 | E06 | AGTTATTCACAGCATTG AGC | 40 | SEQ ID NO: 333 |
| hTfpi-Sp-52 | E06 | CTTGGTTGATTGCGGAG TCA | 50 | SEQ ID NO: 334 |
| hTfpi-Sp-53 | E06 | CCTTGGTTGATTGCGGA GTC | 55 | SEQ ID NO: 335 |
| hTfpi-Sp-54 | E06 | CTGGGAACCTTGGTTGA TTG | 50 | SEQ ID NO: 336 |
| hTfpi-Sp-55 | E06 | CCTGACTCCGCAATCAA CCA | 55 | SEQ ID NO: 337 |
| hTfpi-Sp-56 | E06 | ACCAAAAAGGCTGGGA ACCT | 50 | SEQ ID NO: 338 |
| hTfpi-Sp-57 | E06 | AGATTCTTACCAAAAAG GCT | 35 | SEQ ID NO: 339 |
| hTfpi-Sp-58 | E06 | AAGATTCTTACCAAAAA GGC | 35 | SEQ ID NO: 340 |
| hTfpi-Sp-59 | E06 | ACCAAGGTTCCCAGCCT TTT | 50 | SEQ ID NO: 341 |
| hTfpi-Sp-60 | E06 | CCACAAGATTCTTACCA AAA | 35 | SEQ ID NO: 342 |
| hTfpi-Sp-61 | E06 | CCTTTTTGGTAAGAATC TTG | 35 | SEQ ID NO: 343 |
| hTfpi-Sp-62 | E07 | GTGAAATTCTAAAAAC AATC | 25 | SEQ ID NO: 344 |
| hTfpi-Sp-63 | E07 | TGATTGTTTTTAGAATT TCA | 20 | SEQ ID NO: 345 |
| hTfpi-Sp-64 | E07 | TAGAATTTCACGGTCCC TCA | 45 | SEQ ID NO: 346 |
| hTfpi-Sp-65 | E07 | GCTGGAGTGAGACACC | 55 | SEQ ID NO: 347 |
| | | ATGA | | |
| hTfpi-Sp-66 | E07 | TGCTGGAGTGAGACAC CATG | 55 | SEQ ID NO: 348 |
| hTfpi-Sp-67 | E07 | CGACACAATCCTCTGTC TGC | 55 | SEQ ID NO: 349 |
| hTfpi-Sp-68 | E07 | TGTCTCACTCCAGCAGA CAG | 55 | SEQ ID NO: 350 |
| hTfpi-Sp-69 | E07 | GTAGTAGAATCTGTTCT CAT | 35 | SEQ ID NO: 351 |
| hTfpi-Sp-70 | E07 | TTCTACTACAATTCAGT CAT | 30 | SEQ ID NO: 352 |
| hTfpi-Sp-71 | E07 | TCTACTACAATTCAGTC ATT | 30 | SEQ ID NO: 353 |
| hTfpi-Sp-72 | E07 | ATCCACTGTACTTAAAT GGG | 40 | SEQ ID NO: 354 |
| hTfpi-Sp-73 | E07 | CACATCCACTGTACTTA AAT | 35 | SEQ ID NO: 355 |
| hTfpi-Sp-74 | E07 | CCACATCCACTGTACTT AAA | 40 | SEQ ID NO: 356 |
| hTfpi-Sp-75 | E07 | TGCCGCCCATTTAAGTA CAG | 50 | SEQ ID NO: 357 |
| hTfpi-Sp-76 | E07 | CCATTTAAGTACAGTGG ATG | 40 | SEQ ID NO: 358 |
| hTfpi-Sp-77 | E07 | CATTTAAGTACAGTGGA TGT | 35 | SEQ ID NO: 359 |
| hTfpi-Sp-78 | E07 | ATTTAAGTACAGTGGAT GTG | 35 | SEQ ID NO: 360 |
| hTfpi-Sp-79 | E07 | TTTAAGTACAGTGGATG TGG | 40 | SEQ ID NO: 361 |
| hTfpi-Sp-80 | E07 | TGCCCTCAGACATTCTT GTT | 45 | SEQ ID NO: 362 |
| hTfpi-Sp-81 | E07 | CTTCCAAACAAGAATGT CTG | 40 | SEQ ID NO: 363 |
| hTfpi-Sp-82 | E07 | TTCCAAACAAGAATGTC TGA | 35 | SEQ ID NO: 364 |
| hTfpi-Sp-83 | E07 | TGTCTGAGGGCATGTAA AAA | 40 | SEQ ID NO: 365 |
| hTfpi-Sp-84 | E08 | ATGAAACCTATAAGAG GAAG | 35 | SEQ ID NO: 366 |
| hTfpi-Sp-85 | E08 | CTTTGGATGAAACCTAT AAG | 35 | SEQ ID NO: 367 |
| hTfpi-Sp-86 | E08 | GGCCTCCTTTTGATATT CTT | 40 | SEQ ID NO: 368 |
| hTfpi-Sp-87 | E08 | TTCATCCAAAGAATATC AAA | 25 | SEQ ID NO: 369 |
| hTfpi-Sp-88 | E08 | ATCCAAAGAATATCAA AAGG | 30 | SEQ ID NO: 370 |
| hTfpi-Sp-89 | E08 | TTTTTCTTTTGGTTTTAA TT | 15 | SEQ ID NO: 371 |
| hTfpi-Sp-90 | E08 | CTGCTTCTTTCTTTTTCT TT | 30 | SEQ ID NO: 372 |
| hTfpi-Sa-1 | E02 | TGTGTGTTCTTCATCTTC CT | 40 | SEQ ID NO: 373 |
| hTfpi-Sa-2 | E03 | TTATTTTTACTTTATAG ATA | 10 | SEQ ID NO: 374 |
| hTfpi-Sa-3 | E03 | ATCCGCCTTGAATGCAC AAA | 45 | SEQ ID NO: 375 |
| hTfpi-Sa-4 | E03 | ATTCATTTTGTGCATTC AAG | 30 | SEQ ID NO: 376 |
| hTfpi-Sa-5 | E03 | TACATGGGCCATCATCC GCC | 60 | SEQ ID NO: 377 |
| hTfpi-Sa-6 | E03 | TATATAAATTCTTCGCA | 30 | SEQ ID NO: 378 |
| | | CTG | | |
| hTfpi-Sa-7 | E03 | TATTTTCACTCGACAGT GCG | 45 | SEQ ID NO: 379 |
| hTfpi-Sa-8 | E03 | GTGCGAAGAATTTATAT ATG | 30 | SEQ ID NO: 380 |
| hTfpi-Sa-9 | E03 | ATGGGGATGTGAAGG AAAT | 45 | SEQ ID NO: 381 |
| hTfpi-Sa-10 | E03 | GAATCGATTTGAAAGTC TGG | 40 | SEQ ID NO: 382 |
| hTfpi-Sa-11 | E04 | TTGATTACAGATAATGC AAA | 25 | SEQ ID NO: 383 |
| hTfpi-Sa-12 | E05 | TGCTTTTGGAAGAAGA TCC | 40 | SEQ ID NO: 384 |
| hTfpi-Sa-13 | E05 | AATATAACCTCGACATA TTC | 30 | SEQ ID NO: 385 |
| hTfpi-Sa-14 | E05 | GTGTGAACGTTTCAAGT ATG | 40 | SEQ ID NO: 386 |
| hTfpi-Sa-15 | E05 | GAACAATTTTGAGACAC TGG | 40 | SEQ ID NO: 387 |
| hTfpi-Sa-16 | E06 | TTCCAGCGAATGGTTTC CAG | 50 | SEQ ID NO: 388 |
| hTfpi-Sa-17 | E06 | GAGTTATTCACAGCATT GAG | 40 | SEQ ID NO: 389 |
| hTfpi-Sa-18 | E06 | ATGGAACCCAGCTCAAT GCT | 50 | SEQ ID NO: 390 |
| hTfpi-Sa-19 | E06 | CTTGGTTGATTGCGGAG TCA | 50 | SEQ ID NO: 391 |
| hTfpi-Sa-20 | E06 | CTGGGAACCTTGGTTGA TTG | 50 | SEQ ID NO: 392 |
| hTfpi-Sa-21 | E06 | AGGTTCCCAGCCTTTTT GGT | 50 | SEQ ID NO: 393 |
| hTfpi-Sa-22 | E07 | CGACACAATCCTCTGTC TGC | 55 | SEQ ID NO: 394 |
| hTfpi-Sa-23 | E07 | GTGTCTCACTCCAGCAG ACA | 55 | SEQ ID NO: 395 |
| hTfpi-Sa-24 | E07 | TCCCAATGACTGAATTG TAG | 40 | SEQ ID NO: 396 |
| hTfpi-Sa-25 | E07 | TGGGCGGCATTTCCCAA TGA | 55 | SEQ ID NO: 397 |
| hTfpi-Sa-26 | E07 | ATGCCGCCCATTTAAGT ACA | 45 | SEQ ID NO: 398 |
| hTfpi-Sa-27 | E07 | AAACAATTTTACTTCCA AAC | 25 | SEQ ID NO: 399 |
| hTfpi-Sa-28 | E08 | CCTCTTATAGGTTTCAT CCA | 40 | SEQ ID NO: 400 |
| hTfpi-Sa-29 | E08 | AGGCCTCCTTTTGATAT TCT | 40 | SEQ ID NO: 401 |
| hTfpi-Sa-30 | E08 | GAAATTTTGTTAAAAA TAT | 10 | SEQ ID NO: 402 |
| hTfpi-Cj-1 | E02 | TGGGTTCTGTATTTCAG AGATG | 41 | SEQ ID NO: 403 |
| hTfpi-Cj-2 | E02 | TCTTCATTGTGTAAATC ATCTC | 32 | SEQ ID NO: 404 |
| hTfpi-Cj-3 | E02 | CAGAGATGATTTACACA ATGAA | 32 | SEQ ID NO: 405 |
| hTfpi-Cj -4 | E02 | TGATTTACACAATGAAG AAAGT | 27 | SEQ ID NO: 406 |
| hTfpi-Cj-5 | E02 | CAGGCATACAGAAGCC CAAAGT | 50 | SEQ ID NO: 407 |
| hTfpi-Cj -6 | E02 | GGCAGGGGCAAGATTA AGCAGC | 59 | SEQ ID NO: 408 |
| hTfpi-Cj-7 | E02 | AATGCTGATTCTGAGGA | 41 | SEQ ID NO: 409 |
| | | AGATG | | |
| hTfpi-Cj -8 | E02 | TGCTGATTCTGAGGAAG ATGAA | 41 | SEQ ID NO: 410 |
| hTfpi-Cj -9 | E03 | TACATGGGCCATCATCC GCCTT | 55 | SEQ ID NO: 411 |
| hTfpi-Cj-10 | E03 | TCACATCCCCCATATAT AAATT | 32 | SEQ ID NO: 412 |
| hTfpi-Cj-11 | E03 | AAGTCTGGAAGAGTGC AAAAAA | 36 | SEQ ID NO: 413 |
| hTfpi-Cj-12 | E03 | AACCTACCTCTTGTACA CATTT | 36 | SEQ ID NO: 414 |
| hTfpi-Cj-13 | E03 | AGGGTTCCCAGAAACCT ACCTC | 55 | SEQ ID NO: 415 |
| hTfpi-Cj-14 | E05 | CACTGTTTTGTCTGATT GTTAT | 32 | SEQ ID NO: 416 |
| hTfpi-Cj-15 | E05 | AGGCATCCACCATACTT GAAAC | 45 | SEQ ID NO: 417 |
| hTfpi-Cj-16 | E05 | TTGTTCATATTGCCCAG GCATC | 45 | SEQ ID NO: 418 |
| hTfpi-Cj-17 | E05 | GCCTGGGCAATATGAA CAATTT | 41 | SEQ ID NO: 419 |
| hTfpi-Cj-18 | E07 | CACAATCCTCTGTCTGC TGGAG | 55 | SEQ ID NO: 420 |
| hTfpi-Cj-19 | E07 | TAGTAGAATCTGTTCTC ATTGG | 36 | SEQ ID NO: 421 |
| hTfpi-Cj-20 | E07 | AATTGTAGTAGAATCTG TTCTC | 32 | SEQ ID NO: 422 |
| hTfpi-Cj-21 | E07 | GTCATTGGGAAATGCCG CCCAT | 55 | SEQ ID NO: 423 |
| hTfpi-Cj-22 | E07 | TTGTTTTCATTTCCCCCA CATC | 41 | SEQ ID NO: 424 |

### As one embodiment of the disclosure in the present specification, a guide nucleic acid may be gRNA including a guide sequence complementarily binding to a target sequence of a AT gene and/or TFPI gene.

The "guide sequence" is a nucleotide sequence complementary to partial sequence of either strand of a double strand of a target gene or a nucleic acid. Here, the guide sequence may be a nucleotide sequence having at least 50%, 55%, 60%, 65%, 70%, 75%, 80%, 85%, 90%, 95% or more complementarity or complete complementarity.

The guide sequence may be a sequence of 10 to 25 nucleotides.

In an example, the guide sequence may be a sequence of 10 to 25, 15 to 25 or 20 to 25 nucleotides.

In another example, the guide sequence may be a sequence of 10 to 15, 15 to 20 or 20 to 25 nucleotides.

The target gene disclosed in the specification may be a blood coagulation inhibitory gene.

The target gene disclosed in the specification may be a AT gene (SERPINC1 gene) and/or TFPI gene.

The guide sequence is capable of forming a complementary bond with a target sequence.

The target sequence may be a guide nucleic acid-binding sequence.

The "guide nucleic acid-binding sequence" is a nucleotide sequence having complementarity with a guide sequence included in the guide domain of the guide nucleic acid, and may be complementarily bonded with the guide sequence included in the guide domain of the guide nucleic acid. The target sequence and guide nucleic acid-binding sequence are nucleotide sequences that may vary according to a target gene or a nucleic acid, that is, the subject to be genetically manipulated or edited, and may be designed in various ways according to a target gene or a nucleic acid.

The description related to the target sequence and guide nucleic acid-binding sequence is the same as described above.

The guide nucleic acid-binding sequence may have the same length as a guide sequence.

The guide nucleic acid-binding sequence may have shorter length than a guide sequence.

The guide nucleic acid-binding sequence may have longer length than a guide sequence.

The guide sequence may be a nucleotide sequence having at least 50%, 55%, 60%, 65%, 70%, 75%, 80%, 85%, 90% , 95% or more complementarity or complete complementarity to the guide nucleic acid-binding sequence.

In one example, the guide sequence may have or include a 1 to 8-nucleotide sequence which is not complementary to guide nucleic acid-binding sequence.

In one embodiment, the guide sequence disclosed in the present specification may form a complementary bond with a 10 to 35-nt contiguous sequence located in a promoter region of a blood coagulation inhibitory gene.

In one example, the guide sequence may form a complementary bond with a 10 to 25-nt contiguous sequence located in a promoter region of an AT gene.

In another example, the guide sequence may form a complementary bond with a 10 to 25-nt contiguous sequence located in a promoter region of an TFPI gene.

The guide sequence disclosed in the present specification may form a complementary bond with a 10 to 35-nt contiguous sequence located in an intron region of the blood coagulation inhibitory gene.

In one example, the guide sequence may form a complementary bond with a 10 to 25-nt contiguous sequence located in an intron region of an AT gene.

In another example, the guide sequence may form a complementary bond with a 10 to 25-nt contiguous sequence located in an intron region of an TFPI gene.

The guide sequence disclosed in the present specification may form a complementary bond with a 10 to 35-nt contiguous sequence located in an exon region of the blood coagulation inhibitory gene.

In one example, the guide sequence may form a complementary bond with a 10 to 25-nt contiguous sequence located in an exon region of an AT gene.

In another example, the guide sequence may form a complementary bond with a 10 to 25-nt contiguous sequence located in an exon region of an TFPI gene.

The guide sequence disclosed in the present specification may form a complementary bond with a 10 to 35-nt contiguous sequence located in an enhancer region of the blood coagulation inhibitory gene.

In one example, the guide sequence may form a complementary bond with a 10 to 25-nt contiguous sequence located in an enhancer region of an AT gene.

In another example, the guide sequence may form a complementary bond with a 10 to 25-nt contiguous sequence located in an enhancer region of an TFPI gene.

The guide sequence disclosed in the present specification may form a complementary bond with a 10 to 35-nt contiguous sequence located in a coding region, a non-coding region or a mixed region of the blood coagulation inhibitory gene.

In one example, the guide sequence may form a complementary bond with a 10 to 25-nt contiguous sequence located in a coding region, a non-coding region or a mixed region of an AT gene.

In another example, the guide sequence may form a complementary bond with a 10 to 25-nt contiguous sequence located in a coding region, a non-coding region or a mixed region of an TFPI gene.

The guide sequence disclosed in the present specification may form a complementary bond with a 10 to 35-nt contiguous sequence located in a promoter, an enhancer, a 3' UTR, a polyA region or a mixed region of the blood coagulation inhibitory gene.

In one example, the guide sequence may form a complementary bond with a 10 to 25-nt contiguous sequence located in a promoter, an enhancer, a 3' UTR, a polyA region or a mixed region of an AT gene.

In another example, the guide sequence may form a complementary bond with a 10 to 25-nt contiguous sequence located in a promoter, an enhancer, a 3' UTR, a polyA region or a mixed region of an TFPI gene.

The guide sequence disclosed in the present specification may form a complementary bond with a 10 to 35-nt contiguous sequence located in an exon, an intron or a mixed region of the blood coagulation inhibitory gene.

In one example, the guide sequence may form a complementary bond with a 10 to 25-nt contiguous sequence located in an exon, an intron or a mixed region of an AT gene.

In another example, the guide sequence may form a complementary bond with a 10 to 25-nt contiguous sequence located in an exon, an intron or a mixed region of an TFPI gene.

The guide sequence disclosed in the present specification may form a complementary bond with a 10 to 35-nt contiguous sequence which includes or is adjacent to a mutant part (e. g, a part different from a wild-type gene) of the blood coagulation inhibitory gene.

In one example, the guide sequence may form a complementary bond with a 10 to 25-nt contiguous sequence which includes or is adjacent to a mutant part (e. g, a part different from a wild-type gene) of an AT gene.

In another example, the guide sequence may form a complementary bond with a 10 to 25-nt contiguous sequence which includes or is adjacent to a mutant part (e. g, a part different from a wild-type gene) of an TFPI gene.

The guide sequence disclosed in the present specification may form a complementary bond with a 10 to 35-nt contiguous sequence which is adjacent to the 5' end and/or 3' end of a proto-spacer-adjacent motif (PAM) sequence in the nucleic acid sequence of the blood coagulation inhibitory gene.

The "proto-spacer-adjacent motif (PAM) sequence" is a nucleotide sequence that can be recognized by an editor protein. Here, the PAM sequence may have different nucleotide sequences according to the type of the editor protein and an editor protein-derived species.

Here, the PAM sequence may be, for example, one or more sequences of the following sequences (described in a 5' to 3' direction).

NGG (N is A, T, C or G);
NNNNRYAC (N is each independently A, T, C or G, R is A or G, and Y is C or T);
NNAGAAW (N is each independently A, T, C or G, and W is A or T);
NNNNGATT (N is each independently A, T, C or G);
NNGRR(T) (N is each independently A, T, C or G, and R is A or G); and
TTN (N is A, T, C or G).

In one example, the guide sequence may form a complementary bond with a 10 to 25-nt contiguous sequence which is adjacent to the 5' end and/or 3' end of a proto-spacer-adjacent motif (PAM) sequence in the nucleic acid sequence of an AT gene.

In one exemplary embodiment, when the PAM sequence recognized by an editor protein is 5'-NGG-3', 5'-NAG-3' and/or 5'-NGA-3' (N= A, T, G or C; or A, U, G or C), the guide sequence may form a complementary bond with a 10 to 25-nt contiguous sequence which is adjacent to the 5' end and/or 3' end of 5'-NGG-3', 5'-NAG-3' and/or 5'-NGA-3' (N= A, T, G or C; or A, U, G or C) sequence in the nucleic acid sequence of an AT gene.
In another exemplary embodiment, when the PAM sequence recognized by an editor protein is 5'-NGGNG-3' and/or 5'-NNAGAAW-3' (W = A or T, N= A, T, G or C; or A, U, G or C), the guide sequence may form a complementary bond with a 10 to 25-nt contiguous sequence which is adjacent to the 5' end and/or 3' end of 5'-NGGNG-3' and/or 5'-NNAGAAW-3' (W = A or T, N= A, T, G or C; or A, U, G or C) sequence in the nucleic acid sequence of an AT gene.
In another exemplary embodiment, when the PAM sequence recognized by an editor protein is 5'-NNNNGATT-3' and/or 5'-NNNGCTT-3' (N= A, T, G or C; or A, U, G or C), the guide sequence may form a complementary bond with a 10 to 25-nt contiguous sequence which is adjacent to the 5' end and/or 3' end of 5'-NNNNGATT-3' and/or 5'-NNNGCTT-3' (N= A, T, G or C; or A, U, G or C) sequence in the nucleic acid sequence of an AT gene.
In one exemplary embodiment, when the PAM sequence recognized by an editor protein is 5'-NNNVRYAC-3' (V = G, C or A; R = A or G, Y = C or T, N= A, T, G or C; or A, U, G or C), the guide sequence may form a complementary bond with a 10 to 25-nt contiguous sequence which is adjacent to the 5' end and/or 3' end of 5'-NNNVRYAC-3' (V = G, C or A; R = A or G, Y = C or T, N= A, T, G or C; or A, U, G or C) sequence in the nucleic acid sequence of an AT gene.

In another exemplary embodiment, when the PAM sequence recognized by an editor protein is 5'-NAAR-3'(R = A or G, N= A, T, G or C; or A, U, G or C), the guide sequence may form a complementary bond with a 10 to 25-nt contiguous sequence which is adjacent to the 5' end and/or 3' end of 5'-NAAR-3'(R = A or G, N= A, T, G or C; or A, U, G or C) sequence in the nucleic acid sequence of an AT gene.

In another exemplary embodiment, when the PAM sequence recognized by an editor protein is 5'-NNGRR-3', 5'-NNGRRT-3' and/or 5'-NNGRRV-3' (R = A or G, V = G, C or A, N= A, T, G or C; or A, U, G or C), the guide sequence may form a complementary bond with a 10 to 25-nt contiguous sequence which is adjacent to the 5' end and/or 3' end of 5'-NNGRR-3', 5'-NNGRRT-3' and/or 5'-NNGRRV-3' (R = A or G, V = G, C or A, N= A, T, G or C; or A, U, G or C) sequence in the nucleic acid sequence of an AT gene.
In one exemplary embodiment, when the PAM sequence recognized by an editor protein is 5'-TTN-3' (N= A, T, G or C; or A, U, G or C), the guide sequence may form a complementary bond with a 10 to 25-nt contiguous sequence which is adjacent to the 5' end and/or 3' end of 5'-TTN-3' (N= A, T, G or C; or A, U, G or C) sequence in the nucleic acid sequence of an AT gene.

In another example, the guide sequence may form a complementary bond with a 10 to 25-nt contiguous sequence which is adjacent to the 5' end and/or 3' end of a proto-spacer-adjacent motif (PAM) sequence in the nucleic acid sequence of an TFPI gene.

In one exemplary embodiment, when the PAM sequence recognized by an editor protein is 5'-NGG-3', 5'-NAG-3' and/or 5'-NGA-3' (N= A, T, G or C; or A, U, G or C), the guide sequence may form a complementary bond with a 10 to 25-nt contiguous sequence which is adjacent to the 5' end and/or 3' end of 5'-NGG-3', 5'-NAG-3' and/or 5'-NGA-3' (N= A, T, G or C; or A, U, G or C) sequence in the nucleic acid sequence of an TFPI gene.
In another exemplary embodiment, when the PAM sequence recognized by an editor protein is 5'-NGGNG-3' and/or 5'-NNAGAAW-3' (W = A or T, N= A, T, G or C; or A, U, G or C), the guide sequence may form a complementary bond with a 10 to 25-nt contiguous sequence which is adjacent to the 5' end and/or 3' end of 5'-NGGNG-3' and/or 5'-NNAGAAW-3' (W = A or T, N= A, T, G or C; or A, U, G or C) sequence in the nucleic acid sequence of an TFPI gene.
In another exemplary embodiment, when the PAM sequence recognized by an editor protein is 5'-NNNNGATT-3' and/or 5'-NNNGCTT-3' (N= A, T, G or C; or A, U, G or C), the guide sequence may form a complementary bond with a 10 to 25-nt contiguous sequence which is adjacent to the 5' end and/or 3' end of 5'-NNNNGATT-3' and/or 5'-NNNGCTT-3' (N= A, T, G or C; or A, U, G or C) sequence in the nucleic acid sequence of an TFPI gene.
In one exemplary embodiment, when the PAM sequence recognized by an editor protein is 5'-NNNVRYAC-3' (V = G, C or A; R = A or G, Y = C or T, N= A, T, G or C; or A, U, G or C), the guide sequence may form a complementary bond with a 10 to 25-nt contiguous sequence which is adjacent to the 5' end and/or 3' end of 5'-NNNVRYAC-3' (V = G, C or A; R = A or G, Y = C or T, N= A, T, G or C; or A, U, G or C) sequence in the nucleic acid sequence of an TFPI gene.

In another exemplary embodiment, when the PAM sequence recognized by an editor protein is 5'-NAAR-3'(R = A or G, N= A, T, G or C; or A, U, G or C), the guide sequence may form a complementary bond with a 10 to 25-nt contiguous sequence which is adjacent to the 5' end and/or 3' end of 5'-NAAR-3'(R = A or G, N= A, T, G or C; or A, U, G or C) sequence in the nucleic acid sequence of an TFPI gene.

In another exemplary embodiment, when the PAM sequence recognized by an editor protein is 5'-NNGRR-3', 5'-NNGRRT-3' and/or 5'-NNGRRV-3' (R = A or G, V = G, C or A, N= A, T, G or C; or A, U, G or C), the guide sequence may form a complementary bond with a 10 to 25-nt contiguous sequence which is adjacent to the 5' end and/or 3' end of 5'-NNGRR-3', 5'-NNGRRT-3' and/or 5'-NNGRRV-3' (R = A or G, V = G, C or A, N= A, T, G or C; or A, U, G or C) sequence in the nucleic acid sequence of an TFPI gene.

In one exemplary embodiment, when the PAM sequence recognized by an editor protein is 5'-TTN-3' (N= A, T, G or C; or A, U, G or C), the guide sequence may form a complementary bond with a 10 to 25-nt contiguous sequence which is adjacent to the 5' end and/or 3' end of 5'-TTN-3' (N= A, T, G or C; or A, U, G or C) sequence in the nucleic acid sequence of an TFPI gene.

Hereinafter, examples of guide sequences that can be used in an exemplary embodiment disclosed in the specification are listed in Tables 3 and 4. The guide sequences disclosed in Tables 3 and 4 are guide sequences capable of targeting an AT(SERPINC1 gene) or TFPI gene, and may form a complementary bond with a target sequence located in an AT(SERPINC1 gene) or TFPI gene. The guide sequences disclosed in table 3 and 4 are guide sequences capable of targeting the target sequence of table 1 and 2, respectively. In addition, target names shown in Tables 3 and 4 were named Sp for SpCas9, Sa for SaCas9 and Cj for CjCas9 according to an editor protein.

**[Table 3]**

| Guide sequences capable of targeting SERPINC1 gene(AT gene) |
|---|
| Loci. |
| Exon 01(E01) |
| E01 |
| E01 |
| E01 |
| E01 |
| E01 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E02 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E03 |
| E04 |
| E04 |
| E04 |
| E04 |
| E04 |
| E04 |
| hSerpinc 1-Sp-99 |
| hSerpinc1-Sp-100 |
| hSerpinc1-Sp-101 |
| hSerpinc1-Sp-102 |
| hSerpinc1-Sp-103 |
| hSerpinc1-Sp-104 |
| hSerpinc1-Sp-105 |
| hSerpinc1-Sp-106 |
| hSerpinc1-Sp-107 |
| hSerpinc1-Sp-108 |
| hSerpinc1-Sp-109 |
| hSerpinc1-Sp-110 |
| hSerpinc1-Sp-111 |
| hSerpinc1-Sp-112 |
| hSerpinc1-Sp-113 |
| hSerpinc1-Sp-114 |
| hSerpinc1-Sp-115 |
| hSerpinc1-Sp-116 |
| hSerpinc1-Sp-117 |
| hSerpinc1-Sp-118 |
| hSerpinc1-Sp-119 |
| hSerpinc1-Sp-120 |
| hSerpinc1-Sp-121 |
| hSerpinc1-Sp-122 |
| hSerpinc1-Sp-123 |
| hSerpinc1-Sp-124 |
| hSerpinc1-Sp-125 |
| hSerpinc1-Sp-126 |
| hSerpinc1-Sp-127 |
| hSerpinc1-Sp-128 |
| hSerpinc1-Sp-129 |
| hSerpinc1-Sp-130 |
| hSerpinc1-Sp-131 |
| hSerpinc1-Sp-132 |
| hSerpinc1-Sp-133 |
| hSerpinc 1-Sp-134 |
| hSerpinc 1-Sp-135 |
| hSerpinc1-Sp-136 |
| hSerpinc 1-Sp-137 |
| hSerpinc 1-Sp-138 |
| hSerpinc1-Sp-139 |
| hSerpinc1-Sp-140 |
| hSerpinc1-Sp-141 |
| hSerpinc1-Sp-142 |
| hSerpinc1-Sp-143 |
| hSerpinc1-Sp-144 |
| hSerpinc1-Sp-145 |
| hSerpinc1-Sp-146 |
| hSerpinc1-Sp-147 |
| hSerpinc1-Sp-148 |
| hSerpinc1-Sp-149 |
| hSerpinc1-Sp-150 |
| hSerpinc1-Sp-151 |
| hSerpinc1-Sp-152 |
| hSerpinc1-Sp-153 |
| hSerpinc1-Sp-154 |
| hSerpinc1-Sp-155 |
| hSerpinc1-Sp-156 |
| hSerpinc1-Sp-157 |
| hSerpinc1-Sp-158 |
| hSerpinc1-Sp-159 |
| hSerpinc1-Sp-160 |
| hSerpinc1-Sp-161 |
| hSerpinc1-Sp-162 |
| hSerpinc1-Sp-163 |
| hSerpinc1-Sp-164 |
| hSerpinc1-Sp-165 |
| hSerpinc1-Sp-166 |
| hSerpinc1-Sp-167 |
| hSerpinc1-Sp-168 |
| hSerpinc1-Sp-169 |
| hSerpinc1-Sp-170 |
| hSerpinc1-Sp-171 |
| hSerpinc1-Sp-172 |
| hSerpinc1-Sp-173 |
| hSerpinc1-Sp-174 |
| hSerpinc1-Sp-175 |
| hSerpinc1-Sp-176 |
| hSerpinc1-Sp-177 |
| hSerpinc1-Sp-178 |
| hSerpinc1-Sp-179 |
| hSerpinc1-Sp-180 |
| hSerpinc1-Sp-181 |
| hSerpinc1-Sp-182 |
| hSerpinc1-Sp-183 |
| hSerpinc1-Sp-184 |
| hSerpinc1-Sp-185 |
| hSerpinc1-Sp-186 |
| hSerpinc1-Sp-187 |
| hSerpinc1-Sp-188 |
| hSerpinc1-Sp-189 |
| hSerpinc1-Sp-190 |
| hSerpinc1-Sp-191 |
| hSerpinc1-Sp-192 |
| hSerpinc1-Sp-193 |
| hSerpinc1-Sp-194 |
| hSerpinc1-Sp-195 |
| hSerpinc1-Sp-196 |
| hSerpinc1-Sp-197 |
| hSerpinc1-Sa-1 |
| hSerpinc1-Sa-2 |
| hSerpinc 1-Sa-3 |
| hSerpinc1-Sa-4 |
| hSerpinc1-Sa-5 |
| hSerpinc1-Sa-6 |
| hSerpinc1-Sa-7 |
| hSerpinc1-Sa-8 |
| hSerpinc1-Sa-9 |
| hSerpinc 0 |
| hSerpinc1-Sa-11 |
| hSerpinc1-Sa-12 |
| hSerpinc1-Sa-13 |
| hSerpinc1-Sa-14 |
| hSerpinc1-Sa-15 |
| hSerpinc1-Sa-16 |
| hSerpinc1-Sa-17 |
| hSerpinc1-Sa-18 |
| hSerpinc1-Sa-19 |
| hSerpinc1-Sa-20 |
| hSerpinc1-Sa-21 |
| hSerpinc1-Sa-22 |
| hSerpinc 1-Sa-23 |
| hSerpinc1-Sa-24 |
| hSerpinc1-Sa-25 |
| hSerpinc1-Sa-26 |
| hSerpinc 1-Sa-27 |
| hSerpinc1-Sa-28 |
| hSerpinc1-Sa-29 |
| hSerpinc1-Sa-30 |
| hSerpinc 1-Sa-31 |
| hSerpinc1-Sa-32 |
| hSerpinc1-Sa-33 |
| hSerpinc1-Sa-34 |
| hSerpinc 1-Sa-35 |
| hSerpinc1-Sa-36 |
| hSerpinc1-Sa-37 |
| hSerpinc1-Cj-1 |
| hSerpinc 1-Cj-2 |
| hSerpinc 1-Cj-3 |
| hSerpinc 1-Cj-4 |
| hSerpinc 1-Cj-5 |
| hSerpinc 1-Cj-6 |
| hSerpinc 1-Cj-7 |
| hSerpinc 1-Cj-8 |
| hSerpinc 1-Cj-9 |
| hSerpinc1-Cj-10 |
| hSerpinc1-Cj-11 |
| hSerpinc1-Cj-12 |
| hSerpinc1-Cj-13 |
| hSerpinc1-Cj-14 |
| hSerpinc1-Cj-15 |
| hSerpinc1-Cj-16 |
| hSerpinc1-Cj-17 |
| hSerpinc1-Cj-18 |
| hSerpinc1-Cj-19 |
| hSerpinc1-Cj-20 |
| hSerpinc1-Cj-21 |
| hSerpinc1-Cj-22 |
| hSerpinc1-Cj-23 |
| hSerpinc1-Cj-24 |
| hSerpinc1-Cj-25 |
| hSerpinc1-Cj-26 |
| hSerpinc1-Cj-27 |
| hSerpinc1-Cj-28 |
| hSerpinc1-Cj-29 |
| hSerpinc1-Cj-30 |

**[Table 4]**

| Guide sequences capable of targeting TFPI gene | | | |
|---|---|---|---|
| Target name | Loci. | Guide sequence | SEQ ID NO |
| hTfpi-Sp-1 | E02 | UGAAGAAAGUACAUGC ACUU | SEQ ID NO: 689 |
| hTfpi-Sp-2 | E02 | GAAGAAAGUACAUGCA CUUU | SEQ ID NO: 690 |
| hTfpi-Sp-3 | E02 | CAGGGGCAAGAUUAAG CAGC | SEQ ID NO: 691 |
| hTfpi-Sp-4 | E02 | AUCAGCAUUAAGAGGG GCAG | SEQ ID NO: 692 |
| hTfpi-Sp-5 | E02 | AAUCAGCAUUAAGAGG GGCA | SEQ ID NO: 693 |
| hTfpi-Sp-6 | E02 | GAAUCAGCAUUAAGAG GGGC | SEQ ID NO: 694 |
| hTfpi-Sp-7 | E02 | CUCAGAAUCAGCAUUA AGAG | SEQ ID NO: 695 |
| hTfpi-Sp-8 | E02 | CCUCAGAAUCAGCAUU AAGA | SEQ ID NO: 696 |
| hTfpi-Sp-9 | E02 | UCCUCAGAAUCAGCAU UAAG | SEQ ID NO: 697 |
| hTfpi-Sp-10 | E02 | CCCUCUUAAUGCUGAU UCUG | SEQ ID NO: 698 |
| hTfpi-Sp-11 | E02 | GAAGAACACACAAUUA UCAC | SEQ ID NO: 699 |
| hTfpi-Sp-12 | E03 | UUAUUUUUACUUUAUA | SEQ ID NO: 700 |
| | | GAUA | |
| hTfpi-Sp-13 | E03 | GAAUGCAUAAGUUUCA GUGG | SEQ ID NO: 701 |
| hTfpi-Sp-14 | E03 | AAUGAAUGCAUAAGUU UCAG | SEQ ID NO: 702 |
| hTfpi-Sp-15 | E03 | GCAUUCAUUUUGUGCA UUCA | SEQ ID NO: 703 |
| hTfpi-Sp-16 | E03 | UUCAUUUUGUGCAUUC AAGG | SEQ ID NO: 704 |
| hTfpi-Sp-17 | E03 | UGUGCAUUCAAGGCGG AUGA | SEQ ID NO: 705 |
| hTfpi-Sp-18 | E03 | UUUUCAUGAUUGCUUU ACAU | SEQ ID NO: 706 |
| hTfpi-Sp-19 | E03 | CUUUUCAUGAUUGCUU UACA | SEQ ID NO: 707 |
| hTfpi-Sp-20 | E03 | CAGUGCGAAGAAUUUA UAUA | SEQ ID NO: 708 |
| hTfpi-Sp-21 | E03 | AGUGCGAAGAAUUUAU AUAU | SEQ ID NO: 709 |
| hTfpi-Sp-22 | E03 | GUGCGAAGAAUUUAUA UAUG | SEQ ID NO: 710 |
| hTfpi-Sp-23 | E03 | UGCGAAGAAUUUAUAU AUGG | SEQ ID NO: 711 |
| hTfpi-Sp-24 | E03 | UUUAUAUAUGGGGGAU GUGA | SEQ ID NO: 712 |
| hTfpi-Sp-25 | E03 | UCAGAAUCGAUUUGAA AGUC | SEQ ID NO: 713 |
| hTfpi-Sp-26 | E03 | UGCAAAAAAAUGUGUA CAAG | SEQ ID NO: 714 |
| hTfpi-Sp-27 | E03 | AAAAAAUGUGUACAAG AGGU | SEQ ID NO: 715 |
| hTfpi-Sp-28 | E04 | UGAUUACAGAUAAUGC AAAC | SEQ ID NO: 716 |
| hTfpi-Sp-29 | E04 | AUAAAGACAACAUUGC AACA | SEQ ID NO: 717 |
| hTfpi-Sp-30 | E05 | UCUUCCAAAAAGCAGA AAUC | SEQ ID NO: 718 |
| hTfpi-Sp-31 | E05 | AAAGCCAGAUUUCUGC UUUU | SEQ ID NO: 719 |
| hTfpi-Sp-32 | E05 | UGCUUUUUGGAAGAAG AUCC | SEQ ID NO: 720 |
| hTfpi-Sp-33 | E05 | AUAUAACCUCGACAUA UUCC | SEQ ID NO: 721 |
| hTfpi-Sp-34 | E05 | GAAGAUCCUGGAAUAU GUCG | SEQ ID NO: 722 |
| hTfpi-Sp-35 | E05 | UAUGUCGAGGUUAUAU UACC | SEQ ID NO: 723 |
| hTfpi-Sp-36 | E05 | CUGAUUGUUAUAAAAA UACC | SEQ ID NO: 724 |
| hTfpi-Sp-37 | E05 | CAGUGUGAACGUUUCA AGUA | SEQ ID NO: 725 |
| hTfpi-Sp-38 | E05 | UGUGAACGUUUCAAGU AUGG | SEQ ID NO: 726 |
| hTfpi-Sp-39 | E05 | UUUCAAGUAUGGUGGA UGCC | SEQ ID NO: 727 |
| hTfpi-Sp-40 | E05 | UUCAAGUAUGGUGGAU GCCU | SEQ ID NO: 728 |
| hTfpi-Sp-41 | E05 | CAAAAUUGUUCAUAUU GCCC | SEQ ID NO: 729 |
| hTfpi-Sp-42 | E05 | UAUGAACAAUUUUGAG ACAC | SEQ ID NO: 730 |
| hTfpi-Sp-43 | E05 | UGCAAGAACAUUUGUG | SEQ ID NO: 731 |
| | | AAGA | |
| hTfpi-Sp-44 | E06 | CUGUAUUUUUUCCAG CGAA | SEQ ID NO: 732 |
| hTfpi-Sp-45 | E06 | UCCACCUGGAAACCAU UCGC | SEQ ID NO: 733 |
| hTfpi-Sp-46 | E06 | UUUUCCAGCGAAUGGU UUCC | SEQ ID NO: 734 |
| hTfpi-Sp-47 | E06 | UCCAGCGAAUGGUUUC CAGG | SEQ ID NO: 735 |
| hTfpi-Sp-48 | E06 | GGGUUCCAUAAUUAUC CACC | SEQ ID NO: 736 |
| hTfpi-Sp-49 | E06 | GGUUUCCAGGUGGAUA AUUA | SEQ ID NO: 737 |
| hTfpi-Sp-50 | E06 | GUUAUUCACAGCAUUG AGCU | SEQ ID NO: 738 |
| hTfpi-Sp-51 | E06 | AGUUAUUCACAGCAUU GAGC | SEQ ID NO: 739 |
| hTfpi-Sp-52 | E06 | CUUGGUUGAUUGCGGA GUCA | SEQ ID NO: 740 |
| hTfpi-Sp-53 | E06 | CCUUGGUUGAUUGCGG AGUC | SEQ ID NO: 741 |
| hTfpi-Sp-54 | E06 | CUGGGAACCUUGGUUG AUUG | SEQ ID NO: 742 |
| hTfpi-Sp-55 | E06 | CCUGACUCCGCAAUCA ACCA | SEQ ID NO: 743 |
| hTfpi-Sp-56 | E06 | ACCAAAAAGGCUGGGA ACCU | SEQ ID NO: 744 |
| hTfpi-Sp-57 | E06 | AGAUUCUUACCAAAAA GGCU | SEQ ID NO: 745 |
| hTfpi-Sp-58 | E06 | AAGAUUCUUACCAAAA AGGC | SEQ ID NO: 746 |
| hTfpi-Sp-59 | E06 | ACCAAGGUUCCCAGCC UUUU | SEQ ID NO: 747 |
| hTfpi-Sp-60 | E06 | CCACAAGAUUCUUACC AAAA | SEQ ID NO: 748 |
| hTfpi-Sp-61 | E06 | CCUUUUUGGUAAGAAU CUUG | SEQ ID NO: 749 |
| hTfpi-Sp-62 | E07 | GUGAAAUUCUAAAAAC AAUC | SEQ ID NO: 750 |
| hTfpi-Sp-63 | E07 | UGAUUGUUUUUAGAAU UUCA | SEQ ID NO: 751 |
| hTfpi-Sp-64 | E07 | UAGAAUUUCACGGUCC CUCA | SEQ ID NO: 752 |
| hTfpi-Sp-65 | E07 | GCUGGAGUGAGACACC AUGA | SEQ ID NO: 753 |
| hTfpi-Sp-66 | E07 | UGCUGGAGUGAGACAC CAUG | SEQ ID NO: 754 |
| hTfpi-Sp-67 | E07 | CGACACAAUCCUCUGU CUGC | SEQ ID NO: 755 |
| hTfpi-Sp-68 | E07 | UGUCUCACUCCAGCAG ACAG | SEQ ID NO: 756 |
| hTfpi-Sp-69 | E07 | GUAGUAGAAUCUGUUC UCAU | SEQ ID NO: 757 |
| hTfpi-Sp-70 | E07 | UUCUACUACAAUUCAG UCAU | SEQ ID NO: 758 |
| hTfpi-Sp-71 | E07 | UCUACUACAAUUCAGU CAUU | SEQ ID NO: 759 |
| hTfpi-Sp-72 | E07 | AUCCACUGUACUUAAA UGGG | SEQ ID NO: 760 |
| hTfpi-Sp-73 | E07 | CACAUCCACUGUACUU AAAU | SEQ ID NO: 761 |
| hTfpi-Sp-74 | E07 | CCACAUCCACUGUACU | SEQ ID NO: 762 |
| | | UAAA | |
| hTfpi-Sp-75 | E07 | UGCCGCCCAUUUAAGU ACAG | SEQ ID NO: 763 |
| hTfpi-Sp-76 | E07 | CCAUUUAAGUACAGUG GAUG | SEQ ID NO: 764 |
| hTfpi-Sp-77 | E07 | CAUUUAAGUACAGUGG AUGU | SEQ ID NO: 765 |
| hTfpi-Sp-78 | E07 | AUUUAAGUACAGUGGA UGUG | SEQ ID NO: 766 |
| hTfpi-Sp-79 | E07 | UUUAAGUACAGUGGAU GUGG | SEQ ID NO: 767 |
| hTfpi-Sp-80 | E07 | UGCCCUCAGACAUUCU UGUU | SEQ ID NO: 768 |
| hTfpi-Sp-81 | E07 | CUUCCAAACAAGAAUG UCUG | SEQ ID NO: 769 |
| hTfpi-Sp-82 | E07 | UUCCAAACAAGAAUGU CUGA | SEQ ID NO: 770 |
| hTfpi-Sp-83 | E07 | UGUCUGAGGGCAUGUA AAAA | SEQ ID NO: 771 |
| hTfpi-Sp-84 | E08 | AUGAAACCUAUAAGAG GAAG | SEQ ID NO: 772 |
| hTfpi-Sp-85 | E08 | CUUUGGAUGAAACCUA UAAG | SEQ ID NO: 773 |
| hTfpi-Sp-86 | E08 | GGCCUCCUUUGAUAU UCUU | SEQ ID NO: 774 |
| hTfpi-Sp-87 | E08 | UUCAUCCAAAGAAUAU CAAA | SEQ ID NO: 775 |
| hTfpi-Sp-88 | E08 | AUCCAAAGAAUAUCAA AAGG | SEQ ID NO: 776 |
| hTfpi-Sp-89 | E08 | UUUUCUUUUGGUUUU AAUU | SEQ ID NO: 777 |
| hTfpi-Sp-90 | E08 | CUGCUUCUUUCUUUUU CUUU | SEQ ID NO: 778 |
| hTfpi-Sa-1 | E02 | UGUGUGUUCUUCAUCU UCCU | SEQ ID NO: 779 |
| hTfpi-Sa-2 | E03 | UUAUUUUUACUUUAUA GAUA | SEQ ID NO: 780 |
| hTfpi-Sa-3 | E03 | AUCCGCCUUGAAUGCA CAAA | SEQ ID NO: 781 |
| hTfpi-Sa-4 | E03 | AUUCAUUUUGUGCAUU CAAG | SEQ ID NO: 782 |
| hTfpi-Sa-5 | E03 | UACAUGGGCCAUCAUC CGCC | SEQ ID NO: 783 |
| hTfpi-Sa-6 | E03 | UAUAUAAAUUCUUCGC ACUG | SEQ ID NO: 784 |
| hTfpi-Sa-7 | E03 | UAUUUUCACUCGACAG UGCG | SEQ ID NO: 785 |
| hTfpi-Sa-8 | E03 | GUGCGAAGAAUUUAUA UAUG | SEQ ID NO: 786 |
| hTfpi-Sa-9 | E03 | AUGGGGAUGUGAAGG AAAU | SEQ ID NO: 787 |
| hTfpi-Sa-10 | E03 | GAAUCGAUUUGAAAGU CUGG | SEQ ID NO: 788 |
| hTfpi-Sa-11 | E04 | UUGAUUACAGAUAAUG CAAA | SEQ ID NO: 789 |
| hTfpi-Sa-12 | E05 | UGCUUUUUGGAAGAAG AUCC | SEQ ID NO: 790 |
| hTfpi-Sa-13 | E05 | AAUAUAACCUCGACAU AUUC | SEQ ID NO: 791 |
| hTfpi-Sa-14 | E05 | GUGUGAACGUUUCAAG UAUG | SEQ ID NO: 792 |
| hTfpi-Sa-15 | E05 | GAACAAUUUGAGACA | SEQ ID NO: 793 |
| | | CUGG | |
| hTfpi-Sa-16 | E06 | UUCCAGCGAAUGGUUU CCAG | SEQ ID NO: 794 |
| hTfpi-Sa-17 | E06 | GAGUUAUUCACAGCAU UGAG | SEQ ID NO: 795 |
| hTfpi-Sa-18 | E06 | AUGGAACCCAGCUCAA UGCU | SEQ ID NO: 796 |
| hTfpi-Sa-19 | E06 | CUUGGUUGAUUGCGGA GUCA | SEQ ID NO: 797 |
| hTfpi-Sa-20 | E06 | CUGGGAACCUUGGUUG AUUG | SEQ ID NO: 798 |
| hTfpi-Sa-21 | E06 | AGGUUCCCAGCCUUUU UGGU | SEQ ID NO: 799 |
| hTfpi-Sa-22 | E07 | CGACACAAUCCUCUGU CUGC | SEQ ID NO: 800 |
| hTfpi-Sa-23 | E07 | GUGUCUCACUCCAGCA GACA | SEQ ID NO: 801 |
| hTfpi-Sa-24 | E07 | UCCCAAUGACUGAAUU GUAG | SEQ ID NO: 802 |
| hTfpi-Sa-25 | E07 | UGGGCGGCAUUUCCCA AUGA | SEQ ID NO: 803 |
| hTfpi-Sa-26 | E07 | AUGCCGCCCAUUUAAG UACA | SEQ ID NO: 804 |
| hTfpi-Sa-27 | E07 | AAACAAUUUUACUUCC AAAC | SEQ ID NO: 805 |
| hTfpi-Sa-28 | E08 | CCUCUUAUAGGUUUCA UCCA | SEQ ID NO: 806 |
| hTfpi-Sa-29 | E08 | AGGCCUCCUUUUGAUA UUCU | SEQ ID NO: 807 |
| hTfpi-Sa-30 | E08 | GAAAUUUUGUUAAAA AUAU | SEQ ID NO: 808 |
| hTfpi-Cj-1 | E02 | UGGGUUCUGUAUUUCA GAGAUG | SEQ ID NO: 809 |
| hTfpi-Cj-2 | E02 | UCUUCAUUGUGUAAAU CAUCUC | SEQ ID NO: 810 |
| hTfpi-Cj-3 | E02 | CAGAGAUGAUUUACAC AAUGAA | SEQ ID NO: 811 |
| hTfpi-Cj -4 | E02 | UGAUUUACACAAUGAA GAAAGU | SEQ ID NO: 812 |
| hTfpi-Cj-5 | E02 | CAGGCAUACAGAAGCC CAAAGU | SEQ ID NO: 813 |
| hTfpi-Cj -6 | E02 | GGCAGGGGCAAGAUUA AGCAGC | SEQ ID NO: 814 |
| hTfpi-Cj-7 | E02 | AAUGCUGAUUCUGAGG AAGAUG | SEQ ID NO: 815 |
| hTfpi-Cj -8 | E02 | UGCUGAUUCUGAGGAA GAUGAA | SEQ ID NO: 816 |
| hTfpi-Cj -9 | E03 | UACAUGGGCCAUCAUC CGCCUU | SEQ ID NO: 817 |
| hTfpi-Cj-10 | E03 | UCACAUCCCCCAUAUA UAAAUU | SEQ ID NO: 818 |
| hTfpi-Cj-11 | E03 | AAGUCUGGAAGAGUGC AAAAAA | SEQ ID NO: 819 |
| hTfpi-Cj-12 | E03 | AACCUACCUCUUGUAC ACAUUU | SEQ ID NO: 820 |
| hTfpi-Cj-13 | E03 | AGGGUUCCCAGAAACC UACCUC | SEQ ID NO: 821 |
| hTfpi-Cj-14 | E05 | CACUGUUUGUCUGAU UGUUAU | SEQ ID NO: 822 |
| hTfpi-Cj-15 | E05 | AGGCAUCCACCAUACU UGAAAC | SEQ ID NO: 823 |
| hTfpi-Cj-16 | E05 | UUGUUCAUAUUGCCCA | SEQ ID NO: 824 |
| | | GGCAUC | |
| hTfpi-Cj-17 | E05 | GCCUGGGCAAUAUGAA CAAUUU | SEQ ID NO: 825 |
| hTfpi-Cj-18 | E07 | CACAAUCCUCUGUCUG CUGGAG | SEQ ID NO: 826 |
| hTfpi-Cj-19 | E07 | UAGUAGAAUCUGUUCU CAUUGG | SEQ ID NO: 827 |
| hTfpi-Cj-20 | E07 | AAUUGUAGUAGAAUCU GUUCUC | SEQ ID NO: 828 |
| hTfpi-Cj-21 | E07 | GUCAUUGGGAAAUGCC GCCCAU | SEQ ID NO: 829 |
| hTfpi-Cj-22 | E07 | UUGUUUUCAUUUCCCC CACAUC | SEQ ID NO: 830 |

### One aspect of the disclosure of the present specification relates to a composition for gene manipulation for artificially manipulating a blood coagulation inhibitory gene.

The composition for gene manipulation may be used in the generation of an artificially manipulated blood coagulation inhibitory gene. In addition, the blood coagulation inhibitory gene artificially manipulated by the composition for gene manipulation may regulate a blood coagulation system.

The "artificially manipulated (artificially modified or engineered or artificially engineered)" refers to an artificially modified state, rather than as it exists in a naturally-occurring state. Hereinafter, an unnatural artificially manipulated or modified blood coagulation inhibitory gene may be used interchangeably with an artificial blood coagulation inhibitory gene.

Gene manipulation may be done in consideration of a gene expression regulation process. In an embodiment, the gene manipulation may be achieved by selecting a manipulation tool suitable for each of the steps of transcriptional regulation, RNA processing regulation, RNA transport regulation, RNA cleavage regulation, translational regulation, or protein modification regulation.

For example, the expression of genetic information may be controlled using RNAi (RNA interference or RNA silencing) to allow small RNA (sRNA) to interfere with mRNA or reduce the stability of mRNA, and optionally break down the mRNA, thereby making it prevent from informing on protein synthesis.

In an embodiment, gene manipulation may be performed using a wild-type or variant enzyme that can catalyze the hydrolysis (cleavage) of bonds between nucleotides in DNA or RNA molecules, preferably DNA molecules. A guide nucleic acid-editor protein complex may be used.

For example, the expression of genetic information may be controlled by manipulating a gene using one or more nucleases selected from the group consisting of meganucleases, Zinc finger nucleases, CRISPR/Cas9 proteins, CRISPR-Cpfl proteins, and TALE-nucleases.

The composition for gene manipulation disclosed in the present specification may include a guide nucleic acid and an editor protein.

In one embodiment, the composition for gene manipulation may include
(a) a guide nucleic acid capable of targeting a target sequence of a blood coagulation inhibitory gene or a nucleic acid sequence encoding the same; and
(b) one or more editor proteins or a nucleic acid sequence encoding the same.

The description related to the blood coagulation inhibitory gene is the same as described above.

The description related to the target sequence is the same as described above.

In another embodiment, the composition for gene manipulation may include
(a) a guide nucleic acid including a guide sequence capable of forming a complementary bond with a target sequence of a blood coagulation inhibitory gene or a nucleic acid sequence encoding the same; and
(b) one or more editor proteins or a nucleic acid sequence encoding the same.

The description related to the blood coagulation inhibitory gene is the same as described above.

The description related to the target sequence is the same as described above.

The description related to the guide sequence is the same as described above.

The composition for gene manipulation may include a guide nucleic acid-editor protein complex.

The term "guide nucleic acid-editor protein complex" refers to a complex formed through the interaction between a guide nucleic acid and an editor protein.

A description related to the guide nucleic acid is as described above.

The term "editor protein" refers to a peptide, polypeptide or protein which is able to directly bind to or interact with, without direct binding to, a nucleic acid.

Here, the nucleic acid may be a nucleic acid included in a target nucleic acid, gene or chromosome.

Here, the nucleic acid may be a guide nucleic acid.

The editor protein may be an enzyme.

Here, the term "enzyme" refers to a polypeptide or protein that contains a domain capable of cleaving a nucleic acid, gene or chromosome.

The enzyme may be a nuclease or restriction enzyme.

The editor protein may include a complete active enzyme.

Here, the "complete active enzyme" refers to an enzyme having the same function as the nucleic acid, gene or chromosome cleavage function of a wild-type enzyme. For example, the wild-type enzyme that cleaves double-stranded DNA may be a complete active enzyme that entirely cleaves double-stranded DNA. As another example, when the wild-type enzyme cleaving double-stranded DNA undergoes a deletion or substitution of a partial sequence of an amino acids sequence due to artificial engineering, the artificially engineered enzyme variant cleaves double-stranded DNA like the wild-type enzyme, the artificially engineered enzyme variant may be a complete active enzyme.

In addition, the complete active enzyme may include an enzyme having an improved function, compared to the wild-type enzyme. For example, a specific modified or manipulated form of the wild-type enzyme cleaving double-stranded DNA may have a complete enzyme activity, which is greater than the wild-type enzyme, that is, an increased activity of cleaving double-stranded DNA.

The editor protein may include an incomplete or partially active enzyme.

Here, the "incomplete or partially active enzyme" refers to an enzyme having some of the nucleic acid, gene or chromosome cleavage function of the wild-type enzyme. For example, a specific modified or manipulated form of the wild-type enzyme that cleaves double-stranded DNA may be a form having a first function or a form having a second function. Here, the first function is a function of cleaving the first strand of double-stranded DNA, and the second function may be a function of cleaving the second strand of double-stranded DNA. Here, the enzyme with the first function or the enzyme with the second function may be an incomplete or partially active enzyme.

The editor protein may include an inactive enzyme.

Here, the "inactive enzyme" refers to an enzyme in which the nucleic acid, gene or chromosome cleavage function of the wild-type enzyme is entirely inactivated. For example, a specific modified or manipulated form of the wild-type enzyme may be a form in which both of the first and second functions are lost, that is, both of the first function of cleaving the first strand of double-stranded DNA and the second function of cleaving the second strand thereof are lost. Here, the enzyme in which all of the first and second functions are lost may be inactive enzyme.

The editor protein may be a fusion protein.

Here, the term "fusion protein" refers to a protein produced by fusing an enzyme with an additional domain, peptide, polypeptide or protein.

The additional domain, peptide, polypeptide or protein may have a same or different function as a functional domain, peptide, polypeptide, or protein included in the enzyme.

The fusion protein may be a form in which the functional domain, peptide, polypeptide or protein is added to one or more of the amino end of an enzyme or the proximity thereof; the carboxyl end of the enzyme or the proximity thereof; the middle part of the enzyme; or a combination thereof.

Here, the functional domain, peptide, polypeptide or protein may be a domain, peptide, polypeptide or protein having methylase activity, demethylase activity, transcription activation activity, transcription repression activity, transcription release factor activity, histone modification activity, RNA cleavage activity or nucleic acid binding activity, or a tag or reporter gene for isolation and purification of a protein (including a peptide), but the present invention is not limited thereto.

The functional domain, peptide, polypeptide or protein may be a deaminase.

The tag includes a histidine (His) tag, a V5 tag, a FLAG tag, an influenza hemagglutinin (HA) tag, a Myc tag, a VSV-G tag and a thioredoxin (Trx) tag, and the reporter gene includes glutathione-S-transferase (GST), horseradish peroxidase (HRP), chloramphenicol acetyltransferase (CAT) β-galactosidase, β-glucoronidase, luciferase, auto fluorescent proteins including the green fluorescent protein (GFP), HcRed, DsRed, cyan fluorescent protein (CFP), yellow fluorescent protein (YFP) and blue fluorescent protein (BFP), but the present invention is not limited thereto.

In addition, the functional domain, peptide, polypeptide or protein may be a nuclear localization sequence or signal (NLS) or a nuclear export sequence or signal (NES).

The NLS may be NLS of SV40 virus large T-antigen with an amino acid sequence PKKKRKV (SEQ ID NO: 831); NLS derived from nucleoplasmin (e.g., nucleoplasmin bipartite NLS with a sequence KRPAATKKAGQAKKKK(SEQ ID NO: 832)); c-myc NLS with an amino acid sequence PAAKRVKLD(SEQ ID NO: 833) or RQRRNELKRSP(SEQ ID NO: 834); hRNPA1 M9 NLS with a sequence NQSSNFGPMKGGNFGGRSSGPYGGGGQYFAKPRNQGGY(SEQ ID NO: 835); an importin-α-derived IBB domain sequence RMRIZFKNKGKDTAELRRRRVEVSVELRKAKKDEQILKRRNV(SEQ ID NO: 836); myoma T protein sequences VSRKRPRP(SEQ ID NO: 837) and PPKKARED(SEQ ID NO: 838); human p53 sequence PQPKKKPL(SEQ ID NO: 839); a mouse c-abl IV sequence SALIKKKKKMAP(SEQ ID NO: 840); influenza virus NS1 sequences DRLRR(SEQ ID NO: 841) and PKQKKRK(SEQ ID NO: 842); a hepatitis virus-δ antigen sequence RKLKKKIKKL(SEQ ID NO: 843); a mouse Mx1 protein sequence REKKKFLKRR(SEQ ID NO: 844); a human poly(ADP-ribose) polymerase sequence KRKGDEVDGVDEVAKKKSKK(SEQ ID NO: 845); or steroid hormone receptor (human) glucocorticoid sequence RKCLQAGMNLEARKTKK(SEQ ID NO: 846), but the present invention is not limited thereto.

The additional domain, peptide, polypeptide or protein may be a non-functional domain, peptide, polypeptide or protein that does not perform a specific function. Here, the non-functional domain, peptide, polypeptide or protein may be a domain, peptide, polypeptide or protein that does not affect the enzyme function.

The fusion protein may be a form in which the non-functional domain, peptide, polypeptide or protein is added to one or more of the amino end of an enzyme or the proximity thereof; the carboxyl end of the enzyme or the proximity thereof; the middle part of the enzyme; or a combination thereof.

The editor protein may be a wild-type of enzyme or fusion protein that exists in nature.

The editor protein may be a form of a partially modified enzyme or fusion protein.

The editor protein may be an artificially produced enzyme or fusion protein, which does not exist in nature.

The editor protein may be a form of a partially modified artificial enzyme or fusion protein, which does not exist in nature.

Here, the modification may be substitution, removal, addition of amino acids contained in the editor protein, or a combination thereof.

Alternatively, the modification may be substitution, removal, addition of some nucleotides in the nucleotide sequence encoding the editor protein, or a combination thereof.

In addition, optionally, the composition for gene manipulation may further include a donor having a desired specific nucleotide sequence, which is to be inserted, or a nucleic acid sequence encoding the same.

Here, the nucleic acid sequence to be inserted may be a partial nucleotide sequence of the blood coagulation inhibitory gene.

Here, the nucleic acid sequence to be inserted may be a nucleic acid sequence for being introduced into the blood coagulation inhibitory gene to be manipulated and used to correct a mutation of the blood coagulation inhibitory gene.

The term "donor" refers to a nucleotide sequence that helps homologous recombination (HR)-based repair of a damaged gene or nucleic acid.

The donor may be a double- or single-stranded nucleic acid.

The donor may be present in a linear or circular shape.

The donor may include a nucleotide sequence having homology with a target gene or a nucleic acid.

For example, the donor may include a nucleotide sequence having homology with each of nucleotide sequences of upstream (left) and downstream (right) of a location where a specific nucleotide sequence is inserted, in which a damaged nucleic acid exists. Here, the specific nucleotide sequence to be inserted may be located between a nucleotide sequence having homology with a left nucleotide sequence of the damaged nucleic acid and a nucleotide sequence having homology with a right nucleotide sequence of the damaged nucleic acid. Here, the nucleotide sequence having homology may have at least 50%, 55%, 60%, 65%, 70%, 75%, 80%, 85%, 90% or 95% or more homology or complete homology.

The donor may selectively include an additional nucleotide sequence. Here, the additional nucleic acid sequence may play a role in increasing stability, insertion efficiency into a target or homologous recombination efficiency of the donor.

For example, the additional nucleotide sequence may be a nucleic acid sequence rich in nucleotides A and T, that is, an A-T rich domain. For example, the additional nucleotide sequence may be a scaffold/matrix attachment region (SMAR).

The guide nucleic acid, editor protein or guide nucleic acid-editor protein complex disclosed in the specification may be delivered or introduced into a subject in various ways.

Here, the term "subject" refers to an organism into which a guide nucleic acid, editor protein or guide nucleic acid-editor protein complex is introduced, an organism in which a guide nucleic acid, editor protein or guide nucleic acid-editor protein complex operates, or a specimen or sample obtained from the organism.

The subject may be an organism including a target gene or chromosome of a guide nucleic acid-editor protein complex.

The organism may be an animal, animal tissue or an animal cell.

The organism may be a human, human tissue or a human cell.

The tissue may be eyeball, skin, liver, kidney, heart, lung, brain, muscle tissue, or blood.

The cells may be a liver cell, an immune cell, a blood cell, or a stem cell.

The specimen or sample may be acquired from an organism including a target gene or chromosome such as saliva, blood, liver tissue, brain tissue, a liver cell, a nerve cell, a phagocyte, a macrophage, a T cell, a B cell, an astrocyte, a cancer cell or a stem cell, etc.

Preferably, the subject may be an organism including a blood coagulation inhibitory gene.

The guide nucleic acid, editor protein or guide nucleic acid-editor protein complex may be delivered or introduced into a subject in the form of DNA, RNA or a mixed form.

Here, the form of DNA, RNA or a mixture thereof, which encodes the guide nucleic acid and/or editor protein may be delivered or introduced into a subject by a method known in the art.

Or, the form of DNA, RNA or a mixture thereof, which encodes the guide nucleic acid and/or editor protein may be delivered or introduced into a subject by a vector, a non-vector or a combination thereof.

The vector may be a viral or non-viral vector (e.g., a plasmid).

The non-vector may be naked DNA, a DNA complex or mRNA.

In one embodiment, the nucleic acid sequence encoding the guide nucleic acid and/or editor protein may be delivered or introduced into a subject by a vector.

The vector may include a nucleic acid sequence encoding a guide nucleic acid and/or editor protein.

In one example, the vector may simultaneously include nucleic acid sequences, which encode the guide nucleic acid and the editor protein.

In another example, the vector may include the nucleic acid sequence encoding the guide nucleic acid.

As an example, domains included in the guide nucleic acid may be contained all in one vector, or may be divided and then contained in different vectors.

In another example, the vector may include the nucleic acid sequence encoding the editor protein.

As an example, in the case of the editor protein, the nucleic acid sequence encoding the editor protein may be contained in one vector, or may be divided and then contained in several vectors.

The vector may include one or more regulatory/control components.

Here, the regulatory/control components may include a promoter, an enhancer, an intron, a polyadenylation signal, a Kozak consensus sequence, an internal ribosome entry site (IRES), a splice acceptor and/or a 2A sequence.

The promoter may be a promoter recognized by RNA polymerase II.

The promoter may be a promoter recognized by RNA polymerase III.

The promoter may be an inducible promoter.

The promoter may be a subject-specific promoter.

The promoter may be a viral or non-viral promoter.

The promoter may use a suitable promoter according to a control region (that is, a nucleic acid sequence encoding a guide nucleic acid or editor protein).

For example, a promoter useful for the guide nucleic acid may be a H1, EF-1a, tRNA or U6 promoter. For example, a promoter useful for the editor protein may be a CMV, EF-la, EFS, MSCV, PGK or CAG promoter.

The vector may be a viral vector or recombinant viral vector.

The virus may be a DNA virus or an RNA virus.

Here, the DNA virus may be a double-stranded DNA (dsDNA) virus or single-stranded DNA (ssDNA) virus.

Here, the RNA virus may be a single-stranded RNA (ssRNA) virus.

The virus may be a retrovirus, a lentivirus, an adenovirus, adeno-associated virus (AAV), vaccinia virus, a poxvirus or a herpes simplex virus, but the present invention is not limited thereto.

Generally, the virus may infect a host (e.g., cells), thereby introducing a nucleic acid encoding the genetic information of the virus into the host or inserting a nucleic acid encoding the genetic information into the host genome. The guide nucleic acid and/or editor protein may be introduced into a subject using a virus having such a characteristic. The guide nucleic acid and/or editor protein introduced using the virus may be temporarily expressed in the subject (e.g., cells). Alternatively, the guide nucleic acid and/or editor protein introduced using the virus may be continuously expressed in a subject (e.g., cells) for a long time (e.g., 1, 2, 3 weeks, 1, 2, 3, 6, 9 months, 1, 2 years, or permanently).

The packaging capability of the virus may vary from at least 2 kb to 50 kb according to the type of virus. Depending on such a packaging capability, a viral vector including a guide nucleic acid or an editor protein or a viral vector including both of a guide nucleic acid and an editor protein may be designed. Alternatively, a viral vector including a guide nucleic acid, an editor protein and additional components may be designed.

In one example, a nucleic acid sequence encoding a guide nucleic acid and/or editor protein may be delivered or introduced by a recombinant lentivirus.

In another example, a nucleic acid sequence encoding a guide nucleic acid and/or editor protein may be delivered or introduced by a recombinant adenovirus.

In still another example, a nucleic acid sequence encoding a guide nucleic acid and/or editor protein may be delivered or introduced by recombinant AAV.

In yet another example, a nucleic acid sequence encoding a guide nucleic acid and/or editor protein may be delivered or introduced by a hybrid virus, for example, one or more hybrids of the virus listed herein.

In another embodiment, the nucleic acid sequence encoding the guide nucleic acid and/or editor protein may be delivered or introduced into a subject by a non-vector.

The non-vector may include a nucleic acid sequence encoding a guide nucleic acid and/or editor protein.

The non-vector may be naked DNA, a DNA complex, mRNA, or a mixture thereof.

The non-vector may be delivered or introduced into a subject by electroporation, gene gun, sonoporation, magnetofection, transient cell compression or squeezing (e.g., described in the literature [Lee, et al, (2012) Nano Lett., 12, 6322-6327]), lipid-mediated transfection, a dendrimer, nanoparticles, calcium phosphate, silica, a silicate (Ormosil), or a combination thereof.

In one example, the delivery through electroporation may be performed by mixing cells and a nucleic acid sequence encoding a guide nucleic acid and/or editor protein in a cartridge, chamber or cuvette, and applying electrical stimuli with a predetermined duration and amplitude to the cells.

In another example, the non-vector may be delivered using nanoparticles. The nanoparticles may be inorganic nanoparticles (e.g., magnetic nanoparticles, silica, etc.) or organic nanoparticles (e.g., a polyethylene glycol (PEG)-coated lipid, etc.). The outer surface of the nanoparticles may be conjugated with a positively-charged polymer which is attachable (e.g., polyethyleneimine, polylysine, polyserine, etc.).

In a certain embodiment, the non-vector may be delivered using a lipid shell.

In a certain embodiment, the non-vector may be delivered using an exosome. The exosome is an endogenous nano-vesicle for transferring a protein and RNA, which can deliver RNA to the brain and another target organ.

In a certain embodiment, the non-vector may be delivered using a liposome. The liposome is a spherical vesicle structure which is composed of single or multiple lamellar lipid bilayers surrounding internal aqueous compartments and an external, lipophilic phospholipid bilayer which is relatively non-transparent. While the liposome may be made from several different types of lipids; phospholipids are most generally used to produce the liposome as a drug carrier.

In addition, the composition for delivery of the non-vector may include other additives.

The editor protein may be delivered or introduced into a subject in the form of a peptide, polypeptide or protein.

The editor protein may be delivered or introduced into a subject in the form of a peptide, polypeptide or protein by a method known in the art.

The peptide, polypeptide or protein form may be delivered or introduced into a subject by electroporation, microinjection, transient cell compression or squeezing (e.g., described in the literature [Lee, et al, (2012) Nano Lett., 12, 6322-6327]), lipid-mediated transfection, nanoparticles, a liposome, peptide-mediated delivery or a combination thereof.

The peptide, polypeptide or protein may be delivered with a nucleic acid sequence encoding a guide nucleic acid.

In one example, the transfer through electroporation may be performed by mixing cells into which the editor protein will be introduced with or without a guide nucleic acid in a cartridge, chamber or cuvette, and applying electrical stimuli with a predetermined duration and amplitude to the cells.

The guide nucleic acid and the editor protein may be delivered or introduced into a subject in the form of mixing a nucleic acid and a protein.

The guide nucleic acid and the editor protein may be delivered or introduced into a subject in the form of a guide nucleic acid-editor protein complex.

For example, the guide nucleic acid may be a DNA, RNA or a mixture thereof. The editor protein may be a peptide, polypeptide or protein.

In one example, the guide nucleic acid and the editor protein may be delivered or introduced into a subject in the form of a guide nucleic acid-editor protein complex containing an RNA-type guide nucleic acid and a protein-type editor protein, that is, a ribonucleoprotein (RNP).

The guide nucleic acid-editor protein complex disclosed in the specification may modify a target nucleic acid, gene or chromosome.

For example, the guide nucleic acid-editor protein complex induces a modification in the sequence of a target nucleic acid, gene or chromosome. As a result, a protein expressed by the target nucleic acid, gene or chromosome may be modified in structure and/or function, or the expression of the protein may be controlled or inhibited.

The guide nucleic acid-editor protein complex may act at the DNA, RNA, gene or chromosome level.

In one example, the guide nucleic acid-editor protein complex may manipulate or modify the target gene to control (e.g., suppress, inhibit, reduce, increase or promote) the expression of a protein encoded by a target gene, or express a protein whose activity is controlled (e.g., suppressed, inhibited, reduced, increased or promoted) or modified.

The guide nucleic acid-editor protein complex may act at the transcription and translation stage of a gene.

In one example, the guide nucleic acid-editor protein complex may promote or inhibit the transcription of a target gene, thereby controlling (e.g., suppressing, inhibiting, reducing, increasing or promoting) the expression of a protein encoded by the target gene.

In another example, the guide nucleic acid-editor protein complex may promote or inhibit the translation of a target gene, thereby controlling (e.g., suppressing, inhibiting, reducing, increasing or promoting) the expression of a protein encoded by the target gene.

### In one embodiment of the disclosure of the present specification, the composition for gene manipulation may include gRNA and a CRISPR enzyme.

In one embodiment, the composition for gene manipulation may include
(a) a gRNA capable of targeting a target sequence of a blood coagulation inhibitory gene or a nucleic acid sequence encoding the same; and
(b) one or more CRISPR enzymes or a nucleic acid sequence encoding the same.

The description related to the blood coagulation inhibitory gene is the same as described above.

The description related to the target sequence is the same as described above.

In another embodiment, the composition for gene manipulation may include
(a) a gRNA including a guide sequence capable of forming a complementary bond with a target sequence of a blood coagulation inhibitory gene or a nucleic acid sequence encoding the same; and
(b) one or more CRISPR enzymes or a nucleic acid sequence encoding the same.

The description related to the blood coagulation inhibitory gene is the same as described above.

The description related to the target sequence is the same as described above.

The description related to the guide sequence is the same as described above.

The composition for gene manipulation may include a gRNA-CRISPR enzyme complex. The term "gRNA-CRISPR enzyme complex" refers to a complex formed by an interaction between gRNA and a CRISPR enzyme.

A description related to the gRNA is as described above.

The term "CRISPR enzyme" is a main protein component of a CRISPR-Cas system, and forms a complex with gRNA, resulting in the CRISPR-Cas system.

The CRISPR enzyme may be a nucleic acid having a sequence encoding the CRISPR enzyme or a polypeptide (or a protein).

The CRISPR enzyme may be a Type II CRISPR enzyme.

The crystal structure of the type II CRISPR enzyme was determined according to studies on two or more types of natural microbial type II CRISPR enzyme molecules (Jinek et al., Science, 343(6176): 1247997, 2014) and studies on *Streptococcus pyogenes* Cas9 (SpCas9) complexed with gRNA (Nishimasu et al., Cell, 156:935-949, 2014; and Anders et al., Nature, 2014, doi: 10.1038/nature13579).

The type II CRISPR enzyme includes two lobes, that is, recognition (REC) and nuclease (NUC) lobes, and each lobe includes several domains.

The REC lobe includes an arginine-rich bridge helix (BH) domain, an REC1 domain and an REC2 domain.

Here, the BH domain is a long α-helix and arginine-rich region, and the REC1 and REC2 domains play an important role in recognizing a double strand formed in gRNA, for example, single-stranded gRNA, double-stranded gRNA or tracrRNA.

The NUC lobe includes a RuvC domain, an HNH domain and a PAM-interaction (PI) domain. Here, the RuvC domain encompasses RuvC-like domains, and the HNH domain encompasses HNH-like domains.

Here, the RuvC domain shares structural similarity with members of the microorganism family existing in nature including the type II CRISPR enzyme, and cleaves a single strand, for example, a non-complementary strand of a target gene or a nucleic acid, that is, a strand not forming a complementary bond with gRNA. The RuvC domain is sometimes referred to as a RuvCI domain, RuvCII domain or RuvCIII domain in the art, and generally called an RuvC I, RuvCII or RuvCIII.

The HNH domain shares structural similarity with the HNH endonuclease, and cleaves a single strand, for example, a complementary strand of a target nucleic acid molecule, that is, a strand forming a complementary bond with gRNA. The HNH domain is located between RuvC II and III motifs.

The PI domain recognizes a specific nucleotide sequence in a target gene or a nucleic acid, that is, a protospacer adjacent motif (PAM), or interacts with PAM Here, the PAM may vary according to the origin of a Type II CRISPR enzyme. For example, when the CRISPR enzyme is SpCas9, the PAM may be 5'-NGG-3', and when the CRISPR enzyme is Streptococcus thermophilus Cas9 (StCas9), the PAM may be 5'-NNAGAAW-3' (W = A or T), when the CRISPR enzyme is Neisseria meningiditis Cas9 (NmCas9), the PAM may be 5'-NNNNGATT-3', and when the CRISPR enzyme is Campylobacter jejuni Cas9 (CjCas9), the PAM may be 5'-NNNVRYAC-3' (V = G or C or A, R = A or G, Y = C or T), herein, N is A, T, G or C; or A, U, G or C). However, while it is generally understood that PAM is determined according to the origin of the above-described enzyme, as the study of a mutant of an enzyme derived from the corresponding origin progresses, the PAM may be changed.

The Type II CRISPR enzyme may be Cas9.

The Cas9 may be derived from various microorganisms such as *Streptococcus pyogenes, Streptococcus thermophilus, Streptococcus* sp., *Staphylococcus aureus, Campylobacter jejuni, Nocardiopsis dassonvillei, Streptomyces pristinaespiralis, Streptomyces viridochromogenes, Streptomyces viridochromogenes, Streptosporangium roseum, Streptosporangium roseum, AlicyclobacHlus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas* sp., *Crocosphaera watsonii, Cyanothece* sp., *Microcystis aeruginosa, Synechococcus* sp., *Acetohalobium arabaticum, Ammonifex degensii, Caldicelulosiruptor bescii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter* sp., *Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc* sp., *Arthrospira maxima, Arthrospira platensis, Arthrospira* sp., *Lyngbya* sp., *Microcoleus chthonoplastes, Oscillatoria* sp., *Petrotoga mobilis, Thermosipho africanus* and *Acaryochloris marina.*

The Cas9 is an enzyme which binds to gRNA so as to cleave or modify a target sequence or position of a target gene or a nucleic acid, and may consist of an HNH domain capable of cleaving a nucleic acid strand forming a complementary bond with gRNA, an RuvC domain capable of cleaving a nucleic acid strand forming a non-complementary bond with gRNA, an REC domain interacting with the target and a PI domain recognizing a PAM Hiroshi Nishimasu et al. (2014) Cell 156:935-949 may be referenced for specific structural characteristics of Cas9.

The Cas9 may be isolated from a microorganism existing in nature or non-naturally produced by a recombinant or synthetic method.

In addition, the CRISPR enzyme may be a Type V CRISPR enzyme.

The type V CRISPR enzyme includes similar RuvC domains corresponding to the RuvC domains of the type II CRISPR enzyme, and the HNH domain of the Type II CRISPR enzyme is deficient. But it instead includes an Nuc domain, and may consist of REC and WED domains interacting with a target, and a PI domain recognizing PAM. For specific structural characteristics of the type V CRISPR enzyme, Takashi Yamano et al. (2016) Cell 165:949-962 may be referenced.

The type V CRISPR enzyme may interact with gRNA, thereby forming a gRNA-CRISPR enzyme complex, that is, a CRISPR complex, and may allow a guide sequence to approach a target sequence including a PAM sequence in cooperation with gRNA. Here, the ability of the type V CRISPR enzyme for interaction with a target gene or a nucleic acid is dependent on the PAM sequence.

The PAM sequence may be a sequence present in a target gene or a nucleic acid, and recognized by the PI domain of a Type V CRISPR enzyme. The PAM sequence may have different sequences according to the origin of the Type V CRISPR enzyme. That is, each species has a specifically recognizable PAM sequence. For example, the PAM sequence recognized by Cpfl may be 5'-TTN-3' (N is A, T, C or G). While it has been generally understood that PAM is determined according to the origin of the above-described enzyme, as the study of mutants of the enzyme derived from the corresponding origin progresses, the PAM may be changed.

The Type V CRISPR enzyme may be Cpfl.

The Cpfl may be derived from *Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacillus, Methylobacterium* or *Acidaminococcus.*

The Cpfl includes a RuvC-like domain corresponding to the RuvC domains of Cas9, and the HNH domain of the Cas9 is deficient. But it instead includes an Nuc domain, and may consist of REC and WED domains interacting with a target, and a PI domain recognizing PAM. For specific structural characteristics of Cpfl, Takashi Yamano et al. (2016) Cell 165:949-962 may be referenced.

The Cpfl may be isolated from a microorganism existing in nature or non-naturally produced by a recombinant or synthetic method.

The CRISPR enzyme may be a nuclease or restriction enzyme having a function of cleaving a double-stranded nucleic acid of a target gene or a nucleic acid.

The CRISPR enzyme may be a complete active CRISPR enzyme.

The term "complete active" refers to a state in which an enzyme has the same function as that of a wild-type CRISPR enzyme, and the CRISPR enzyme in such a state is named a complete active CRISPR enzyme. Here, the "function of the wild-type CRISPR enzyme" refers to a state in which an enzyme has functions of cleaving double-stranded DNA, that is, the first function of cleaving the first strand of double-stranded DNA and a second function of cleaving the second strand of double-stranded DNA.

The complete active CRISPR enzyme may be a wild-type CRISPR enzyme that cleaves double-stranded DNA.

The complete active CRISPR enzyme may be a CRISPR enzyme variant formed by modifying or manipulating the wild-type CRISPR enzyme that cleaves double-stranded DNA.

The CRISPR enzyme variant may be an enzyme in which one or more amino acids of the amino acid sequence of the wild-type CRISPR enzyme are substituted with other amino acids, or one or more amino acids are removed.

The CRISPR enzyme variant may be an enzyme in which one or more amino acids are added to the amino acid sequence of the wild-type CRISPR enzyme. Here, the location of the added amino acids may be the N-end, the C-end or in the amino acid sequence of the wild-type enzyme.

The CRISPR enzyme variant may be a complete active enzyme with an improved function compared to the wild-type CRISPR enzyme.

For example, a specifically modified or manipulated form of the wild-type CRISPR enzyme, that is, the CRISPR enzyme variant may cleave double-stranded DNA while not binding to the double-stranded DNA to be cleaved or maintaining a certain distance therefrom. In this case, the modified or manipulated form may be a complete active CRISPR enzyme with an improved functional activity, compared to the wild-type CRISPR enzyme.

The CRISPR enzyme variant may be a complete active CRISPR enzyme with a reduced function, compared to the wild-type CRISPR enzyme.

For example, the specific modified or manipulated form of the wild-type CRISPR enzyme, that is, the CRISPR enzyme variant may cleave double-stranded DNA while very close to the double-stranded DNA to be cleaved or forming a specific bond therewith. Here, the specific bond may be, for example, a bond between an amino acid at a specific region of the CRISPR enzyme and a DNA nucleotide sequence at the cleavage location. In this case, the modified or manipulated form may be a complete active CRISPR enzyme with a reduced functional activity, compared to the wild-type CRISPR enzyme.

The CRISPR enzyme may be an incomplete or partially active CRISPR enzyme.

The term "incomplete or partially active" refers to a state in which an enzyme has one selected from the functions of the wild-type CRISPR enzyme, that is, a first function of cleaving the first strand of double-stranded DNA and a second function of cleaving the second strand of double-stranded DNA. The CRISPR enzyme in this state is named an incomplete or partially active CRISPR enzyme. In addition, the incomplete or partially active CRISPR enzyme may be referred to as a nickase.

The term "nickase" refers to a CRISPR enzyme manipulated or modified to cleave only one strand of the double strand of a target gene or a nucleic acid, and the nickase has nuclease activity of cleaving a single strand, for example, a strand that is complementary or non-complementary to gRNA of a target gene or a nucleic acid. Therefore, to cleave the double strand, nuclease activity of the two nickases is needed.

The nickase may have nuclease activity by the RuvC domain of a CRISPR enzyme. That is, the nickase may not include nuclease activity of the HNH domain of a CRISPR enzyme, and to this end, the HNH domain may be manipulated or modified.

In one example, when the CRISPR enzyme is a Type II CRISPR enzyme, the nickase may be a Type II CRISPR enzyme including a modified HNH domain.

For example, provided that the Type II CRISPR enzyme is a wild-type SpCas9, the nickase may be a SpCas9 variant in which nuclease activity of the HNH domain is inactived by mutation that the 840^{th} amino acid in the amino acid sequence of the wild-type SpCas9 is mutated from histidine to alanine. Since the nickase produced thereby, that is, the SpCas9 variant has nuclease activity of the RuvC domain, it is able to cleave a strand which is a non-complementary strand of a target gene or a nucleic acid, that is, a strand not forming a complementary bond with gRNA.

For another example, provided that the Type II CRISPR enzyme is a wild-type CjCas9, the nickase may be a CjCas9 variant in which nuclease activity of the HNH domain is inactived by mutation that the 559^{th} amino acid in the amino acid sequence of the wild-type CjCas9 is mutated from histidine to alanine. Since the nickase produced thereby, that is, the CjCas9 variant has nuclease activity of the RuvC domain, it is able to cleave a strand which is a non-complementary strand of a taget gene or a nucleic acid, that is, a strand not forming a complementary bond with gRNA.

In addition, the nickase may have nuclease activity by the HNH domain of a CRISPR enzyme. That is, the nickase may not include the nuclease activity of the RuvC domain, and to this end, the RuvC domain may be manipulated or modified.

In one example, when the CRISPR enzyme is a Type II CRISPR enzyme, the nickase may be a Type II CRISPR enzyme including a modified RuvC domain.

For example, provided that the Type II CRISPR enzyme is a wild-type SpCas9, the nickase may be a SpCas9 variant in which nuclease activity of the RuvC domain is inactived by mutation that the 10^{th} amino acid in the amino acid sequence of the wild-type SpCas9 is mutated from aspartic acid to alanine. Since the nickase produced thereby, that is the SpCas9 variant has nuclease activity of the HNH domain, it is able to cleave a strand which is a complementary strand of a target gene or a nucleic acid, that is, a strand forming a complementary bond with gRNA.

For another example, provided that the Type II CRISPR enzyme is a wild-type CjCas9, the nickase may be a CjCas9 variant in which nuclease activity of the RuvC domain is inactived by mutation that the 8^{th} amino acid in the amino acid sequence of the wild-type CjCas9 is mutated from aspartic acid to alanine. Since the nickase produced thereby, that is, the CjCas9 variant has nuclease activity of the HNH domain, it is able to cleave a strand which is a complementary strand of a target gene or a nucleic acid, that is, a strand forming a complementary bond with gRNA.

The CRISPR enzyme may be an inactive CRISPR enzyme.

The term "inactive" refers to a state in which both of the functions of the wild-type CRISPR enzyme, that is, the first function of cleaving the first strand of double-stranded DNA and the second function of cleaving the second strand of double-stranded DNA are lost. The CRISPR enzyme in such a state is named an inactive CRISPR enzyme.

The inactive CRISPR enzyme may have nuclease inactivity due to variations in the domain having nuclease activity of a wild-type CRISPR enzyme.

The inactive CRISPR enzyme may have nuclease inactivity due to variations in a RuvC domain and an HNH domain. That is, the inactive CRISPR enzyme may not have nuclease activity generated by the RuvC domain and HNH domain of the CRISPR enzyme, and to this end, the RuvC domain and the HNH domain may be manipulated or modified.

In one example, when the CRISPR enzyme is a Type II CRISPR enzyme, the inactive CRISPR enzyme may be a Type II CRISPR enzyme having a modified RuvC domain and HNH domain.

For example, when the Type II CRISPR enzyme is a wild-type SpCas9, the inactive CRISPR enzyme may be a SpCas9 variant in which the nuclease activities of the RuvC domain and the HNH domain are inactivated by mutations of both aspartic acid 10 and histidine 840 in the amino acid sequence of the wild-type SpCas9 to alanine. Here, since, in the produced inactive CRISPR enzyme, that is, the SpCas9 variant, the nuclease activities of the RuvC domain and the HNH domain are inactivated, double-strand of a target gene or a nucleic acid may not be entirely cleaved.

In another example, when the Type II CRISPR enzyme is a wild-type CjCas9, the inactive CRISPR enzyme may be a CjCas9 variant in which the nuclease activities of the RuvC domain and the HNH domain are inactivated by mutations of both aspartic acid 8 and histidine 559 in the amino acid sequence of the wild-type CjCas9 to alanine. Here, since, in the produced inactive CRISPR enzyme, that is, the CjCas9 variant, the nuclease activities of the RuvC domain and HNH domain are inactivated, double-strand of a target gene or a nucleic acid may not be entirely cleaved.

The CRISPR enzyme may have helicase activity, that is, an ability to anneal the helix structure of the double-stranded nucleic acid, in addition to the above-described nuclease activity.

In addition, the CRISPR enzyme may be modified to complete activate, incomplete or partially activate, or inactivate the helicase activity.

The CRISPR enzyme may be a CRISPR enzyme variant produced by artificially manipulating or modifying the wild-type CRISPR enzyme.

The CRISPR enzyme variant may be an artificially manipulated or modified CRISPR enzyme variant for modifying the functions of the wild-type CRISPR enzyme, that is, the first function of cleaving the first strand of double-stranded DNA and/or the second function of cleaving the second strand of double-stranded DNA.

For example, the CRISPR enzyme variant may be a form in which the first function of the functions of the wild-type CRISPR enzyme is lost.

Alternatively, the CRISPR enzyme variant may be a form in which the second function of the functions of the wild-type CRISPR enzyme is lost.

For example, the CRISPR enzyme variant may be a form in which both of the functions of the wild-type CRISPR enzyme, that is, the first function and the second function, are lost.

The CRISPR enzyme variant may form a gRNA-CRISPR enzyme complex by interactions with gRNA.

The CRISPR enzyme variant may be an artificially manipulated or modified CRISPR enzyme variant for modifying a function of interacting with gRNA of the wild-type CRISPR enzyme.

For example, the CRISPR enzyme variant may be a form having reduced interactions with gRNA, compared to the wild-type CRISPR enzyme.

Alternatively, the CRISPR enzyme variant may be a form having increased interactions with gRNA, compared to the wild-type CRISPR enzyme.

For example, the CRISPR enzyme variant may be a form having the first function of the wild-type CRISPR enzyme and reduced interactions with gRNA.

Alternatively, the CRISPR enzyme variant may be a form having the first function of the wild-type CRISPR enzyme and increased interactions with gRNA.

For example, the CRISPR enzyme variant may be a form having the second function of the wild-type CRISPR enzyme and reduced interactions with gRNA.

Alternatively, the CRISPR enzyme variant may be a form having the second function of the wild-type CRISPR enzyme and increased interactions with gRNA.

For example, the CRISPR enzyme variant may be a form not having the first and second functions of the wild-type CRISPR enzyme, and having reduced interactions with gRNA.

Alternatively, the CRISPR enzyme variant may be a form not having the first and second functions of the wild-type CRISPR enzyme and having increased interactions with gRNA.

Here, according to the interaction strength between gRNA and the CRISPR enzyme variant, various gRNA-CRISPR enzyme complexes may be formed, and according to the CRISPR enzyme variant, there may be a difference in function of approaching or cleaving the target sequence.

For example, the gRNA-CRISPR enzyme complex formed by a CRISPR enzyme variant having reduced interactions with gRNA may cleave a double or single strand of a target sequence only when very close to or localized to the target sequence completely complementarily bind to gRNA.

The CRISPR enzyme variant may be in a form in which at least one amino acid of the amino acid sequence of the wild-type CRISPR enzyme is modified.

As an example, the CRISPR enzyme variant may be in a form in which at least one amino acid of the amino acid sequence of the wild-type CRISPR enzyme is substituted.

As another example, the CRISPR enzyme variant may be in a form in which at least one amino acid of the amino acid sequence of the wild-type CRISPR enzyme is deleted.

As still another example, the CRISPR enzyme variant may be in a form in which at least one amino acid of the amino acid sequence of the wild-type CRISPR enzyme is added.

In one example, the CRISPR enzyme variant may be in a form in which at least one amino acid of the amino acid sequence of the wild-type CRISPR enzyme is substituted, deleted and/or added.

In addition, optionally, the CRISPR enzyme variant may further include a functional domain, in addition to the original functions of the wild-type CRISPR enzyme, that is, the first function of cleaving the first strand of double-stranded DNA and the second function of cleaving the second strand thereof. Here, the CRISPR enzyme variant may have an additional function, in addition to the original functions of the wild-type CRISPR enzyme.

The functional domain may be a domain having methylase activity, demethylase activity, transcription activation activity, transcription repression activity, transcription release factor activity, histone modification activity, RNA cleavage activity or nucleic acid binding activity, or a tag or reporter gene for isolating and purifying a protein (including a peptide), but the present invention is not limited thereto.

The tag includes a histidine (His) tag, a V5 tag, a FLAG tag, an influenza hemagglutinin (HA) tag, a Myc tag, a VSV-G tag and a thioredoxin (Trx) tag, and the reporter gene includes glutathione-S-transferase (GST), horseradish peroxidase (HRP), chloramphenicol acetyltransferase (CAT) β-galactosidase, β-glucoronidase, luciferase, autofluorescent proteins including the green fluorescent protein (GFP), HcRed, DsRed, cyan fluorescent protein (CFP), yellow fluorescent protein (YFP) and blue fluorescent protein (BFP), but the present invention is not limited thereto.

The functional domain may be a deaminase.

For example, cytidine deaminase may be further included as a functional domain to an incomplete or partially-active CRISPR enzyme. In one exemplary embodiment, a fusion protein may be produced by adding a cytidine deaminase, for example, apolipoprotein B editing complex 1 (APOBEC1) to a SpCas9 nickase. The [SpCas9 nickase]-[APOBEC1] formed as described above may be used in nucleotide editing of C to T or U, or nucleotide editing of G to A.

In another example, adenine deaminase may be further included as a functional domain to the incomplete or partially-active CRISPR enzyme. In one exemplary embodiment, a fusion protein may be produced by adding adenine deaminases, for example, TadA variants, ADAR2 variants or ADAT2 variants to a SpCas9 nickase. The [SpCas9 nickase]-[TadA variant], [SpCas9 nickase]-[ADAR2 variant] or [SpCas9 nickase]-[ADAT2 variant] formed as described above may be used in nucleotide editing of A to G, or nucleotide editing of T to C, because the fusion protein modifies nucleotide A to inosine, the modified inosine is recognized as nucleotide G by a polymerase, thereby substantially exhibiting nucleotide editing of A to G.

The functional domain may be a nuclear localization sequence or signal (NLS) or a nuclear export sequence or signal (NES).

In one example, the CRISPR enzyme may include one or more NLSs. Here, the NLS may be included at an N-terminus of a CRISPR enzyme or the proximity thereof; a C-terminus of the enzyme or the proximity thereof; or a combination thereof. The NLS may be an NLS sequence derived from the following NLSs, but the present invention is not limited thereto: NLS of a SV40 virus large T-antigen having the amino acid sequence PKKKRKV(SEQ ID NO: 831); NLS from nucleoplasmin (e.g., nucleoplasmin bipartite NLS having the sequence KRPAATKKAGQAKKKK(SEQ ID NO: 832)); c-myc NLS having the amino acid sequence PAAKRVKLD(SEQ ID NO: 833) or RQRRNELKRSP(SEQ ID NO: 834); hRNPA1 M9 NLS having the sequence NQSSNFGPMKGGNFGGRSSGPYGGGGQYFAKPRNQGGY(SEQ ID NO: 835); the sequence RMRIZFKNKGKDTAELRRRRVEVSVELRKAKKDEQILKRRNV(SEQ ID NO: 836) of the IBB domain from importin-a; the sequences VSRKRPRP(SEQ ID NO: 837) and PPKKARED(SEQ ID NO: 838) of a myoma T protein; the sequence PQPKKKPL(SEQ ID NO: 839) of human p53; the sequence SALIKKKKKMAP(SEQ ID NO: 840) of mouse c-abl IV; the sequences DRLRR(SEQ ID NO: 841) and PKQKKRK(SEQ ID NO: 842) of influenza virus NS1; the sequence RKLKKKIKKL(SEQ ID NO: 843) of a hepatitis delta virus antigen; the sequence REKKKFLKRR(SEQ ID NO: 844) of a mouse Mx1 protein; the sequence KRKGDEVDGVDEVAKKKSKK(SEQ ID NO: 845) of a human poly (ADP-ribose) polymerase; or the sequence RKCLQAGMNLEARKTKK(SEQ ID NO: 846), derived from a sequence of a steroid hormone receptor (human) glucocorticoid.

In addition, the CRISPR enzyme mutant may include a split-type CRISPR enzyme prepared by dividing the CRISPR enzyme into two or more parts. The term "split" refers to functional or structural division of a protein or random division of a protein into two or more parts.

The split-type CRISPR enzyme may be a complete, incomplete or partially active enzyme or inactive enzyme.

For example, when the CRISPR enzyme is a SpCas9, the SpCas9 may be divided into two parts between the residue 656, tyrosine, and the residue 657, threonine, thereby generating split SpCas9.

The split-type CRISPR enzyme may selectively include an additional domain, peptide, polypeptide or protein for reconstitution.

The additional domain, peptide, polypeptide or protein for reconstitution may be assembled for formation of the split-type CRISPR enzyme to be structurally the same or similar to the wild-type CRISPR enzyme.

The additional domain, peptide, polypeptide or protein for reconstitution may be FRB and FKBP dimerization domains; intein; ERT and VPR domains; or domains which form a heterodimer under specific conditions.

For example, when the CRISPR enzyme is a SpCas9, the SpCas9 may be divided into two parts between the residue 713, serine, and the residue 714, glycine, thereby generating split SpCas9. The FRB domain may be connected to one of the two parts, and the FKBP domain may be connected to the other one. In the split SpCas9 produced thereby, the FRB domain and the FKBP domain may be formed in a dimer in an environment in which rapamycine is present, thereby producing a reconstituted CRISPR enzyme.

The CRISPR enzyme or CRISPR enzyme variant disclosed in the specification may be a polypeptide, protein or nucleic acid having a sequence encoding the same, and may be codon-optimized for a subject to introduce the CRISPR enzyme or CRISPR enzyme variant.

The term "codon optimization" refers to a process of modifying a nucleic acid sequence by maintaining a native amino acid sequence while replacing at least one codon of the native sequence with a codon more frequently or the most frequently used in host cells so as to improve expression in the host cells. A variety of species have a specific bias to a specific codon of a specific amino acid, and the codon bias (the difference in codon usage between organisms) is frequently correlated with efficiency of the translation of mRNA, which is considered to be dependent on the characteristic of a translated codon and availability of a specific tRNA molecule. The dominance of tRNA selected in cells generally reflects codons most frequently used in peptide synthesis. Therefore, a gene may be customized for optimal gene expression in a given organism based on codon optimization.

The gRNA, CRISPR enzyme or gRNA-CRISPR enzyme complex disclosed in the specification may be delivered or introduced into a subject by various forms.

The subject related description is as described above.

In one embodiment, a nucleic acid sequence encoding the gRNA and/or CRISPR enzyme may be delivered or introduced into a subject by a vector.

The vector may include the nucleic acid sequence encoding the gRNA and/or CRISPR enzyme.

In one example, the vector may simultaneously include the nucleic acid sequences encoding the gRNA and the CRISPR enzyme.

In another example, the vector may include the nucleic acid sequence encoding the gRNA.

For example, domains contained in the gRNA may be contained in one vector, or may be divided and then contained in different vectors.

In another example, the vector may include the nucleic acid sequence encoding the CRISPR enzyme.

For example, in the case of the CRISPR enzyme, the nucleic acid sequence encoding the CRISPR enzyme may be contained in one vector, or may be divided and then contained in several vectors.

The vector may include one or more regulatory/control components.

Here, the regulatory/control components may include a promoter, an enhancer, an intron, a polyadenylation signal, a Kozak consensus sequence, an internal ribosome entry site (IRES), a splice acceptor and/or a 2A sequence.

The promoter may be a promoter recognized by RNA polymerase II.

The promoter may be a promoter recognized by RNA polymerase III.

The promoter may be an inducible promoter.

The promoter may be a subject-specific promoter.

The promoter may be a viral or non-viral promoter.

The promoter may use a suitable promoter according to a control region (that is, a nucleic acid sequence encoding the gRNA and/or CRISPR enzyme).

For example, a promoter useful for the gRNA may be aH1, EF-1a, tRNA or U6 promoter. For example, a promoter useful for the CRISPR enzyme may be a CMV, EF-la, EFS, MSCV, PGK or CAG promoter.

The vector may be a viral vector or recombinant viral vector.

The virus may be a DNA virus or an RNA virus.

Here, the DNA virus may be a double-stranded DNA (dsDNA) virus or single-stranded DNA (ssDNA) virus.

Here, the RNA virus may be a single-stranded RNA (ssRNA) virus.

The virus may be a retrovirus, a lentivirus, an adenovirus, adeno-associated virus (AAV), vaccinia virus, a poxvirus or a herpes simplex virus, but the present invention is not limited thereto.

In one example, a nucleic acid sequence encoding gRNA and/or a CRISPR enzyme may be delivered or introduced by a recombinant lentivirus.

In another example, a nucleic acid sequence encoding gRNA and/or a CRISPR enzyme may be delivered or introduced by a recombinant adenovirus.

In still another example, a nucleic acid sequence encoding gRNA and/or a CRISPR enzyme may be delivered or introduced by recombinant AAV.

In yet another example, a nucleic acid sequence encoding gRNA and/or a CRISPR enzyme may be delivered or introduced by one or more hybrids of hybrid viruses, for example, the viruses described herein.

In one embodiment, the gRNA-CRISPR enzyme complex may be delivered or introduced into a subject.

For example, the gRNA may be present in the form of DNA, RNA or a mixture thereof. The CRISPR enzyme may be present in the form of a peptide, polypeptide or protein.

In one example, the gRNA and CRISPR enzyme may be delivered or introduced into a subject in the form of a gRNA-CRISPR enzyme complex including RNA-type gRNA and a protein-type CRISPR, that is, a ribonucleoprotein (RNP).

The gRNA-CRISPR enzyme complex may be delivered or introduced into a subject by electroporation, microinjection, transient cell compression or squeezing (e.g., described in the literature [Lee, et al, (2012) Nano Lett., 12, 6322-6327]), lipid-mediated transfection, nanoparticles, a liposome, peptide-mediated delivery or a combination thereof.

### The gRNA-CRISPR enzyme complex disclosed in the present specification may be used to artificially manipulate or modify a target gene, that is, a blood coagulation inhibitory gene.

A target gene may be manipulated or modified using the above-described gRNA-CRISPR enzyme complex, that is, the CRISPR complex. Here, the manipulation or modification of a target gene may include both of i) cleaving or damaging of a target gene and ii) repairing of the damaged target gene.

The i) cleaving or damaging of the target gene may be cleavage or damage of the target gene using the CRISPR complex, and particularly, cleavage or damage of a target sequence of the target gene.

The target sequence may become a target of the gRNA-CRISPR enzyme complex, and the target sequence may or may not include a PAM sequence recognized by the CRISPR enzyme. Such a target sequence may provide a critical standard to one who is involved in the designing of gRNA.

The target sequence may be specifically recognized by gRNA of the gRNA-CRISPR enzyme complex, and therefore, the gRNA-CRISPR enzyme complex may be located near the recognized target sequence.

The "cleavage" at a target site refers to the breakage of a covalent backbone of a polynucleotide. The cleavage includes enzymatic or chemical hydrolysis of a phosphodiester bond, but the present invention is not limited thereto. Other than this, the cleavage may be performed by various methods. Both of single strand cleavage and double strand cleavage are possible, wherein the double strand cleavage may result from two distinct single strand cleavages. The double strand cleavage may produce a blunt end or a staggered end (or a sticky end).

In one example, the cleavage or damage of a target gene using the CRISPR complex may be the entire cleavage or damage of the double strand of a target sequence.

In one exemplary embodiment, when the CRISPR enzyme is a wild-type SpCas9, the double strand of a target sequence forming a complementary bond with gRNA may be completely cleaved by the CRISPR complex.

In another exemplary embodiment, when the CRISPR enzymes are SpCas9 nickase (D10A) and SpCas9 nickase (H840A), the two single strands of a target sequence forming a complementary bond with gRNA may be respectively cleaved by the each CRISPR complex. That is, a complementary single strand of a target sequence forming a complementary bond with gRNA may be cleaved by the SpCas9 nickase (D10A), and a non-complementary single strand of the target sequence forming a complementary bond with gRNA may be cleaved by the SpCas9 nickase (H840A), and the cleavages may take place sequentially or simultaneously.

In another example, the cleavage or damage of a target gene or a nucleic acid using the CRISPR complex may be the cleavage or damage of only a single strand of the double strand of a target sequence. Here, the single strand may be a guide nucleic acid-binding sequence of the target sequence complementarily binding to gRNA, that is, a complementary single strand, or a guide nucleic acid non-binding sequence not complementarily binding to gRNA, that is, a non-complementary single strand to gRNA.

In one exemplary embodiment, when the CRISPR enzyme is a SpCas9 nickase (D10A), the CRISPR complex may cleave the guide nucleic acid-binding sequence of a target sequence complementarily binding to gRNA, that is, a complementary single strand, by a SpCas9 nickase (D10A), and may not cleave a guide nucleic acid non-binding sequence not complementarily binding to gRNA, that is, a non-complementary single strand to gRNA.

In another exemplary embodiment, when the CRISPR enzyme is a SpCas9 nickase (H840A), the CRISPR complex may cleave the guide nucleic acid non-binding sequence of a target sequence not complementarily binding to gRNA, that is, a non-complementary single strand to gRNA by a SpCas9 nickase (H840A), and may not cleave the guide nucleic acid-binding sequence of a target sequence complementarily binding to gRNA, that is, a complementary single strand.

In still another example, the cleavage or damage of a target gene or a nucleic acid using the CRISPR complex may be a removal of partial fragment of nucleic acid sequences.

In one exemplary embodiment, when the CRISPR complexes consist of wild-type SpCas9 and two gRNAs complementary binding to different target sequences, a double strand of a target sequence forming a complementary bond with the first gRNA may be cleaved, and a double strand of a target sequence forming a complementary bond with the second gRNA may be cleaved, resulting in the removal of nucleic acid fragments by the first and second gRNAs and SpCas9.

The ii) repairing of the damaged target gene may be repairing or restoring performed through non-homologous end joining (NHEJ) and homology-directed repair (HDR).

The non-homologous end joining (NHEJ) is a method of restoration or repairing double strand breaks in DNA by joining both ends of a cleaved double or single strand together, and generally, when two compatible ends formed by breaking of the double strand (for example, cleavage) are frequently in contact with each other to completely join the two ends, the broken double strand is recovered. The NHEJ is a restoration method that is able to be used in the entire cell cycle, and usually occurs when there is no homologous genome to be used as a template in cells, like the G1 phase.

In the repair process of the damaged gene or nucleic acid using NHEJ, some insertions and/or deletions (indels) in the nucleic acid sequence occur in the NHEJ-repaired region, such insertions and/or deletions cause the leading frame to be shifted, resulting in frame-shifted transcriptome mRNA. As a result, innate functions are lost because of nonsense-mediated decay or the failure to synthesize normal proteins. In addition, while the leading frame is maintained, mutations in which insertion or deletion of a considerable amount of sequence may be caused to destroy the functionality of the proteins. The mutation is locus-dependent because mutation in a significant functional domain is probably less tolerated than mutations in a non-significant region of a protein.

While it is impossible to expect indel mutations produced by NHEJ in a natural state, a specific indel sequence is preferred in a given broken region, and can come from a small region of micro homology. Conventionally, the deletion length ranges from 1 bp to 50 bp, insertions tend to be shorter, and frequently include a short repeat sequence directly surrounding a broken region.

In addition, the NHEJ is a process causing a mutation, and when it is not necessary to produce a specific final sequence, may be used to delete a motif of the small sequence.

A specific knockout of a gene targeted by the CRISPR complex may be performed using such NHEJ. A double strand or two single strands of a target gene or a nucleic acid may be cleaved using the CRISPR enzyme such as Cas9 or Cpfl, and the broken double strand or two single strands may have indels through the NHEJ, thereby inducing specific knockout of the target gene or nucleic acid. Here, the site of a target gene or a nucleic acid cleaved by the CRISPR enzyme may be a non-coding or coding region, and in addition, the site of the target gene or nucleic acid restored by NHEJ may be a non-coding or coding region.

In one example, the double strand of a target gene may be cleaved using the CRISPR complex, and various indels (insertions and deletions) may be generated at a repaired region by repairing through NHEJ.

The term "indel" refers to a variation formed by inserting or deleting a partial nucleotide into/from the nucleotide sequence of DNA. Indels may be introduced into the target sequence during repair by HDR or NHEJ, when the gRNA-CRISPR enzyme complex, as described above, cleaves a nucleic acid (DNA, RNA) of a blood coagulation inhibitory gene.

The homology directed repairing (HDR) is a correction method without an error, which uses a homologous sequence as a template to repair or restore the damaged gene or nucleic acid, and generally, to repair or restoration broken DNA, that is, to restore innate information of cells, the broken DNA is repaired using information of a complementary nucleotide sequence which is not modified or information of a sister chromatid. The most common type of HDR is homologous recombination (HR). HDR is a repair or restore method usually occurring in the S or G2/M phase of actively dividing cells.

To repair or restore damaged DNA using HDR, rather than using a complementary nucleotide sequence or sister chromatin of the cells, a DNA template artificially synthesized using information of a complementary nucleotide sequence or homologous nucleotide sequence, that is, a nucleic acid template including a complementary nucleotide sequence or homologous nucleotide sequence may be provided to the cells, thereby repairing or restoring the broken DNA. Here, when a nucleic acid sequence or nucleic acid fragment is further added to the nucleic acid template to repair or restore the broken DNA, the nucleic acid sequence or nucleic acid fragment further added to the broken DNA may be subjected to knockin. The further added nucleic acid sequence or nucleic acid fragment may be a nucleic acid sequence or nucleic acid fragment for correcting the target gene or nucleic acid modified by a mutation to a normal gene, or a gene or nucleic acid to be expressed in cells, but the present invention is not limited thereto.

In one example, a double or single strand of a target gene or a nucleic acid may be cleaved using the CRISPR complex, a nucleic acid template including a nucleotide sequence complementary to a nucleotide sequence adjacent to the cleavage site may be provided to cells, and the cleaved nucleotide sequence of the target gene or nucleic acid may be repaired or restored through HDR.

Here, the nucleic acid template including the complementary nucleotide sequence may have a complementary nucleotide sequence of the broken DNA, that is, a cleaved double or single strand, and further include a nucleic acid sequence or nucleic acid fragment to be inserted into the broken DNA. An additional nucleic acid sequence or nucleic acid fragment may be inserted into the broken DNA, that is, a cleaved site of the target gene or nucleic acid using the nucleic acid template including the complementary nucleotide sequence and a nucleic acid sequence or nucleic acid fragment to be inserted. Here, the nucleic acid sequence or nucleic acid fragment to be inserted and the additional nucleic acid sequence or nucleic acid fragment may be a nucleic acid sequence or nucleic acid fragment for correcting a target gene or a nucleic acid modified by a mutation to a normal gene or nucleic acid, or a gene or nucleic acid to be expressed in cells. The complementary nucleotide sequence may be a nucleotide sequence complementary binding with broken DNA, that is, right and left nucleotide sequences of the cleaved double or single strand of the target gene or nucleic acid. Alternatively, the complementary nucleotide sequence may be a nucleotide sequence complementary binding with broken DNA, that is, 3' and 5' ends of the cleaved double or single strand of the target gene or nucleic acid. The complementary nucleotide sequence may be a 15 to 3000-nt sequence, a length or size of the complementary nucleotide sequence may be suitably designed according to a size of the nucleic acid template or the target gene or the nucleic acid. Here, the nucleic acid template may be a double- or single-stranded nucleic acid, and may be linear or circular, but the present invention is not limited thereto.

In another example, a double- or single-strand of a target gene or a nucleic acid is cleaved using the CRISPR complex, a nucleic acid template including a nucleotide sequence having homology with a nucleotide sequence adjacent to a cleavage site is provided to cells, and the cleaved nucleotide sequence of the target gene or nucleic acid may be repaired or restored by HDR.

Here, the nucleic acid template including the homologous nucleotide sequence may have a nucleotide sequence having homology with the broken DNA, that is, a cleaved double- or single-strand, and further include a nucleic acid sequence or nucleic acid fragment to be inserted into the broken DNA. An additional nucleic acid sequence or nucleic acid fragment may be inserted into broken DNA, that is, a cleaved site of a target gene or a nucleic acid using the nucleic acid template including a homologous nucleotide sequence and a nucleic acid sequence or nucleic acid fragment to be inserted. Here, the nucleic acid sequence or nucleic acid fragment to be inserted and the additional nucleic acid sequence or nucleic acid fragment may be a nucleic acid sequence or nucleic acid fragment for correcting the target gene or nucleic acid modified by a mutation to a normal gene or nucleic acid, or a gene or nucleic acid to be expressed in cells. The homologous nucleotide sequence may be a nucleotide sequence having homology with the broken DNA, that is, the right and left nucleotide sequence of the cleaved double-strand or single-strand of the target gene or nucleic acid. Alternatively, the complementary nucleotide sequence may be a nucleotide sequence having homology with broken DNA, that is, the 3' and 5' ends of a cleaved double or single strand of the target gene or nucleic acid. The homologous nucleotide sequence may be a 15 to 3000-nt sequence, and a length or size of the homologous nucleotide sequence may be suitably designed according to a size of the nucleic acid template or the target gene or the nucleic acid. Here, the nucleic acid template may be a double- or single-stranded nucleic acid, and may be linear or circular, but the present invention is not limited thereto.

Other than the NHEJ and HDR, there are various methods for repairing or restoring a damaged target gene. For example, the method of repairing or restoring a damaged target gene may be single-strand annealing, single-strand break repair, mismatch repair, nucleotide cleavage repair or a method using the nucleotide cleavage repair.

The single-strand annealing (SSA) is a method of repairing double strand breaks between two repeat sequences present in a target nucleic acid, and generally uses a repeat sequence of more than 30 nucleotides. The repeat sequence is cleaved (to have sticky ends) to have a single strand with respect to a double strand of the target nucleic acid at each of the broken ends, and after the cleavage, a single-strand overhang containing the repeat sequence is coated with an RPA protein such that it is prevented from inappropriately annealing the repeat sequences to each other. RAD52 binds to each repeat sequence on the overhang, and a sequence capable of annealing a complementary repeat sequence is arranged. After annealing, a single-stranded flap of the overhang is cleaved, and synthesis of new DNA fills a certain gap to restore a DNA double strand. As a result of this repair or restore, a DNA sequence between two repeats is deleted, and a deletion length may be dependent on various factors including the locations of the two repeats used herein, and a path or degree of the progress of cleavage.

The SSA, similar to HDR, utilizes a complementary sequence, that is, a complementary repeat sequence, and in contrast, does not requires a nucleic acid template for modifying or correcting a target nucleic acid sequence.

Single strand breaks in a genome are repaired or restored through a separate mechanism, single-strand break repair (SSBR), from the above-described repair mechanisms. In the case of single-strand DNA breaks, PARP1 and/or PARP2 recognizes the breaks and recruits a repair mechanism. PARP1 binding and activity with respect to the DNA breaks are temporary, and SSBR is promoted by promoting the stability of an SSBR protein complex in the damaged regions. The most important protein in the SSBR complex is XRCC1, which interacts with a protein promoting 3' and 5' end processing of DNA to stabilize the DNA. End processing is generally involved in repairing the damaged 3' end to a hydroxylated state, and/or the damaged 5' end to a phosphatic moiety, and after the ends are processed, DNA gap filling takes place. There are two methods for the DNA gap filling, that is, short patch repair and long patch repair, and the short patch repair involves insertion of a single nucleotide. After DNA gap filling, a DNA ligase promotes end joining.

The mismatch repair (MMR) works on mismatched DNA nucleotides. Each of an MSH2/6 or MSH2/3 complex has ATPase activity and thus plays an important role in recognizing a mismatch and initiating a repair, and the MSH2/6 primarily recognizes nucleotide-nucleotide mismatches and identifies one or two nucleotide mismatches, but the MSH2/3 primarily recognizes a larger mismatch.

The base excision repair (BER) is a repair method which is active throughout the entire cell cycle, and used to remove a small non-helix-distorting base damaged region from the genome. In the damaged DNA, damaged nulceotides are removed by cleaving an N-glycoside bond joining a nucleotide to the phosphate-deoxyribose backbone, and then the phosphodiester backbone is cleaved, thereby generating breaks in single-strand DNA. The broken single strand ends formed thereby were removed, a gap generated due to the removed single strand is filled with a new complementary nucleotide, and then an end of the newly-filled complementary nucleotide is ligated with the backbone by a DNA ligase, resulting in repair or restore of the damaged DNA.

The nucleotide excision repair (NER) is an excision mechanism important for removing large helix-distorting damage from DNA, and when the damage is recognized, a short single-strand DNA segment containing the damaged region is removed, resulting in a single strand gap of 22 to 30 nucleotides. The generated gap is filled with a new complementary nucleotide, and an end of the newly filled complementary nucleotide is ligated with the backbone by a DNA ligase, resulting in the repair or restore of the damaged DNA.

Effects of artificially manipulating a target gene, that is, a blood coagulation inhibitory gene, by the gRNA-CRISPR enzyme complex may be largely knockout (knock-out), knockdown, knockin (knock-in).

The "knockout" refers to inactivation of a target gene or nucleic acid, and the "inactivation of a target gene or nucleic acid" refers to a state in which transcription and/or translation of a target gene or nucleic acid does not occur. Transcription and translation of a gene causing a disease or a gene having an abnormal function may be inhibited through knockout, resulting in the prevention of protein expression.

For example, when a target gene or a chromosome is edited using the gRNA-CRISPR enzyme complex, that is, the CRISPR complex, the target gene or the chromosome may be cleaved using the CRISPR complex. The target gene or the chromosome damaged using the CRISPR complex may be repaired by NHEJ. In the damaged target gene or chromosome, an indel is generated by NHEJ and thereby inducing target gene or chromosome-specific knockout.

In another example, when a target gene or a chromosome is edited using the gRNA-CRISPR enzyme complex, that is, the CRISPR complex, and a donor, the target gene or nucleic acid may be cleaved using the CRISPR complex. The target gene or nucleic acid damaged using the CRISPR complex may be repaired by HDR using a donor. Here, the donor includes a homologous nucleotide sequence and a nucleotide sequence desired to be inserted. Here, the number of nucleotide sequences to be inserted may vary according to an insertion location or purpose. When the damaged target gene or chromosome is repaired using a donor, a nucleotide sequence to be inserted is inserted into the damaged nucleotide sequence region, and thereby inducing target gene or chromosome-specific knockout.

The "knockdown" refers to a decrease in transcription and/or translation of a target gene or nucleic acid or the expression of a target protein. The onset of a disease may be prevented or a disease may be treated by regulating the overexpression of a gene or protein through the knockdown.

For example, when a target gene or a chromosome is edited using a gRNA-CRISPR inactive enzyme-transcription inhibitory activity domain complex, that is, a CRISPR-inactive complex including a transcription inhibitory activity domain, the CRISPR-inactive complex may specifically bind to the target gene or chromosome, and the transcription of the target gene or chromosome is inhibited by the transcription inhibitory activity domain included in the CRISPR-inactive complex, thereby inducing knockdown in which the expression of a target gene or chromosome is inhibited.

In another example, when a target gene or a chromosome is edited using the gRNA-CRISPR enzyme complex, that is, the CRISPR complex, the promoter and/or enhancer region(s) of the target gene or chromosome may be cleaved using the CRISPR complex. Here, the gRNA may recognize a partial nucleotide sequence of the promoter and/or enhancer region(s) of the target gene or chromosome as a target sequence. The target gene or chromosome damaged using the CRISPR complex may be repaired by NHEJ. In the damaged target gene or chromosome, an indel is generated by NHEJ, thereby inducing target gene or chromosome-specific knockdown. Alternatively, when a donor is optionally used, the target gene or chromosome damaged using the CRISPR complex may be repaired by HDR. When the damaged target gene or chromosome is repaired using a donor, a nucleotide sequence to be inserted is inserted into the damaged nucleotide sequence region, thereby inducing target gene or chromosome-specific knockdown.

The "knockin" refers to insertion of a specific nucleic acid or gene into a target gene or nucleic acid, and here, the "specific nucleic acid or gene" refers to a gene or nucleic acid of interest to be inserted or expressed. A mutant gene triggering a disease may be utilized in disease treatment by correction to normal or insertion of a normal gene to induce expression of the normal gene through the knockin.

In addition, the knockin may further need a donor.

For example, when a target gene or nucleic acid is edited using the gRNA-CRISPR enzyme complex, that is, the CRISPR complex, and a donor, the target gene or nucleic acid may be cleaved using the CRISPR complex. The target gene or nucleic acid damaged using the CRISPR complex may be repaired by HDR using a donor. Here, the donor may include a specific nucleic acid or gene, and may be used to insert a specific nucleic acid or gene into the damaged gene or chromosome. Here, the inserted specific nucleic acid or gene may induce the expression of a protein.

### In one embodiment of the disclosure of the present specification, the gRNA-CRISPR enzyme complex may impart an artificial manipulation or modification to a AT gene and/or a TFPI gene.

The gRNA-CRISPR enzyme complex may specifically recognize a target sequence of the AT gene and/or the TFPI gene.

The target sequence may be specifically recognized by gRNA of the gRNA-CRISPR enzyme complex, and therefore, the gRNA-CRISPR enzyme complex may be located near the recognized target sequence.

The target sequence may be a region or area in which an artificial modification occurs in the AT gene and/or the TFPI gene.

The target sequence may be a contiguous nucleotide sequence of 10 to 25 bp, located in a promoter region of the AT gene and/or the TFPI gene.

The target sequence may be a contiguous nucleotide sequence of 10 to 25 bp, located in an intron region of the AT gene and/or the TFPI gene.

The target sequence may be a contiguous nucleotide sequence of 10 to 25 bp, located in an exon region of the AT gene and/or the TFPI gene.

The target sequence may be a contiguous nucleotide sequence of 10 to 25 bp, located in an enhancer region of the AT gene and/or the TFPI gene.

The target sequence may be a contiguous nucleotide sequence of 10 to 25 bp, located near the 5' end and/or 3' end of a PAM sequence in the nucleic acid sequence of the AT gene and/or the TFPI gene.

Here, the PAM sequence may be one or more of the following sequences (described in a 5' to 3' direction):
NGG (N is A, T, C or G);
NNNNRYAC (N is each independently A, T, C or G, R is A or G, and Y is C or T);
NNAGAAW (N is each independently A, T, C or G, and W is A or T);
NNNNGATT (N is each independently A, T, C or G);
NNGRR(T) (N is each independently A, T, C or G, and R is A or G); and
TTN (N is A, T, C or G).

In one embodiment, the target sequence may be one or more nucleotide sequences selected from the nucleotide sequences described in Table 1.

The gRNA-CRISPR enzyme complex may consist of a gRNA and a CRISPR enzyme.

The gRNA may include a guide domain capable of partial or complete complementary binding with a guide nucleic acid-binding sequence of a target sequence of the AT gene and/or the TFPI gene.

The guide domain may have at least 70%, 75%, 80%, 85%, 90% or 95% complementarity or complete complementarity to the guide nucleic acid-binding sequence.

The guide domain may include a nucleotide sequence complementary to the guide nucleic acid-binding sequence of the target sequence of the AT gene. Here, the complementary nucleotide sequence may include 0 to 5, 0 to 4, 0 to 3, or 0 to 2 mismatches.

The guide domain may include a nucleotide sequence complementary to the guide nucleic acid-binding sequence of the target sequence of the TFPI gene. Here, the complementary nucleotide sequence may include 0 to 5, 0 to 4, 0 to 3, or 0 to 2 mismatches.

The gRNA may include one or more domains selected from the group consisting of a first complementary domain, a linker domain, a second complementary domain, a proximal domain and a tail domain.

The CRISPR enzyme may be one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein. In one example, the editor protein may be a Campylobacter jejuni-derived Cas9 protein or a Staphylococcus aureus-derived Cas9 protein.

The gRNA-CRISPR enzyme complex may impart various artificial manipulations or modifications to the AT gene and/or the TFPI gene.

In the artificially manipulated or modified AT gene and/or the TFPI gene, one or more modifications may occur in a contiguous nucleotide sequence region of 1 to 50 bp, located in a target sequence or near the 5' end and/or 3' end of a target sequence. The modifications are as follows:
i) deletion of one or more nucleotides,
ii) substitution of one or more nucleotides with nucleotide(s) different from a wild-type gene,
iii) insertion of one or more nucleotides, or
iv) a combination of two or more selected from i) to iii).

For example, in the artificially manipulated or modified AT gene and/or the TFPI gene, one or more nucleotides may be deleted in a contiguous nucleotide sequence region of 1 to 50 bp, located in a target sequence or near the 5' end and/or 3' end of a target sequence. In one example, the deleted nucleotides may be a 1, 2, 3, 4 or 5 bp sequential or non-sequential nucleotides. In another example, the deleted nucleotides may be a nucleotide fragment consisting of sequential nucleotides of 2 bp or more. Here, the nucleotide fragment may be 2 to 5 bp, 6 to 10 bp, 11 to 15 bp, 16 to 20 bp, 21 to 25 bp, 26 to 30 bp, 31 to 35 bp, 36 to 40 bp, 41 to 45 bp or 46 to 50 bp in size. In still another example, the deleted nucleotides may be two or more nucleotide fragments. Here, two or more nucleotide fragments may be independent nucleotide fragments, which are not contiguous, that is, have one or more nucleotide sequence gaps, and two or more deletion sites may be generated due to the two or more deleted nucleotide fragments.

Alternatively, for example, in the artificially manipulated or modified AT gene and/or the TFPI gene, the insertion of one or more nucleotides may occur in a contiguous nucleotide sequence region of 1 to 50 bp, located in a target sequence or near the 5' end and/or 3' end of a target sequence. In one example, the inserted nucleotides may be a 1, 2, 3, 4 or 5 bp sequential nucleotides. In another example, the inserted nucleotides may be a nucleotide fragment consisting of 5 bp or more sequential nucleotides. Here, the nucleotide fragment may be 5 to 10 bp, 11 to 50 bp, 50 to 100 bp, 100 to 200 bp, 200 to 300 bp, 300 to 400 bp, 400 to 500 bp, 500 to 750 bp or 750 to 1000 bp in size. In still another example, the inserted nucleotides may be a partial or complete nucleotide sequence of a specific gene. Here, the specific gene may be a gene introduced from the outside, which is not included in an object including a AT gene and/or the TFPI gene, for example, a human cell. Alternatively, the specific gene may be a gene included in an object including a AT gene and/or the TFPI gene, for example, a human cell. For example, the specific gene may be a gene present in the genome of a human cell.

Alternatively, for example, in the artificially manipulated or modified AT gene and/or the TFPI gene, one or more nucleotides may be deleted and inserted in a contiguous nucleotide sequence region of 1 to 50 bp, located in a target sequence or near the 5' end and/or 3' end of a target sequence. In one example, the deleted nucleotides may be a 1, 2, 3, 4 or 5 bp sequential or non-sequential nucleotides. Here, the inserted nucleotides may be a 1, 2, 3, 4 or 5 bp nucleotides; a nucleotide fragment; or a partial or complete nucleotide sequence of a specific gene, and the deletion and insertion may sequentially or simultaneously occur. Here, the inserted nucleotide fragment may be 5 to 10 bp, 11 to 50 bp, 50 to 100 bp, 100 to 200 bp, 200 to 300 bp, 300 to 400 bp, 400 to 500 bp, 500 to 750 bp or 750 to 1000 bp in size. Here, the specific gene may be a gene introduced from the outside, which is not included in an object including a AT gene and/or the TFPI gene, for example, a human cell. Alternatively, the specific gene may be a gene included in an object including a AT gene and/or the TFPI gene, for example, a human cell. For example, the specific gene may be a gene present in the genome of a human cell. In another example, the deleted nucleotides may be a nucleotide fragment consisting of 2 bp or more nucleotides. Here, the deleted nucleotide fragment may be 2 to 5 bp, 6 to 10 bp, 11 to 15 bp, 16 to 20 bp, 21 to 25 bp, 26 to 30 bp, 31 to 35 bp, 36 to 40 bp, 41 to 45 bp or 46 to 50 bp in size. Here, the inserted nucleotides may be 1, 2, 3, 4 or 5 bp nucleotides; a nucleotide fragment; or a partial or complete nucleotide sequence of a specific gene, and the deletion and insertion may sequentially or simultaneously occur. In still another example, the deleted nucleotides may be two or more nucleotide fragments. Here, the inserted nucleotides may be 1, 2, 3, 4 or 5 bp nucleotides; a nucleotide fragment; or a partial or complete nucleotide sequence of a specific gene, and the deletion and insertion may sequentially or simultaneously occur. In addition, the insertion may occur in a partial or complete part of the two or more deleted parts.

The gRNA-CRISPR enzyme complex may impart various artificial manipulations or modifications to the AT gene and/or the TFPI gene according to the types of gRNA and CRISPR enzyme.

In one example, when the CRISPR enzyme is a SpCas9 protein, in the artificially manipulated or modified AT gene and/or the TFPI gene, one or more modifications may be included in a contiguous nucleotide sequence region of 1 to 50 bp, 1 to 40 bp or 1 to 30 bp, and preferably, 1 to 25 bp, located near the 5' end and/or 3' end of the PAM sequence of 5'-NGG-3' (N is A, T, G or C) present in a target region of each gene. The modifications are as follows:
i) deletion of one or more nucleotides,
ii) substitution of one or more nucleotides with nucleotide(s) different from a wild-type gene,
iii) insertion of one or more nucleotides, or
iv) a combination of two or more selected from i) to iii).

In another example, when the CRISPR enzyme is a CjCas9 protein, in the artificially manipulated or modified AT gene and/or the TFPI gene, one or more modifications may be included in a contiguous nucleotide sequence region of 1 to 50 bp, 1 to 40 bp or 1 to 30 bp, and preferably, 1 to 25 bp, located near the 5' end and/or 3' end of the PAM sequence of 5'-NNNNRYAC-3' (N is each independently A, T, C or G, R is A or G, and Y is C or T) present in a target region of each gene. The modifications are as follows:
i) deletion of one or more nucleotides,
ii) substitution of one or more nucleotides with nucleotide(s) different from a wild-type gene,
iii) insertion of one or more nucleotides, or
iv) a combination of two or more selected from i) to iii).

In still another example, when the CRISPR enzyme is a StCas9 protein, in the artificially manipulated or modified AT gene and/or the TFPI gene, one or more modifications may be included in a contiguous nucleotide sequence region of 1 to 50 bp, 1 to 40 bp or 1 to 30 bp, and preferably, 1 to 25 bp, located near the 5' end and/or 3' end of the PAM sequence of 5'-NNAGAAW-3'(N is each independently A, T, C or G, and W is A or T) present in a target region of each gene. The modifications are as follows:
i) deletion of one or more nucleotides,
ii) substitution of one or more nucleotides with nucleotide(s) different from a wild-type gene,
iii) insertion of one or more nucleotides, or
iv) a combination of two or more selected from i) to iii).

In one example, when the CRISPR enzyme is a NmCas9 protein, in the artificially manipulated or modified AT gene and/or the TFPI gene, one or more modifications may be included in a contiguous nucleotide sequence region of 1 to 50 bp, 1 to 40 bp or 1 to 30 bp, and preferably, 1 to 25 bp, located near the 5' end and/or 3' end of the PAM sequence of 5'-NNNNGATT-3' (N is each independently A, T, C or G) present in a target region of each gene. The modifications are as follows:
i) deletion of one or more nucleotides,
ii) substitution of one or more nucleotides with nucleotide(s) different from a wild-type gene,
iii) insertion of one or more nucleotides, or
iv) a combination of two or more selected from i) to iii).

In yet another example, when the CRISPR enzyme is a SaCas9 protein, in the artificially manipulated or modified AT gene and/or the TFPI gene, one or more modifications may be included in a contiguous nucleotide sequence region of 1 to 50 bp, 1 to 40 bp or 1 to 30 bp, and preferably, 1 to 25 bp, located near the 5' end and/or 3' end of the PAM sequence of 5'-NNGRR(T)-3' (N is each independently A, T, C or G, R is A or G, and (T) means any insertable sequence) present in a target region of each gene. The modifications are as follows:
i) deletion of one or more nucleotides,
ii) substitution of one or more nucleotides with nucleotide(s) different from a wild-type gene,
iii) insertion of one or more nucleotides, or
iv) a combination of two or more selected from i) to iii).

In yet another example, when the CRISPR enzyme is a Cpfl protein, in the artificially manipulated or modified AT gene and/or the TFPI gene, one or more modifications may be included in a contiguous nucleotide sequence region of 1 to 50 bp, 1 to 40 bp or 1 to 30 bp, and preferably, 1 to 25 bp, located near the 5' end and/or 3' end of the PAM sequence of 5'-TTN-3' (N is A, T, C or G) present in a target region of each gene. The modifications are as follows:
i) deletion of one or more nucleotides,
ii) substitution of one or more nucleotides with nucleotide(s) different from a wild-type gene,
iii) insertion of one or more nucleotides, or
iv) a combination of two or more selected from i) to iii).

The artificial manipulation effect on the AT gene and/or the TFPI gene, caused by the gRNA-CRISPR enzyme complex, may be knock-out.

The artificial manipulation effect on the AT gene and/or the TFPI gene, caused by the gRNA-CRISPR enzyme complex, may be to inhibit the expression of a protein encoded by each of the AT gene and/or the TFPI gene.

The artificial manipulation effect on the AT gene and/or the TFPI gene, caused by the gRNA-CRISPR enzyme complex, may be knock-down.

The artificial manipulation effect on the AT gene and/or the TFPI gene, caused by the gRNA-CRISPR enzyme complex, may be to reduce the expression of a protein encoded by each of the AT gene and/or the TFPI gene.

The artificial manipulation effect on the AT gene and/or the TFPI gene, caused by the gRNA-CRISPR enzyme complex, may be knock-in.

Here, the knock-in effect may be induced by the gRNA-CRISPR enzyme complex and a donor additionally including a foreign nucleotide sequence or gene.

The artificial manipulation effect on the AT gene and/or the TFPI gene, caused by the gRNA-CRISPR enzyme complex and a donor, may be to express a peptide or protein encoded by a foreign nucleotide sequence or gene.

### One aspect disclosed in the present specification relates to a method of treating a coagulopathy using a composition for gene manipulation for treating a coagulopathy.

One embodiment disclosed in the present specification provides a use for treating a coagulopathy using a method including administering a composition for gene manipulation for artificially manipulating a blood coagulation inhibitory gene into a treatment subject.

Here, the treatment subject may be a mammal including primates such as a human, a monkey, etc. and rodents such as a rat, a mouse, etc.

The term "coagulopathy (i.e., a bleeding disorder)" refers to a condition in which the formation of blood clots is inhibited or suppressed by an abnormal blood coagulation system. This coagulopathy includes conditions in which blood coagulation, that is, thrombogenesis is inhibited or suppressed, and thus, bleeding does not stop or hemostasis is delayed. It refers to a pathological condition including all types of diseases caused thereby.

In this case, the coagulopathy includes all types of diseases induced by the abnormal blood coagulation system and the blood coagulation inhibitory factors.

The term "blood coagulation inhibitory factor-induced disease" refers to all the conditions that include all types of diseases caused due to the abnormal forms (for example, mutations, and the like) or abnormal expression of the blood coagulation inhibitory factor.

Coagulopathy may be hemophilia that is a disease caused by the abnormal blood coagulation system *in vivo.*

In one embodiment, coagulopathy may be hemophilia A, hemophilia B, or hemophilia C.

Hemophilia (or haemophilia) is a congenital bleeding disease that is inherently caused by the deficiency of blood coagulation factors. There are 12 blood coagulation factors known so far. Among the symptoms of congenital blood coagulation deficiency, hemophilia A is caused by the deficiency of factor VIII, hemophilia B is caused by the deficiency of factor IX (Christmas disease), and hemophilia C is caused by the deficiency of factor XI. Hemophilia A and B account for 95% or more of hemophilia, and may not be easily clinically distinguished from each other because both of the two diseases have coagulation factors associated with an intrinsic coagulation mechanism. Hemophilia A and B are inherited as an X-linked recessive trait, and females are silent carriers, and only the male babies remain as patients.

Hemophilia A is symptomatic of factor VIII deficiency. 20 to 30% of hemophilia A is caused by mutations without any family history. Its incidence is approximately one in every 4,000 to 10,000 male babies, and hemophilia A has an incidence approximately 5- to 8-fold higher than hemophilia B.

Hemophilia B is the second most common type of hereditary coagulopathic disorder, and the bleeding symptom of hemophilia B is observed to be more pronounced in children and teenagers than in adults. Female carriers have slight symptoms, but 10% of the female carriers have a risk of bleeding. Its incidence is approximately one in every 20,000 to 25,000 male babies.

Hemophilia C accounts for approximately 2 to 3% of all coagulation factor deficiencies. Hemophilia C cases have not been reported in Korea, which is inherited as autosomally recessive.

The clinical symptom of hemophilia A is uncontrolled bleeding after traumatic injury, tooth extraction, and surgery. Severe hemophilia A is likely to be diagnosed within a year after birth, and spontaneous bleeding symptoms appear at 2 to 5 months when it is not treated.

Also, moderately severe hemophilia A tends to develop spontaneous bleeding. However, due to a delay in the appearance of symptoms, it is diagnosed before the age of 5 to 6 years by uncontrolled bleeding after minor trauma. Spontaneous bleeding is not observed in the case of mild hemophilia A. Mild hemophilia A has a symptom of uncontrolled bleeding after surgery, tooth extraction, and severe injuries, and in many case, may not be diagnosed during a lifetime. The bleeding symptom of hemophilia A is observed to be more pronounced in children and teenagers than in adults. Female carriers have mild symptoms, but 10% of the female carriers have a risk of bleeding.

Due to the deficiency of factor IX, hemophilia B has a symptom of uncontrolled bleeding after initial bleeding after traumatic injury, tooth extraction, or surgery has stopped. Hemarthrosis is frequently observed in severe hemophilia B. Severe hemophilia B is likely to be diagnosed within a year after birth, and spontaneous bleeding symptoms appear at 2 to 5 months when it is not treated. Also, moderately severe hemophilia B tends to develop spontaneous bleeding, but its appearance is delayed compared to severe hemophilia B. Moderately severe hemophilia B has a symptom of uncontrolled bleeding after traumatic injury, and is diagnosed before the age of 5 to 6 years. Mild hemophilia B has no spontaneous bleeding, and has a symptom of uncontrolled bleeding after surgery, tooth extraction, and severe injuries. Mild hemophilia B may not be diagnosed at all during a lifetime because it has no symptoms.

General treatment of hemophilia is achieved by supplementing insufficient blood coagulation factors. In this case, factor VIII and factor IX preparations are currently used, and a bypass factor is administered when antibodies are produced during treatment (Kemton CL et al., Blood. 2009; 113(1): 11-17).

One embodiment of the disclosure of the present specification provides a pharmaceutical composition including a composition for gene manipulation, which may artificially manipulate a blood coagulation inhibitory gene.

The description related to the composition for gene manipulation is as described above.

In one embodiment, the composition for gene manipulation may include the following components:
(a) a guide nucleic acid capable of targeting a target sequence of a blood coagulation inhibitory gene or a nucleic acid sequence encoding the same; and
(b) an editor protein including one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein, or nucleic acid sequence(s) encoding the same.

Here, the blood coagulation inhibitory gene may be a AT gene and/or a TFPI gene.

Here, the composition for gene manipulation may include a vector including nucleic acid sequences encoding a guide nucleic acid and/or an editor protein, respectively.

Here, the guide nucleic acid or a nucleic acid sequence encoding the same; and a nucleic acid sequence encoding the editor protein may be present in the form of one or more vectors. They may be present in form of a homologous or heterologous vector.

In another embodiment, the composition for gene manipulation may include the following components:
(a) a guide nucleic acid capable of targeting a target sequence of a blood coagulation inhibitory gene or a nucleic acid sequence encoding the same;
(b) an editor protein including one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein, or nucleic acid sequence(s) encoding the same; and
(c) a donor including a nucleic acid sequence to be inserted or a nucleic acid sequence encoding the same.

Here, the nucleic acid sequence to be inserted may be a partial sequence of the blood coagulation inhibitory gene.

Alternatively, the nucleic acid sequence to be inserted may be a complete or partial sequence of a gene encoding a blood coagulation associated protein.

Here, the blood coagulation associated protein may be factor XII, factor XIIa, factor XI, factor XIa, factor IX, factor IXa, factor X, factor Xa, factor VIII, factor VIIIa, factor VII, factor VIIa, factor V, factor Va, prothrombin, thrombin, factor XIII, factor XIIIa, fibrinogen, fibrin or Tissue factor.

Here, the guide nucleic acid or a nucleic acid sequence encoding the same; and a nucleic acid sequence encoding the editor protein; and a donor including a nucleic acid sequence to be inserted or a nucleic acid sequence encoding the same may be present in the form of one or more vectors. They may be present in the form of a homologous or heterologous vector.

The pharmaceutical composition may further include an additional component.

The additional component may include a suitable carrier for delivery into the body of a subj ect.

In another embodiment, the composition for gene manipulation may include the following components:
(a) a guide nucleic acid including guide sequence capable of forming a complementary bond with a target sequence of a blood coagulation inhibitory gene or a nucleic acid sequence encoding the same; and
(b) an editor protein including one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein, or nucleic acid sequence(s) encoding the same.

Here, the blood coagulation inhibitory gene may be an AT gene and/or a TFPI gene.

Here, the composition for gene manipulation may include a vector including nucleic acid sequences encoding a guide nucleic acid and/or an editor protein, respectively.

Here, the guide nucleic acid or a nucleic acid sequence encoding the same; and a nucleic acid sequence encoding the editor protein may be present in the form of one or more vectors. They may be present in the form of a homologous or heterologous vector.

In another embodiment, the composition for gene manipulation may include the following components:
(a) a guide nucleic acid including guide sequence capable of forming a complementary bond with a target sequence of a blood coagulation inhibitory gene or a nucleic acid sequence encoding the same;
(b) an editor protein including one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein, or nucleic acid sequence(s) encoding the same; and
(c) a donor including a nucleic acid sequence to be inserted or a nucleic acid sequence encoding the same.

Here, the nucleic acid sequence to be inserted may be a partial sequence of the blood coagulation inhibitory gene.

Alternatively, the nucleic acid sequence to be inserted may be a complete or partial sequence of a gene encoding a blood coagulation associated protein.

Here, the blood coagulation associated protein may be factor XII, factor XIIa, factor XI, factor XIa, factor IX, factor IXa, factor X, factor Xa, factor VIII, factor VIIIa, factor VII, factor VIIa, factor V, factor Va, prothrombin, thrombin, factor XIII, factor XIIIa, fibrinogen, fibrin or Tissue factor.

Here, the guide nucleic acid or a nucleic acid sequence encoding the same; and a nucleic acid sequence encoding the editor protein; and a donor including a nucleic acid sequence to be inserted or a nucleic acid sequence encoding the same may be present in the form of one or more vectors. They may be present in the form of a homologous or heterologous vector.

The pharmaceutical composition may further include an additional component.

The additional component may include a suitable carrier for delivery into the body of a subj ect.

One embodiment of the disclosure of the present specification provides a method of treating a coagulopathy, which include administering a composition for gene manipulation to an organism with a coagulopathy to treat the coagulopathy.

The treatment method may be a treatment method of regulating a blood coagulation system by manipulating a gene of an organism. Such a treatment method may be achieved by directly injecting a composition for gene manipulation into a body to manipulate a gene of an organism.

The description related to the composition for gene manipulation is as described above.

In one embodiment, the composition for gene manipulation may include the following components:
(a) a guide nucleic acid capable of targeting a target sequence of a blood coagulation inhibitory gene or a nucleic acid sequence encoding the same; and
(b) an editor protein including one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein, or nucleic acid sequence(s) encoding the same.

Alternatively, in another embodiment, the composition for gene manipulation may include the following components:
(a) a guide nucleic acid including guide sequence capable of forming a complementary bond with a target sequence of a blood coagulation inhibitory gene or a nucleic acid sequence encoding the same; and
(b) an editor protein including one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein, or nucleic acid sequence(s) encoding the same.

Here, the blood coagulation inhibitory gene may be an AT gene and/or a TFPI gene.

The guide nucleic acid and the editor protein may each be present in one or more vectors in the form of a nucleic acid sequence, or present by forming a complex by combining the guide nucleic acid and the editor protein.

Optionally, the composition for gene manipulation may further include a donor including a nucleic acid sequence to be inserted or a nucleic acid sequence encoding the same.

Each of the guide nucleic acid and the editor protein, and/or a donor may be present in one or more vectors in the form of a nucleic acid sequence.

Here, the vector may be a plasmid or a viral vector.

Here, the viral vector may be one or more selected from the group consisting of a retrovirus, a lentivirus, an adenovirus, an adeno-associated virus (AAV), a vaccinia virus, a poxvirus and a herpes simplex virus.

The description related to the coagulopathy is as described above.

The coagulopathy may be hemophilia A, hemophilia B or hemophilia C.

The composition for gene manipulation may be administered into a treatment subject with a coagulopathy.

The treatment subject may be a mammal including primates such as a human, a monkey, etc. and rodents such as a rat, a mouse, etc.

The composition for gene manipulation may be administered to a treatment subject.

The administration may be performed by injection, transfusion, implantation or transplantation.

The administration may be performed via an administration route selected from intrahepatic, subcutaneous, intradermal, intraocular, intravitreal, intratumoral, intranodal, intramedullary, intramuscular, intravenous, intralymphatic or intraperitoneal routes.

A dose (a pharmaceutically effective amount for obtaining a predetermined, desired effect) of the composition for gene manipulation is approximately 10⁴ to 10⁹ cells/kg (body weight of an administration subject), for example, approximately 10⁵ to 10⁶ cells/kg (body weight), which may be selected from all integers in the numerical range, but the present invention is not limited thereto. The composition may be suitably prescribed in consideration of an age, health condition and body weight of an administration subject, the type of concurrent treatment, and if possible, the frequency of treatment and a desired effect.

When the blood coagulation inhibitory gene is artificially manipulated using the method and the composition according to some embodiment disclosed in the present specification, it is possible to normalize the blood coagulation system, thereby achieving an effect of inhibiting or improving an abnormal bleeding symptom, and the like.

### One embodiment disclosed in the present specification relates to a method of modifying a blood coagulation inhibitory gene in eukaryotic cells, which may be performed in vivo, ex vivo or in vitro.

In some embodiments, the method includes sampling a cell or a cell population from a human or non-human animal, and modifying the cell or cells. Culturing may occur at any stage *ex vivo.* The cell or cells may even be re-introduced into a non-human animal or plant.

The method may be a method of artificially manipulating eukaryotic cells, which includes introducing a composition for gene manipulation into eukaryotic cells.

The description related to the composition for gene manipulation is as described above.

In one embodiment, the composition for gene manipulation may include the following components:
(a) a guide nucleic acid capable of targeting a target sequence of a blood coagulation inhibitory gene or a nucleic acid sequence encoding the same; and
(b) an editor protein including one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein, or nucleic acid sequence(s) encoding the same.

Alternatively, in another embodiment, the composition for gene manipulation may include the following components:
(a) a guide nucleic acid including guide sequence capable of forming a complementary bond with a target sequence of a blood coagulation inhibitory gene or a nucleic acid sequence encoding the same; and
(b) an editor protein including one or more proteins selected from the group consisting of a Streptococcus pyogenes-derived Cas9 protein, a Campylobacter jejuni-derived Cas9 protein, a Streptococcus thermophilus-derived Cas9 protein, a Staphylococcus aureus-derived Cas9 protein, a Neisseria meningitidis-derived Cas9 protein and a Cpfl protein, or nucleic acid sequence(s) encoding the same.

Here, the blood coagulation inhibitory gene may be a AT gene and/or a TFPI gene.

The guide nucleic acid and the editor protein may each be present in one or more vectors in the form of a nucleic acid sequence, or present by forming a complex by combining the guide nucleic acid and the editor protein.

The introduction step may be performed *in vivo* or *ex vivo.*

For example, the introduction step may be performed by one or more methods selected from electroporation, liposomes, plasmids, viral vectors, nanoparticles and a protein translocation domain (PTD) fusion protein method.

For example, the viral vector may be one or more selected from the group consisting of a retrovirus, a lentivirus, an adenovirus, an adeno-associated virus (AAV), a vaccinia virus, a poxvirus and a herpes simplex virus.

### [Examples]

Hereinafter, the present invention will be described in further detail with reference to examples.

These examples are merely provided to illustrate the present invention, and it will be obvious to those of ordinary skill in the art that the scope of the present invention is not limited by the following examples.

### Experimental method

### 1. Design of sgRNA

Targets for CRISPR/SpCas9 or CRISPR/SaCas9 or CRISPR/CjCas9 were extracted from human SERPINC1 and TFPI genes, in consideration of each PAM, using a Cas-Designer program of CRISPR RGEN Tools (Institute for Basic Science, Korea). Also, the targets whose 1- and 2-base mismatched off-target sites are not expected in the human genome were screened using the Cas-Offinder program. The sgRNA used in this experiment was designed to include at least one of the guide sequences listed in Tables3 and 4.

### 2. Verification of Guide RNA activity and off-target analysis

HEK293 and Jurkat human cell lines (cell counts: 2 × 10⁵ cells) were transfected with 250 ng of an sgRNA expression vector cloned with each guide RNA sequence together with 750 ng of a Cas9 expression vector using Lipofectamine 2000. Alternatively, 1 µg of *in vitro* transcribed sgRNA and 4 µg of Cas9 were mixed in the form of an RNP complex, and transfected through electroporation. After approximately 2 to 3 days, genomic DNA was extracted, and at an on-target site was amplified by PCR, and the resulting PCR product was subjected to additional PCR to ligate an adaptor specific to sequencing primers for Next-Generation Sequencing and TruSeq HT Dual Index primers to the PCR product. Thereafter, reads obtained through the paired sequencing were analyzed to evaluate the activities of guide RNAs through confirmation of insertions or deletions (Indels) at on-target genomic sites.

For the off-target analysis of the screened guide RNAs, first, 3-base mismatched off-target lists were screened by an in-silico method using Cas-Offinder of the CRISPR RGEN Tools, and verified by a method through targeted-deep sequencing of a certain region of the genome corresponding to each off-target site. As a second method, the human whole genomic DNA, which had been treated with the guide RNA and a Cas9 protein overnight at 37°C, was subjected to whole genome sequencing, and potential lists were secured through Digenome-seq analysis. Then, it was verified whether the indels were added at off-target sites by a method through targeted-deep sequencing of a certain region of the genome of each of off-target candidates.

### 3. AAV construction

An SpCas9 gene editing tool was delivered using a dual AAV system. That is, each of a vector (pAAV-SpCas9) including mammalian expression promoters (EFS, TBG, and ICAM2) between inverted tandem repeats (ITRs) of AAV2, human codon-optimized Cas9 having NLS-HA-tags at the C- or N-termini thereof, and BGHA and a vector (pAAV-U6-sgRNA) including a U6 promoter sgRNA sequence was constructed by synthesis.

Also, another dual AAV system used Split SpCas9. In one AAV, a vector including a promoter (TBG, ICAM2)-SpCas9-Nterm-Intein between the ITRs was constructed (pAAV-SpCas9-split-Nterm), and, in the other AAV, a vector including a promoter (TBG, ICAM2)-Intein-SpCas9-Cterm-U6-SgRNA between the ITRs was constructed (pAAV-SpCas9-split-C-term-U6-SgRNA).

A CjCas9 gene editing tool was delivered using a single AAV system. That is, a vector (pAAV-CjCas9-U6-sgRNA) including promoters (EFS, TBG, and ICAM2) between AAV2 ITRs, human codon-optimized Cas9 having NLS- HA-tags at the C- or N-termini thereof, BGHA, U6 promoter, and an sgRNA sequence was constructed by synthesis. For AAV production, HEK293 cells were transfected with a vector for a pseudotype of an AAV capsid, the constructed pAAV vector (pAAV-EFS-SpCas9, or pAAV-U6-sgRNA, or pAAV-EFS-CjCas9-U6-sgRNA), and a pHelper vector at a 1:1:1 molar concentration at the same time. After 72 hours, the cells were lysed, and the resulting virus particles were than separated and purified by a step-gradient ultracentrifuge using iodixanol (Sigma-Aldrich), and quantitative determination of AAV was performed by a titration method using qPCR.

### 4. Animals and injection

F8-KO mice and F9-KO rats were used as hemophilia A and B model animals, respectively. Each CRISPR/Cas9 was delivered to an animal model using a method of intravenously or intraperitoneally injecting the constructed AAV and LNP.

### 5. In vivo editing test

Approximately 4 to 6 weeks after AAV and LNP injection, the liver was removed, and genomic DNA was extracted to evaluate the indels at On-target and Off-target sites through PCR and targeted-deep-sequencing.

### 6. Hemophilia indicator test

After the AAV and LNP injection, blood was collected at various time points (1W-4W-8W-16W-32W, and the like) to evaluate an amount of Serpinc1 and TFPI proteins in blood by ELISA or to evaluate a hemophilia indicator by PT, an aPTT assay, immunohistochemistry, a tail cutting assay (blood loss quantification), and the like.

### Experimental results

### 1. Verification of activity of Guide RNA

The activity of gRNA targeting each of the SERPINC1 gene (AT gene) and the TFPI gene was confirmed through the indels (%) to screen the gRNA.

### 1) SERPINC1 gene (AT gene)

### ① SpCas9

**[Table 5]**

| gRNA selection to target SERPINC1 gene(AT gene) for SpCas9 | | | | |
|---|---|---|---|---|
| Target name | Target(w/o PAM) (SEQ ID NO) | PAM | GC content | Indels (%) |
| hMyd88-Sp-Ctrl | GTCCA TCTCCTCCGCCAGCG(SEQ ID NO: 847) | CGG | | 75.0 |
| hSerpinc 1-Sp-3 | GCCATGTATTCCAATGTGAT(SEQ ID NO: 21) | AGG | 40 | 49.9 |
| hSerpinc1-Sp-4 | GTGATAGGAACTGTAACCTC(SEQ ID NO: 22) | TGG | 45 | 42.2 |
| hSerpinc1-Sp-12 | GGGACTGCGTGACCTGTCAC(SEQ ID NO: 30) | GGG | 65 | 61.2 |
| hSerpinc1-Sp-13 | GTCCACAGGGCTCCCGTGAC(SEQ ID NO: 31) | AGG | 70 | 29.4 |
| hSerpinc1-Sp-19 | CA TGGGAATGTCCCGCGGCT(SEQ ID NO: 37) | TGG | 65 | 56.1 |
| hSerpinc1-Sp-20 | GGATTCATGGGAATGTCCCG(SEQ ID NO: 38) | CGG | 55 | 1.3 |
| hSerpinc 1-Sp-23 | GGGGAGCGGTAAATGCACAT(SE Q ID NO: 41) | GGG | 55 | 49.3 |
| hSerpinc 1-Sp-24 | CGGGGAGCGGTAAATGCACA(SE Q ID NO: 42) | TGG | 60 | 4.2 |
| hSerpinc 1-Sp-25 | CATGTGCATTTACCGCTCCC(SEQ ID NO: 43) | CGG | 55 | 58.3 |
| hSerpinc1-Sp-30 | TTACCGCTCCCCGGAGAAGA(SEQ ID NO: 48) | AGG | 60 | 49.5 |
| hSerpinc 1-Sp-34 | GGGCTCAGAACAGAAGATCC(SE Q ID NO: 52) | CGG | 55 | 45.7 |
| hSerpinc1-Sp-36 | CACGCCGGTTGGTGGCCTCC(SEQ ID NO: 54) | GGG | 75 | 14.9 |
| hSerpinc1-Sp-37 | ACACGCCGGTTGGTGGCCTC(SEQ ID NO: 55) | CGG | 70 | 6.2 |
| hSerpinc1-Sp-39 | TTCCCAGACACGCCGGTTGG(SEQ ID NO: 57) | TGG | 65 | 22.5 |
| hSerpinc 1-Sp-40 | CAGTTCCCAGACACGCCGGT(SEQ ID NO: 58) | TGG | 65 | 22.1 |
| hSerpinc 1-Sp-42 | AGGCCACCAACCGGCGTGTC(SE Q ID NO: 60) | TGG | 70 | 0.2 |
| hSerpinc 1-Sp-43 | GGCCACCAACCGGCGTGTCT(SEQ ID NO: 61) | GGG | 70 | 8.2 |
| hSerpinc 1-Sp-45 | AGCAAAGCGGGAATTGGCCT(SE Q ID NO: 63) | TGG | 55 | 0.0 |
| hSerpinc1-Sp-49 | TGCCAGGTGCTGATAGAAAG(SE Q ID NO: 67) | TGG | 50 | 34.4 |
| hSerpinc 1-Sp-50 | TACCACTTTCTATCAGCACC(SEQ ID NO: 68) | TGG | 45 | 27.3 |

### ② SaCas9

**[Table 6]**

| gRNA selection to target SERPINC1 gene(AT gene) for SaCas9 | | | | |
|---|---|---|---|---|
| Target name | Target(w/o PAM) (SEQ ID NO) | PAM | GC content | Indels (%) |
| hAPOC3 -Cj -Ctrl | GAGAGGGCCAGAAATCACCCAA( SEQ ID NO: 848) | AGACA CAC | | 64.1 |
| hSerpinc 1-Sa-1 | GGTT ACAGTTCCT A TCACA T(SEQ ID NO: 216) | TGGAA T | 40 | 51.6 |
| hSerpinc 1-Sa-2 | CCAAGCCGCGGGACATTCCC(SEQ ID NO: 217) | ATGAA T | 70 | 65.2 |
| hSerpinc 1-Sa-3 | TAAATGCACATGGGATTCAT(SEQ ID NO: 218) | GGGAA T | 35 | 49.2 |
| hSerpinc1-Sa-4 | CGGGGAGCGGTAAATGCACA(SE Q ID NO: 219) | TGGGA T | 60 | 34.5 |
| hSerpinc 1-Sa-5 | CCCCGGAGAAGAAGGCAACT(SE Q ID NO: 220) | GAGGA T | 60 | 44.7 |
| hSerpinc1-Sa-6 | ACACGCCGGTTGGTGGCCTC(SEQ ID NO: 221) | CGGGA T | 70 | 0.8 |
| hSerpinc1-Sa-7 | ATAGAAAGTGGTAGCAAAGC(SE Q ID NO: 222) | GGGAA T | 40 | 68.3 |
| hSerpinc1-Sa-9 | AATGTTATCATTGTCATTCT(SEQ ID NO: 224) | TGGAA T | 25 | 12.9 |
| hSerpinc1-Sa-11 | CAAAAGCCGTGGAGATACTC(SEQ ID NO: 226) | AGGGG T | 50 | 58.9 |
| hSerpinc1-Sa-13 | CGTACCTCCATCAGTTGCTG(SEQ ID NO: 228) | GAGGG T | 55 | 75.5 |
| hSerpinc 1-Sa-14 | TTCAGTTTGGCAAAGAAGAA(SEQ ID NO: 229) | GTGGA T | 35 | 26.5 |
| hSerpinc1-Sa-17 | GGTAGGTCTCATTGAAGGTA(SEQ ID NO: 232) | AGGGA T | 45 | 61.8 |
| hSerpinc 1-Sa-18 | TGAGACCTACCAGGACATCA(SEQ ID NO: 233) | GTGAG T | 50 | 70.2 |
| hSerpinc 1-Sa-20 | CCCATTTGTTGATGGCCGCT(SEQ ID NO: 235) | CTGGA | 55 | 3.4 |
| | | T | | |
| hSerpinc 1-Sa-21 | TCCAGAGCGGCCATCAACAA(SEQ ID NO: 236) | ATGGG T | 55 | 68.0 |

### ③ CjCas9

**[Table 7]**

| gRNA selection to target SERPINC1 gene(AT gene) for CjCas9 | | | | |
|---|---|---|---|---|
| Target name | Target(w/o PAM) | PAM | GC content | Indels (%) |
| hAPOC3 -Cj -Ctrl | GAGAGGGCCAGAAATCACCCAA(S EQ ID NO: 848) | AGACA CAC | | 36.8 |
| hSerpinc 1-Cj-1 | GAGGTTACAGTTCCTATCACAT(SE Q ID NO: 253) | TGGAA TAC | 41 | 24.6 |
| hSerpinc 1-Cj-2 | CTGTCACGGGAGCCCTGTGGAC(SE Q ID NO: 254) | ATCTG CAC | 68 | 0.3 |
| hSerpinc 1-Cj-3 | GCCTTCTTCTCCGGGGAGCGGT(SE Q ID NO: 255) | AAATG CAC | 68 | 1.3 |
| hSerpinc 1-Cj-4 | GGGAATTGGCCTTGGACAGTTC(SE Q ID NO: 256) | CCAGA CAC | 55 | 3.9 |
| hSerpinc 1-Cj-5 | TCCCGCTTTGCTACCACTTTCT(SEQ ID NO: 257) | ATCAG CAC | 50 | 0 |
| hSerpinc 1-Cj-6 | GCTTGGTCATAGCAAAAGCCGT(SE Q ID NO: 258) | GGAGA TAC | 50 | 3.1 |
| hSerpinc 1-Cj -7 | TATGACCAAGCTGGGTGCCTGT(SE Q ID NO: 259) | AATGA CAC | 55 | 17.5 |
| hSerpinc 1-Cj-8 | TCAGTTGCTGGAGGGTGTCATT(SE Q ID NO: 260) | ACAGG CAC | 50 | 0.9 |
| hSerpinc 1-Cj-9 | ATGACACCCTCCAGCAACTGAT(SE Q ID NO: 261) | GGAGG TAC | 50 | 3.2 |
| hSerpinc1-Cj-10 | TTGACTTCT A T AGGT A TTT AAG(SE Q ID NO: 262) | TTTGA CAC | 27 | 0.3 |
| hSerpincl-Cj-11 | TTTCTCAGAT ATGGTGTCAAAC(SE Q ID NO: 263) | TTAAA TAC | 36 | 0 |
| hSerpinc 1-Cj -12 | TTTGTCTCCAAAAAGGCGATTG(SE Q ID NO: 264) | GCTGA TAC | 41 | 0.2 |
| hSerpinc 1-Cj -13 | GTCCAGGGGCTGGAGCTTGGCT(SE Q ID NO: 265) | CCATA TAC | 68 | 0 |
| hSerpinc 1-Cj -14 | GGTGATTCGGCCTTCGGTCTTA(SE Q ID NO: 266) | TTGGA CAC | 55 | 0.1 |
| hSerpinc 1-Cj -15 | TGAGCTCACTGTTCTGGTGCTG(SE Q ID NO: 267) | GTTAA CAC | 55 | 7.7 |
| hSerpinc 1-Cj -16 | TACCTTGAAGTAAA TGGTGTT A(SE Q ID NO: 268) | ACCAG CAC | 32 | 0.1 |
| hSerpinc 1-Cj -17 | TGCTGGTTAACACCATTTACTT(SEQ ID NO: 269) | CAAGG TAC | 36 | 0.1 |
| hSerpinc 1-Cj-18 | GTGGAAGTCAAAGTTCAGCCCT(SE Q ID NO: 270) | GAGAA CAC | 50 | 20.4 |
| hSerpinc 1-Cj -19 | GGAGAGTCGTGTTCAGCATCTA(SE Q ID NO: 271) | TGATG TAC | 50 | 0 |
| hSerpinc1-Cj-20 | CCTTCCTGGT ACATCAT AGATG(SE Q ID NO: 272) | CTGAA CAC | 45 | 10.1 |
| hSerpinc 1-Cj -21 | CGCCGATAACGGAACTTGCCTT(SE Q ID NO: 273) | CCTGG TAC | 55 | 0.1 |
| hSerpinc 1-Cj -22 | GTTCCGTTATCGGCGCGTGGCT(SE Q ID NO: 274) | GAAGG CAC | 64 | 0.8 |
| hSerpinc 1-Cj -23 | GTCATCACCTTTGAAGGGCAAC(SE Q ID NO: 275) | TCAAG CAC | 50 | 0.1 |
| hSerpinc 1-Cj -24 | CTCCAATTCATCCAGCCACTCT(SE Q ID NO: 276) | TGCAG CAC | 50 | 0 |
| hSerpinc1-Cj-25 | AGAGGTCATCTCGGCCTTCTGC(SE Q ID NO: 277) | AACAA TAC | 59 | 0.3 |
| hSerpinc 1-Cj-26 | ATTCCATAAGGCATTTCTTGAG(SEQ ID NO: 278) | GTGAG | 36 | 2.7 |
| | | TAC | | |
| hSerpinc1-Cj-28 | GCGAACGGCCAGCAATCACAAC(SE Q ID NO: 280) | AGCGG TAC | 59 | 0.4 |
| hSerpinc1-Cj-29 | GGTTTTT ATAAGAGAAGTTCCT(SE Q ID NO: 281) | CTGAA CAC | 32 | 1.3 |
| hSerpinc1-Cj-30 | TGCAAAGAATAAGAACATTTTA(SE Q ID NO: 282) | CTTAA CAC | 23 | 0.1 |

### 2) TFPI gene

### ① SpCas9

**[Table 8]**

| gRNA selection to target TFPI gene for SpCas9 | | | | |
|---|---|---|---|---|
| Target name | Target(w/o PAM) (SEQ ID NO) | PAM | GC content | Indels (%) |
| hMyd88-Sp-Ctrl | GTCCA TCTCCTCCGCCAGCG(SEQ ID NO: 847) | CGG | | 75.2 |
| hTfpi-Sp-4 | A TCAGCATTAAGAGGGGCAG(SEQ ID NO: 286) | GGG | 50 | 54.9 |
| hTfpi-Sp-6 | GAATCAGCATTAAGAGGGGC(SEQ ID NO: 288) | AGG | 50 | 26.4 |
| hTfpi-Sp-17 | TGTGCATTCAAGGCGGATGA(SEQ ID NO: 299) | TGG | 50 | 45.7 |
| hTfpi-Sp-21 | AGTGCGAAGAATTTATATAT(SEQ ID NO: 303) | GGG | 25 | 62.1 |
| hTfpi-Sp-22 | GTGCGAAGAATTTATATATG(SEQ ID NO: 304) | GGG | 30 | 31.1 |
| hTfpi-Sp-33 | ATATAACCTCGACATATTCC(SEQ ID NO: 315) | AGG | 35 | 26.7 |
| hTfpi-Sp-38 | TGTGAACGTTTCAAGTATGG(SEQ ID NO: 320) | TGG | 40 | 57.0 |
| hTfpi-Sp-43 | TGCAAGAACATTTGTGAAGA(SEQ ID NO: 325) | TGG | 35 | 1.0 |
| hTfpi-Sp-45 | TCCACCTGGAAACCATTCGC(SEQ ID NO: 327) | TGG | 55 | 25.5 |
| hTfpi-Sp-47 | TCCAGCGAATGGTTTCCAGG(SEQ ID NO: 329) | TGG | 55 | 26.0 |
| hTfpi-Sp-52 | CTTGGTTGATTGCGGAGTCA(SEQ ID NO: 334) | GGG | 50 | 33.6 |
| hTfpi-Sp-53 | CCTTGGTTGA TTGCGGAGTC(SEQ ID NO: 335) | AGG | 55 | 3.2 |
| hTfpi-Sp-54 | CTGGGAACCTTGGTTGATTG(SEQ ID NO: 336) | CGG | 50 | 37.0 |
| hTfpi-Sp-60 | CCACAAGATTCTTACCAAAA(SEQ ID NO: 342) | AGG | 35 | 13.2 |

### ② SaCas9

**[Table 9]**

| gRNA selection to target TFPI gene for SaCas9 | | | | |
|---|---|---|---|---|
| Target name | Target(w/o PAM) (SEQ ID NO) | PAM | GC content | Indels (%) |
| hAPOC3 -Cj -Ctrl | GAGAGGGCCAGAAATCACCCAA(S EQ ID NO: 848) | AGACA CAC | | 57.3 |
| hTfpi-Sa-3 | ATCCGCCTTGAATGCACAAA(SEQ ID NO: 375) | ATGAA T | 45 | 3.0 |
| hTfpi-Sa-5 | TACATGGGCCATCATCCGCC(SEQ ID NO: 377) | TTGAA T | 60 | 68.2 |
| hTfpi-Sa-8 | GTGCGAAGAATTTATATATG(SEQ ID NO: 380) | GGGGA T | 30 | 34.6 |
| hTfpi-Sa-10 | GAATCGATTTGAAAGTCTGG(SEQ ID NO: 382) | AAGAG T | 40 | 72.2 |
| hTfpi-Sa-13 | AATATAACCTCGACATATTC(SEQ ID NO: 385) | CAGGA T | 30 | 15.1 |
| hTfpi-Sa-14 | GTGTGAACGTTTCAAGTATG(SEQ ID NO: 386) | GTGGA T | 40 | 38.8 |
| hTfpi-Sa-16 | TTCCAGCGAATGGTTTCCAG(SEQ ID NO: 388) | GTGGA T | 50 | 58.6 |
| hTfpi-Sa-17 | GAGTTATTCACAGCATTGAG(SEQ ID NO: 389) | CTGGG T | 40 | 62.6 |
| hTfpi-Sa-18 | ATGGAACCCAGCTCAA TGCT(SEQ ID NO: 390) | GTGAA T | 50 | 38.9 |
| hTfpi-Sa-19 | CTTGGTTGATTGCGGAGTCA(SEQ ID NO: 391) | GGGAG T | 50 | 67.1 |
| hTfpi-Sa-20 | CTGGGAACCTTGGTTGATTG(SEQ ID NO: 392) | CGGAG T | 50 | 73.6 |
| hTfpi-Sa-22 | CGACACAATCCTCTGTCTGC(SEQ ID NO: 394) | TGGAG T | 55 | 47.0 |
| hTfpi-Sa-24 | TCCCAATGACTGAATTGTAG(SEQ ID NO: 396) | TAGAA T | 40 | 49.5 |
| hTfpi-Sa-25 | TGGGCGGCATTTCCCAATGA(SEQ ID NO: 397) | CTGAA T | 55 | 57.1 |
| hTfpi-Sa-26 | ATGCCGCCCATTTAAGTACA(SEQ ID NO: 398) | GTGGA T | 45 | 39.0 |

### ③ CjCas9

**[Table 10]**

| gRNA selection to target TFPI gene for CjCas9 | | | | |
|---|---|---|---|---|
| Target name | Target(w/o PAM) (SEQ ID NO) | PAM | GC content | Indels (%) |
| hAPOC3-Cj-Ctrl | GAGAGGGCCAGAAATCACCCAA(SEQ ID NO: 848) | AGACA CAC | | 36.8 |
| hTfpi-Cj-1 | TGGGTTCTGTATTTCAGAGATG(SEQ ID NO: 403) | ATTTAC AC | 41 | 0 |
| hTfpi-Cj-2 | TCTTCATTGTGTAAATCATCTC(SEQ ID NO: 404) | TGAAA | 32 | 1.2 |
| | | TAC | | |
| hTfpi-Cj-3 | CAGAGATGATTTACACAATGAA(SE Q ID NO: 405) | GAAAG TAC | 32 | 3.9 |
| hTfpi-Cj-5 | CAGGCATACAGAAGCCCAAAGT(SE Q ID NO: 407) | GCATG TAC | 50 | 0.8 |
| hTfpi-Cj-6 | GGCAGGGGCAAGATTAAGCAGC(SE Q ID NO: 408) | AGGCA TAC | 59 | 19.3 |
| hTfpi-Cj-7 | AATGCTGATTCTGAGGAAGATG(SE Q ID NO: 409) | AAGAA CAC | 41 | 22.1 |
| hTfpi-Cj-8 | TGCTGATTCTGAGGAAGATGAA(SE Q ID NO: 410) | GAACA CAC | 41 | 17.5 |
| hTfpi-Cj-9 | TACATGGGCCATCATCCGCCTT(SEQ ID NO: 411) | GAATG CAC | 55 | 0 |
| hTfpi-Cj-10 | TCACATCCCCCATATATAAATT(SEQ ID NO: 412) | CTTCGC AC | 32 | 0 |
| hTfpi-Cj-11 | AAGTCTGGAAGAGTGCAAAAAA(SE Q ID NO: 413) | ATGTG TAC | 36 | 0.3 |
| hTfpi-Cj-12 | AACCTACCTCTTGTACACATTT(SEQ ID NO: 414) | TTTTGC AC | 36 | 0 |
| hTfpi-Cj-13 | AGGGTTCCCAGAAACCTACCTC(SEQ ID NO: 415) | TTGTAC AC | 55 | 1.6 |
| hTfpi-Cj-14 | CACTGTTTTGTCTGATTGTTAT(SEQ ID NO: 416) | AAAAA TAC | 32 | 0.1 |
| hTfpi-Cj-15 | AGGCATCCACCATACTTGAAAC(SE Q ID NO: 417) | GTTCA CAC | 45 | 1 |
| hTfpi-Cj-16 | TTGTTCATATTGCCCAGGCATC(SEQ ID NO: 418) | CACCA TAC | 45 | 0.3 |
| hTfpi-Cj-17 | GCCTGGGCAATATGAACAATTT(SEQ ID NO: 419) | TGAGA CAC | 41 | 0.1 |
| hTfpi-Cj-18 | CACAATCCTCTGTCTGCTGGAG(SEQ ID NO: 420) | TGAGA CAC | 55 | 16.5 |
| hTfpi-Cj-19 | TAGTAGAATCTGTTCTCATTGG(SEQ ID NO: 421) | CACGA CAC | 36 | 8.7 |
| hTfpi-Cj-20 | AATTGTAGTAGAATCTGTTCTC(SEQ ID NO: 422) | ATTGG CAC | 32 | 0.1 |
| hTfpi-Cj-21 | GTCATTGGGAAATGCCGCCCAT(SEQ ID NO: 423) | TTAAG TAC | 55 | 1.5 |
| hTfpi-Cj-22 | TTGTTTTCATTTCCCCCACATC(SEQ ID NO: 424) | CACTG TAC | 41 | 0 |

Therefore, it is expected that the composition, which includes gRNA and Cas9 targeting the AT gene and the TFPI gene presented in this study, may be used to treat hemophilia.

### [Industrial Applicability]

According to the present invention, a blood coagulation system can be regulated using a composition for gene manipulation, which includes a guide nucleic acid targeting a blood coagulation inhibitory gene, and an editor protein, by artificially manipulating and/or modifying the blood coagulation inhibitory gene to regulate the function and/or expression of the blood coagulation inhibitory gene, and thus coagulopathy can be treated or improved using the composition for gene manipulation.

### [Sequence List Text]

Target sequences of AT gene and TFPI gene and guide sequences capable of targeting the target sequences.

## Claims

1. A method of treating a hemophilia, the method including administration of a composition for gene manipulation into a subject to be treated,
wherein the composition for gene manipulation includes the following:
a guide nucleic acid including a guide sequence forming a complementary bond with a target sequence located in a blood coagulation inhibitory gene, or a nucleic acid sequence encoding the same; and
an editor protein, or a nucleic acid sequence encoding the same,
wherein the blood coagulation inhibitory gene is AT(antithrombin) gene or TFPI(tissue factor pathway inhibition) gene,
wherein the guide sequence is one or more guide sequences selected from a SEQ ID NO:425 to 830,
wherein the complementary bond includes mismatching bonds of 0 to 5.

2. The method of claim 1,
wherein the guide sequence is one or more sequences selected from a SEQ ID NO : 427, 428, 436, 437, 443, 444, 447, 448, 449, 454, 458, 460, 461, 463, 464, 466, 467, 469, 473, 474, 622, 623, 624, 625, 626, 627, 628, 630, 632, 634, 635, 638, 639, 641, 642, 659, 660, 661, 662, 663, 664, 665, 666, 667, 668, 669, 670, 671, 672, 673, 674, 675, 676, 677, 678, 679, 680, 681, 682, 683, 684, 686, 687 and 688.

3. The method of claim 1,
wherein the guide sequence is one or more sequences selected from a SEQ ID NO: 692, 694, 705, 709, 710, 721, 726, 731, 733, 735, 740, 741, 742, 748, 781, 783, 786, 788, 791, 792, 794, 795, 796, 797, 798, 800, 802, 803, 804, 809, 810, 811, 813, 814, 815, 816, 817, 818, 819, 820, 821, 822, 823, 824, 825, 826, 827, 828, 829 and 830.

4. The method of claim 1,
wherein the guide sequence forms a complementary bond with a target sequence located in exon 1, exon 2, exon 3, exon 4, exon 5, exon 6 or exon 7 of AT gene.

5. The method of claim 1,
wherein the guide sequence forms a complementary bond with a target sequence located in exon 2, exon 3, exon 5, exon 6 or exon 7 of TFPI gene.

6. The method of claim 1,
wherein the editor protein is a Streptococcus pyogenes-derived Cas9 protein, a Staphylococcus aureus-derived Cas9 protein, or a *Campylobacter jejuni*-derived Cas9 protein.

7. The method of claim 1,
wherein the composition for gene manipulation is a form of guide nucleic acid-editor protein complex combined guide nucleic acid and editor protein,
wherein the guide nucleic acid is a guide RNA(gRNA).

8. The method of claim 1,
wherein the guide nucleic acid and the editor protein are present in one or more vectors in a form of a nucleic acid sequence, respectively.

9. The method of claim 1,
wherein the composition for gene manipulation optionally further includes a donor including a nucleic acid sequence to be inserted, or a nucleic acid sequence encoding the same.

10. The method of claim 9,
wherein the nucleic acid sequence to be inserted is a partial nucleic acid sequence of blood coagulation inhibitory gene.

11. The method of claim 9,
wherein the nucleic acid sequence to be inserted is a complete or partial sequence of a gene encoding a blood coagulation associated protein.

12. The method of claim 11,
wherein the blood coagulation associated protein is one or more proteins selected from the group consisting of a factor XII, factor XIIa, factor XI, factor XIa, factor IX, factor IXa, factor X, factor Xa, factor VIII, factor VIIIa, factor VII, factor VIIa, factor V, factor Va, prothrombin, thrombin, factor XIII, factor XIIIa, fibrinogen, fibrin and tissue factor.

13. The method of claim 9,
wherein the guide nucleic acid, the editor protein and donor are present in one or more vectors in a form of a nucleic acid sequence, respectively.

14. The method of claim 8 or 13,
wherein the vector is a plasmid or viral vector.

15. The method of claim 14,
wherein the viral vector is a one or more viral vectors selected from the group consisting of a retrovirus, a lentivirus, an adenovirus, an adeno-associated virus (AAV), a vaccinia virus, a poxvirus and a herpes simplex virus.

16. The method of claim 1,
wherein the administration is performed by injection, transfusion, implantation or transplantation.

17. The method of claim 1,
wherein the administration is performed via an administration route selected from intrahepatic, subcutaneous, intradermal, intraocular, intravitreal, intratumoral, intranodal, intramedullary, intramuscular, intravenous, intralymphatic and intraperitoneal route.

18. The method of claim 1,
wherein the hemophilia is a hemophilia A, hemophilia B or hemophilia C.

19. The method of claim 1,
wherein the subject to be treated is a mammal including a human, a monkey, a mouse and a rat.

20. A composition for gene manipulation including
a guide nucleic acid including a guide sequence forming a complementary bond with a target sequence located in blood coagulation inhibitory gene, or a nucleic acid sequence encoding the same; and
an editor protein, or a nucleic acid sequence encoding the same,
wherein the blood coagulation inhibitory gene is AT(antithrombin) gene or TFPI(tissue factor pathway inhibition) gene,
wherein the guide sequence is one or more guide sequences selected from a SEQ ID NO:425 to 830,
wherein the complementary bond includes mismatching bonds of 0 to 5.

21. The composition of claim 20,
Wherein the target sequence is one or more sequences selected from a SEQ ID NO:19 to 424.

22. The composition of claim 21,
Wherein the target sequence is one or more sequences selected from a SEQ ID NO: 21, 22, 30, 31, 37, 38, 41, 42, 43, 48, 52, 54, 55, 57, 58, 60, 61, 63, 67, 68, 216, 217, 218, 219, 220, 221, 222, 224, 226, 228, 229, 232, 233, 235, 236, 253, 254, 255, 256, 257, 258, 259, 260, 261, 262, 263, 264, 265, 266, 267, 268, 269, 270, 271, 272, 273, 274, 275, 276, 277, 278, 280, 281 and 282.

23. The composition of claim 21,
Wherein the target sequence is one or more sequences selected from a SEQ ID NO: 286, 288, 299, 303, 304, 315, 320, 325, 327, 329, 334, 335, 336, 342, 375, 377, 380, 382, 385, 386, 388, 389, 390, 391, 392, 394, 396, 397, 398, 403, 404, 405, 407, 408, 409, 410, 411, 412, 413, 414, 415, 416, 417, 418, 419, 420, 421, 422, 423 and 424.

24. The composition of claim 20,
Wherein the guide sequence is one or more sequences selected from a SEQ ID NO: 427, 428, 436, 437, 443, 444, 447, 448, 449, 454, 458, 460, 461, 463, 464, 466, 467, 469, 473, 474, 622, 623, 624, 625, 626, 627, 628, 630, 632, 634, 635, 638, 639, 641, 642, 659, 660, 661, 662, 663, 664, 665, 666, 667, 668, 669, 670, 671, 672, 673, 674, 675, 676, 677, 678, 679, 680, 681, 682, 683, 684, 686, 687 and 688.

25. The composition of claim 20,
Wherein the guide sequence is one or more sequences selected from a SEQ ID NO: 692, 694, 705, 709, 710, 721, 726, 731, 733, 735, 740, 741, 742, 748, 781, 783, 786, 788, 791, 792, 794, 795, 796, 797, 798, 800, 802, 803, 804, 809, 810, 811, 813, 814, 815, 816, 817, 818, 819, 820, 821, 822, 823, 824, 825, 826, 827, 828, 829 and 830.

26. The composition of claim 20,
wherein the guide sequence forms a complementary bond with a target sequence located in exon 1, exon 2, exon 3, exon 4, exon 5, exon 6 or exon 7 of AT gene.

27. The composition of claim 20,
wherein the guide sequence forms a complementary bond with a target sequence located in exon 2, exon 3, exon 5, exon 6 or exon 7 of TFPI gene.

28. The composition of claim 20,
Wherein the guide nucleic acid includes a guide domain.

29. The composition of claim 28,
Wherein the guide sequence is included in the guide domain.

30. The composition of claim 20,
Wherein the guide nucleic acid includes one or more domains selected from a first complementary domain, a second complementary domain, a linker domain, a proximal domain and a tail domain.

31. The composition of claim 20,
wherein the editor protein is a Streptococcus pyogenes-derived Cas9 protein, a Staphylococcus aureus-derived Cas9 protein, or a *Campylobacter jejuni*-derived Cas9 protein.

32. The composition of claim 20,
wherein the composition for gene manipulation is a form of guide nucleic acid-editor protein complex combined guide nucleic acid and editor protein,
wherein the guide nucleic acid is a guide RNA(gRNA).

33. The composition of claim 20,
wherein the guide nucleic acid and the editor protein are present in one or more vectors in a form of a nucleic acid sequence, respectively.

34. The composition of claim 20,
wherein the composition for gene manipulation optionally further includes a donor including a nucleic acid sequence to be inserted, or a nucleic acid sequence encoding the same.

35. The composition of claim 34,
Wherein the nucleic acid sequence to be inserted is a partial nucleic acid sequence of blood coagulation inhibitory gene.

36. The composition of claim 34,
wherein the nucleic acid sequence to be inserted is a complete or partial sequence of a gene encoding a blood coagulation associated protein.

37. The composition of claim 36,
wherein the blood coagulation associated protein is one or more proteins selected from the group consisting of a factor XII, factor XIIa, factor XI, factor XIa, factor IX, factor IXa, factor X, factor Xa, factor VIII, factor VIIIa, factor VII, factor VIIa, factor V, factor Va, prothrombin, thrombin, factor XIII, factor XIIIa, fibrinogen, fibrin and tissue factor.

38. The composition of claim 34,
wherein the guide nucleic acid, the editor protein and donor are present in one or more vectors in a form of a nucleic acid sequence, respectively.

39. The composition of claim 33 or 38,
Wherein the vector is a plasmid or viral vector.

40. The composition of claim 39,
Wherein the viral vector is a one or more viral vectors selected from the group consisting of a retrovirus, a lentivirus, an adenovirus, an adeno-associated virus (AAV), a vaccinia virus, a poxvirus and a herpes simplex virus.

41. A guide nucleic acid including a guide sequence forming a complementary bond with a target sequence located in a blood coagulation inhibitory gene,
wherein the blood coagulation inhibitory gene is AT(antithrombin) gene or TFPI(tissue factor pathway inhibition) gene,
wherein the guide sequence is one or more guide sequences selected from a SEQ ID NO:425 to 830,
wherein the complementary bond includes mismatching bonds of 0 to 5,
wherein the guide nucleic acid is capable of forming a complex with an editor protein.

42. The guide nucleic acid of claim 41,
wherein the guide sequence is one or more sequences selected from a SEQ ID NO: 427, 428, 436, 437, 443, 444, 447, 448, 449, 454, 458, 460, 461, 463, 464, 466, 467, 469, 473, 474, 622, 623, 624, 625, 626, 627, 628, 630, 632, 634, 635, 638, 639, 641, 642, 659, 660, 661, 662, 663, 664, 665, 666, 667, 668, 669, 670, 671, 672, 673, 674, 675, 676, 677, 678, 679, 680, 681, 682, 683, 684, 686, 687 and 688.

43. The guide nucleic acid of claim 41,
wherein the guide sequence is one or more sequences selected from a SEQ ID NO: 692, 694, 705, 709, 710, 721, 726, 731, 733, 735, 740, 741, 742, 748, 781, 783, 786, 788, 791, 792, 794, 795, 796, 797, 798, 800, 802, 803, 804, 809, 810, 811, 813, 814, 815, 816, 817, 818, 819, 820, 821, 822, 823, 824, 825, 826, 827, 828, 829 and 830.

44. The guide nucleic acid of claim 41,
wherein the guide sequence forms a complementary bond with a target sequence located in exon 1, exon 2, exon 3, exon 4, exon 5, exon 6 or exon 7 of AT gene.

45. The guide nucleic acid of claim 41,
wherein the guide sequence forms a complementary bond with a target sequence located in exon 2, exon 3, exon 5, exon 6 or exon 7 of TFPI gene.

46. The guide nucleic acid of claim 41,
wherein the guide nucleic acid includes a guide domain.

47. The guide nucleic acid of claim 46,
wherein the guide sequence is included in the guide domain.

48. The guide nucleic acid of claim 41,
wherein the guide nucleic acid includes one or more domains selected from a first complementary domain, a second complementary domain, a linker domain, a proximal domain and a tail domain.
